# EUROPEAN PATENT APPLICATION

(11) **EP 4 095 120 A1**
(43) Date of publication of application: **30.11.2022**
(21) Application number: 21744691.3
(22) Date of filing: 21.01.2021
(51) Int. Cl.: C06B 29/02

(54) **COMPOUNDS AND PREPARATION METHOD THEREFOR AND USE THEREOF AS ENERGETIC MATERIALS**

(30) Priority: 22.01.2020 CN 202010075667; 24.03.2020 CN 202010211524; 14.07.2020 CN 202010672415
(71) Applicant: Xi'an Crysten Materials Technology Corporation Limited, Xi'an City, Shaanxi Province 710000 (CN)
(72) Inventor: ZHANG, Weixiong, Guangzhou, Guangdong 510275 (CN); SHANG, Yu, Guangzhou, Guangdong 510275 (CN); CHEN, Shaoli, Guangzhou, Guangdong 510275 (CN); CHEN, Xiaoming, Guangzhou, Guangdong 510275 (CN)
(74) Representative: AWA Sweden AB
(86) International application number: PCT/CN2021/073128
(87) International publication number: WO 2021/147960

(57) **Abstract**

Provided are a series of ionic compounds and a preparation method therefor and a use thereof as energetic materials.

## Description

### TECHNICAL FIELD

The present disclosure relates to the field of energetic materials, and particularly relates to a series of compounds, a preparation method therefor and a use thereof as energetic materials.

### BACKGROUND ART

An energetic material is a compound or a mixture which can quickly perform self-oxidation-reduction reaction under the stimulation of external energy and quickly release a large amount of heat and gas so as to apply work to a surrounding medium. The energetic material has very wide use in the military (such as signal bullets of arms and ammunition, propellants and pyrotechnics) and the civil field (such as fireworks and crackers, perforating bullets for oil exploitation, civil engineering and mining blasting), it plays an important role in promoting national defense construction and national economy development, and has the characteristics of high temperature, high pressure and high speed reaction and instant one-time effect.

The energetic material as a special energy source is a power source for various thermal systems to perform projectile launching and rocket missile in military, it is a technical and material basis for national military actual combat power and deterrent power and is widely used in the fields of engineering blasting, machining, geological exploration, ejection devices, fireworks and crackers, safety airbags, *etc.* in civil. The earliest energetic material is Chinese black powder which appeared in the 9^{th} Century AD, it is a mixture of sulfur, potassium nitrate and charcoal powder, and has poor effect and unstable performance. An elementary substance energetic material has been on the rise since the mid-19^{th} Century, and nitroglycerin is the early prominent representation. Nobel used kieselguhrto adsorb nitroglycerin so as to invent the dynamite explosive, thus achieving the industrial use of nitroglycerin. After entering the modern society, elementary substance energetic materials with higher performance than nitroglycerin have been continuously found and put into use, such as famous trinitrotoluene (TNT) and organic energetic materials which are hexogen (RDX) and octogen (HMX) with higher explosion performance than TNT In the development history of the energetic material, the research of the elementary substance energetic material has always been in a core position. Each great innovation of the elementary substance energetic material (such as invention and application of compounds like TNT, RDX and HMX) brought great changes to the energetic material and even weaponry (Agrawal, J. P. ISBN: 978-3-527-32610-5. Wiley, 2010.).

According to the use occasions and performance characteristics, the energetic material can be mainly divided into four categories, namely primary explosives, high explosives (also called secondary explosives), propellants and pyrotechnics. In order to adapt to different use occasions, the four categories of energetic materials have different emphasis on performance requirements of related elementary substance energetic materials.

The primary explosive is a kind of explosive which deflagrates and can be quickly converted into detonation under the action of initial external weak impact energy (such as friction, impact, needling, electric energy, flame and laser). Such kind of explosive has the characteristics of high sensitivity, quick conversion from deflagrating to detonation, *etc.,* and detonation waves generated by the explosive are used for detonating the high explosive, so the explosive is also called as an initial explosive, an initial charge or a primary explosive. The primary explosive is the initial energy which has the most sensitivity to detonation and ignition, and is widely used to military and civil detonation or ignition devices, such as industrial detonators, igniters of detonatingtubes or ignition devices of rockets, satellites, weapons, mining and tunneling. The earliest used primary explosive is mercuric fulminate (MF), which has been an important component of detonator charges and detonating cap percussion powder for more than two hundred years after being discovered in 1628. However, the primary explosive was gradually replaced by lead azide (LA), lead styphnate (LS) and the like from the beginning of 20th Century due to its defects of relatively poor stability, high toxicity, high possibility of corroding bores and cartridges and the like, which opened an era of lead-based primary explosives. At present, the LA and the LS are still the most widely used primary explosives. However, with the improvement of the environmental awareness of people and the increasing attention to lead pollution, the search for a new generation of relatively safe and environment-friendly lead-free primary explosive has become a hot spot in the field of current initiating explosive material research.

The high explosive (also called secondary explosive) has higher stability than the primary explosive, it is generally not detonated by heat or impact in the using process, but is detonated by means of impact waves of the primary explosive to generate detonation, which can generate stronger destructive power. The high explosives in service at present mainly include TNT, RDX, HMX and TATB (Klapätke, T. M., High energy density materials, Springer, 2007). Along with the development of weaponry, there are higher requirements on the safety and detonation performance of the high explosives. TNT and TATB are insensitive, but have low energy level. RDX and HMX have various crystal forms, and have the defects of high mechanical sensitivity, poor safety performance and the like. Therefore, designing a high explosive with high synthesis energy level and good stability is the target pursued in the field of energetic materials all the time.

The propellants and propellant powder belong to gunpowder, both are regularly combusted to release energy and generate gas to push rockets and missiles (the propellants) or to launch bullets in a gun chamber (the propellants). Such kind of gunpowder is usually an energetic compound without metal components. For example, HMX, RDX and the like are usually used as important additives in a propellant formula to increase the specific impulse value of the formula and are used in formulas of some high-energy modified double-base propellants (Tan Huimin, the Chemistry and Technology of Solid Rocket Propellant, Beijing Institute of Technology Press, 2015). A large number of novel modern energetic materials emerging from at present, such as organic energetic materials represented by hexanitrohexaazaisowurtzitane (CL-20) and the like and all-nitrogen compounds which are still in a basic research stage, are good at explosion and specific impulse performance, but are always difficult to be used on a large scale due to their defects of poor structural stability, complex synthesis process, too high price, *etc.* Therefore, designing and synthesizing a high-energy energetic material with the advantages of low cost, high energy density, high safety and the like is a durable pursuit in the field of energetic materials, particularly in the field of propellants.

The pyrotechnics belong to an important energetic material. Pyrotechnics are a kind of agent which is relatively low in decomposition reaction speed (can be combusted or deflagrated), such agent can generate light, smoke and noise, and can also emit a specific spectral color (Thomas M. Klapötke, Chemistry of High-Energy Materials, De Gruyter, 2012) after adding metal and metal salt and is often used for manufacturing fireworks and making a tracer bullet in military. The field of pyrotechnics is overlapped with the fields of propellants and explosives, and some pyrotechnics can be often used in the field of explosives or propellants. Some modern high-energy explosives can also be applied to a pyrotechnics formula (Conkling, J. A. & Mocella C. J. ISBN: 978-1-4200-1809-7. CRC Press, 2010.) under a low constraint condition. The earliest pyrotechnics are famous black powder, which appeared in the Tang Dynasty at the end of the 9th Century AD and was prepared by mixing potassium nitrate, sulfur and charcoal. The combustion and explosion performance of a black powder mixture is greatly dependent on a preparation process and a using condition. The black powder under an unconstrained condition is just a fuel used by the alchemists in the Middle Ages, and with the improvement of the preparation process, the black powder was gradually used for making performance pyrotechnics, and finally used as explosives, which opened the era of thermal weapons. At present, the black powder is still an important pyrotechnics agent for civil use and military use, but it is easily fired and exploded in the production process of mixed agent, which often causes accidents. Therefore, designing a new generation of elementary-substance pyrotechnics with high invariability, simple process and high stability through theory has become a hot spot in the field of pyrotechnics.

The amount of the newly synthesized energetic material is rapidly increased in the past few decades, and particularly, the development of the energetic material is greatly promoted by the research of crystalline energetic materials such as organic energetic molecules and binary salts thereof, all nitrogen compounds, eutectic compounds, metal complexes and molecular perovskite compounds.

For the energetic material which takes oxidation-reduction reaction as a main mechanism to "quickly release energy and produce gas", oxygen balance, crystal density and enthalpies of formation are the important physical parameters in material design. The oxygen balance parameter reflects the difference degree between the actual oxygen content in the material and the oxygen content required for complete oxidation of carbon and hydrogen in the material, and when the oxygen balance is zero, the carbon and the hydrogen can be respectively and fully converted into carbon dioxide and water theoretically, namely complete combustion is achieved, so that energy is released to the maximum extent. The density of the energetic material directly influences the detonation velocity and the detonation pressure, and the higher the density is, the higher the theoretical detonation velocity and the detonation pressure are. However, the oxygen balance and the crystal density are often correlated and are difficult to improve at the same time so as to comprehensively improve the detonation parameter; in addition, although the improvement of the enthalpies of formation is favorable for improving the energy level of the energetic material, it often brings lower thermal stability. Therefore, how to reasonably select components based on a crystal engineering principle so as to optimize the oxygen balance and the crystal density of the energetic compound at the same time, and consider the energy level and the thermal stability is an important challenge topic in the process of designing and synthesizing a novel energetic compound.

A metal-free molecular perovskite type energetic material with NH₄⁺ cations as a site B, with the advantages of no metal residue after explosion or decomposition, large gas production rate, high explosion heat or combustion heat value and the like, has a good use prospect in the fields of propellants, high explosive, *etc.*

However, it is currently found that a metal-free molecular perovskite type energetic compound constructed by adjusting A-site component has encountered a bottleneck in the aspects of improving crystal density and enthalpies of formation at the same time, it is difficult to further break through its energetic characteristic. For example, for the first metal-free molecular perovskite energetic compound (C₆H₁₄N₂)(NH₄)(ClO₄)₃ (DAP-4) and one of its optimized compounds (C₆H₁₄N₂O)(NH₄)(ClO₄)₃ (DAP-O4] obtained by adjusting the A-site component, the crystal density is respectively 1.87 g/cm³ and 1.85 g/cm³, the enthalpies of formation are respectively -484.0 kJ/mol and -436.1 kJ/mol, the heat of detonation is respectively 1.40 kcal/g and 1.48 kcal/g, the detonation velocity is respectively 8.806 km/s and 8.900 km/s, and the detonation pressure is respectively 35.2 GPa and 35.7 GPa. The performance of the optimized compound is not significantly improved when compared with that of the first metal-free molecular perovskite energetic compound.

An ethylenediamine cation (H₂EA²⁺) is a positive divalent cation which can exist stably and has a small molecular weight, it can form a stable salt with twice the equivalent of negative monovalent oxidative anions, and thus an oxygen balance parameter close to zero can be obtained. For example, early ethylenediamine dinitrate, ethylenediamine diperchlorate and the like all have better detonation performance than TNT, but both have serious hygroscopicity and are not further applied (The Chemistry and Technology of Propellants and Explosives (Volume II), by Urbanski (Poland), translated by Niu Bingyi and Chen Shaoliang; National Defense Industry Press, 1976, pages 344 and 356). In 2008, Zhu Shunguan and others from Nanjing University of Science and Technology co-crystallized ethylenediamine diperchlorate and triethylene diamine diperchlorate to obtain an energetic compound (C₆H₁₄N₂)(H₂EA)(ClO₄)₄ (named SY, Chinese invention patent ZL200810025381.3), and found that SY has smaller hygroscopicity and better thermal stability than pure ethylenediamine diperchlorate, and shows good performance in the aspect of primary explosives. However, the crystallographic density value (reported value is 1.834 g/cm³, the real value should be 1.867 g/cm³) of SY does not reach 1.9 g/cm³, and the oxygen balance (-27.8%) is also lower than the corresponding values (-21.6%) of the currently commonly used RDX and HMX. In addition, covalent bonds of the ethylenediamine cation are all single bonds, have a significant "flexible" structure, and can be used as hydrogen bond donors.

For the metal-free molecular perovskite type energetic material, how to adjust the components of the energetic material to further improve the crystal density, enthalpy generation and/or oxygen balance parameters at the same time so as to improve the energetic characteristic of the energetic material is a challenging subject with important significance.

The Chinese patent 201610665880.3 discloses an energetic material with a specific structure.

### SUMMARY

In some embodiments, a compound is provided. In some embodiments, a crystalline compound is provided. In some embodiments, a metal-free compound, such as a metal-free perovskite-type compound, is provided. In some embodiments, a silver-containing compound, such as a silver-containing perovskite-type compound, is provided. In some embodiments, a potassium-containing compound, such as a potassium-containing perovskite-type compound, is provided. In some embodiments, a hydroxylammonium-containing compound is provided. In some embodiments, a hydrazinium-containing compound is provided.

In some embodiments, an energetic material having good thermal stability, such as a metal-free compound having good thermal stability, such as a metal-free perovskite-type compound, is provided. In some embodiments, an energetic material having good explosive properties, such as a metal-free compound having good explosive properties, such as a metal-free perovskite-type compound, is provided. In some embodiments, a high energy density metal-free compound, such as a high energy density metal-free compound, such as a metal-free perovskite-type compound, is provided. In some embodiments, a metal-free compound having a high theoretical specific impulse, such as a metal-free compound having a high theoretical specific impulse, such as a metal-free perovskite-type compound, is provided. In some embodiments, a more sensitive silver-containing compound, such as a silver-containing perovskite-type compound, is provided. In some embodiments, a silver-containing compound having good explosive properties, such as a silver-containing perovskite-type compound, is provided. In some embodiments, a high energy density silver-containing compound, such as a silver-containing perovskite-type compound, is provided. In some embodiments, a potassium-containing compound having characteristic flame colors, such as potassium-containing perovskite-type compound, is provided. In some embodiments, a potassium-containing compound having good stabilizing properties, such as a potassium-containing perovskite-type compound, is provided. In some embodiments, a crystalline compound assembled based on hydroxylammonium and/or hydrazinium ions, a solid solution therefor, and a use thereof as energetic materials, are provided. In some embodiments, the metal-free compounds of the present application are suitable at least, in particular, as propellants and dynamite. In some embodiments, the silver-containing compounds of the present application are suitable at least, in particular, as a primer. In some embodiments, the potassium-containing compounds of the present application are suitable at least, in particular, as pyrotechnics and dynamite. In some embodiments, the compounds having hydroxylammonium and/or hydrazinium ions of the present application have excellent energetic properties and utility during use.

In some embodiments, the compounds of the present application have energetic ligands. The energetic ligands refer to groups with explosivity, and common groups with explosivity include ClO₃⁻, ClO₄⁻, NO₃⁻, ONC⁻, diazenyl groups, and azide ion groups, for example, the ClO₄⁻ group. Such energetic materials may be used as, but are not limited to, explosives. For example, it may also be used as propellants, rocket fuel or gas generant for airbags.

In some embodiments, the compounds of the present application are perovskite-type compounds. Perovskite-type compounds are a class of solid state compounds with a similar crystal structure to calcium titanate (CaTiO₃), having the same general chemical formula ABX₃, wherein A and B are cations of different sizes and X is an anion. The ideal structure of perovskite-type compounds belongs to the cubic crystal system with high symmetry, and the structural features can be described as: each B cation is connected to six adjacent X anions, and each X anion is connected to two adjacent B cations, thereby forming a three-dimensional anionic backbone consisting of cubic cage-like units; the A cations then fill the pores of the cubic cage-like units. Wherein A is a cation, B is a cation, and X is an anion. However, the perovskite-type compound may also exhibit other structures, such as a hexagonal perovskite structure, and in other embodiments, its structural features may be described as: each B cation is surrounded by six X anions to form a BX₆ octahedral unit, adjacent B cations are connected by three X anions to form a one-dimensional chain, and A cations are filled in the chains. When the perovskite-type compound ABX₃ includes more than one A cation, different A cations may be distributed on the A site in an ordered or disordered manner. When the perovskite-type compound ABX₃ includes more than one B cation, different B cations may be distributed on the B site in an ordered or disordered manner. When the perovskite-type compound ABX₃ includes more than one X anion, different X anions may be distributed on the X site in an ordered or disordered manner.

In some embodiments, the compounds of the present application are compounds of a general formula ABX₃, consisting of A cation, B cation, and X anion. In some embodiments, the compounds of the present application are compounds of a general formula AB'X₄, consisting of A cation, B cation, and X anion. In some embodiments, the compounds of the present application are compounds of a general formula B'₂A'X₅, consisting of A' cation, a B cation, and X anion. In some embodiments, the compounds ABX₃ are perovskite-type compounds ABX₃.

In some embodiments, in the compounds ABX₃, compounds AB'X₄, and/or compounds B'₂A'X₅ of the present application:
In some embodiments, the A cation is an organic cation. In some embodiments, the A cation is a nitrogen-containing heterocyclic organic cation. In some embodiments, the A cation is a divalent nitrogen-containing organic cation. In some embodiments, the A cation is a divalent nitrogen-containing heterocyclic organic cation. In some embodiments, the A cation is selected from at least one of a divalent nitrogen-containing five-membered ring cation, a divalent nitrogen-containing six-membered ring cation, and a divalent nitrogen-containing seven-membered ring cation.

In some embodiments, the A cation may be selected from at least one of ions of Formula (I) and Formula (II), and derivatives thereof, wherein preferably *n*₁, *n*₂*, n*₃, *n*₄, and *n*₅ each may be positive integer of 1, 2, 3, 4, or 5, more preferably 1, 2, or 3; R₁, R₂, R₃, and R₄ may be selected from at least one of hydrogen, methyl, ethyl, propyl, isopropyl, butyl, isobutyl, *sec*-butyl, *tert*-butyl, hydroxyl, carbonyl, carboxyl, amino, halogen, mercapto, peroxy, diazenyl, and nitro groups; the derivatives refer to in the organic cationic moiety in which hydrogen atoms are substituted, simultaneously or not, with substituents, and common substituents including methyl, ethyl, propyl, isopropyl, butyl, isobutyl, *sec*-butyl, *tert*-butyl, hydroxyl, carbonyl, carboxyl, amino, halogen, mercapto, peroxy, diazenyl, nitro, *etc.*

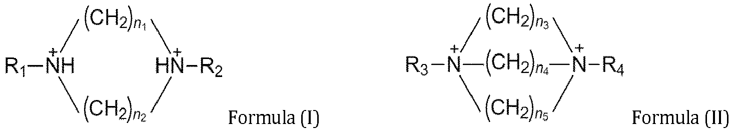

In some embodiments, the A cation is selected from at least one of ions of Formula (I) and derivatives thereof, and
any one of *n*₁ and *n*₂ is greater than 2, or any one of R₁ and R₂ includes at least one carbon atom;
or
the A cation is selected from at least one of ions of Formula (II) and derivatives thereof, and
any one of *n*₃, *n*₄, and *n*₅ is greater than 2, or any one of R₃ and R₄ includes at least one carbon atom.

In some embodiments, the A cation is selected from at least one of ions of Formula (I) and derivatives thereof; and any one of *n*₁ and *n*₂ is greater than 2, or any one of R₁ and R₂ includes at least one carbon atom. In some embodiments, the A cation is selected from at least one of Formula (II) ions and derivatives thereof; and any one of *n*₃, *n*₄, and *n*₅ is greater than 2, or any one of R₃ and R₄ includes at least one carbon atom. In some embodiments, the A cation is selected from at least one of ions of Formula (I) and derivatives thereof, and any one of *n*₁ and *n*₂ is greater than 2; or the A cation is selected from at least one of ions of Formula (II) and derivatives thereof, and any one of *n*₃, *n*₄, and *n*₅ is greater than 2. In some embodiments, the A cation is selected from at least one of ions of Formula (I) and derivatives thereof, and any one of R₁ and R₂ includes at least one carbon atom; or the A cation is selected from at least one of ions of Formula (II) and derivatives thereof, and any one of R₃ and R₄ includes at least one carbon atom.

In some embodiments, the A cation is selected from at least one of ions of Formula (I) and derivatives thereof, and any one of R₁ and R₂ is methyl; or the A cation is selected from at least one of ions of Formula (II) and derivatives thereof, and any one of R₃ and R₄ is methyl.

In some embodiments, the A cation is selected from at least one of ions of Formula (I) and derivatives thereof, and any one of *n*₁ and *n*₂ is 2. In some embodiments, the A cation is selected from at least one of ions of Formula (I) and derivatives thereof, and both of *n*₁ and *n*₂ are 2. In some embodiments, the A cation is selected from at least one of ions of Formula (I) and derivatives thereof, and *n*₁ is 2, *n*₂ is greater than or equal to 2. In some embodiments, the A cation is selected from at least one of Formula (I) ions and derivatives thereof, and any one of *n*₁ and *n*₂ is 3. In some embodiments, the A cation is selected from at least one of ions of Formula (II) and derivatives thereof, and any one of *n*₃, *n*₄, and *n*₅ is 2. In some embodiments, the A cation is selected from at least one of ions of Formula (II) and derivatives thereof, and *n*₃, *n*₄, and *n*₅ are all 2.

In some embodiments, the A cation is selected from any one of 1,4-diazabicyclo[2.2.2]octane-1,4-diium (Formula (III)),1-hydroxy-1,4-diazabicyclo[2.2.2]octane-1,4-diium (Formula (IV)), 1,4-dihydroxy-1,4-diazabicyclo[2.2.2]octane-1,4-diium (Formula (V)), pyrazine-1,4-diium (Formula (VI)), piperazine-1,4-diium (Formula (VII)), 1-methylpiperazine-1,4-diium(Formula (VIII)), 1-methyl-1,4-diazabicyclo[2.2.2]octane-1,4-diium (Formula (IX)), and 1,4-diazepane-1,4-diium (Formula (X)), and derivatives thereof.

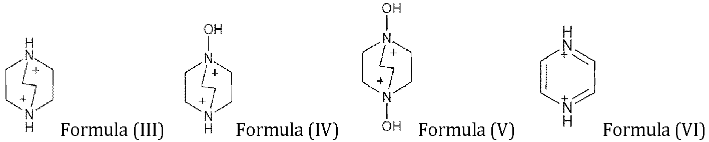

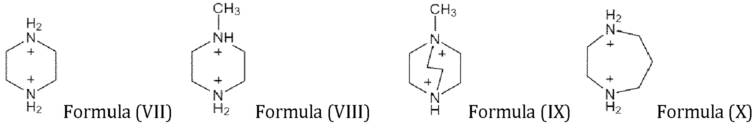

In some embodiments, the A cation is selected from at least one of piperazine-1,4-diium, 1-methylpiperazine-1,4-diium, 1,4-diazabicyclo[2.2.2]octane-1,4-diium, 1-hydroxy-1,4-diazabicyclo[2.2.2]octane-1,4-diium, 1-methyl-1,4-diazabicyclo[2.2.2]octane-1,4-diium, and 1,4-diazepane-1,4-diium, and derivatives thereof.

In some embodiments, the A cation is selected from at least one of piperazine-1,4-diium, and 1,4-diazepane-1,4-diium, and derivatives thereof.

In some embodiments, the A cation is selected from at least one of piperazine-1,4-diium, 1-methylpiperazine-1,4-diium, 1,4-diazabicyclo[2.2.2]octane-1,4-diium, and 1,4-diazepane-1,4-diium, and derivatives thereof.

In some embodiments, the A cation is selected from at least one of 1-methylpiperazine-1,4-diium, 1-hydroxy-1,4-diazabicyclo[2.2.2]octane-1,4-diium, 1-methyl-1,4-diazabicyclo[2.2.2]octane-1,4-diium, and 1,4-diazepane-1,4-diium, and derivatives thereof. In some embodiments, the A cation is selected from at least one of 1-methylpiperazine-1,4-diium, 1-hydroxy-1,4-diazabicyclo[2.2.2]octane-1,4-diium, and 1,4-diazepane-1,4-diium, and derivatives thereof.

In some embodiments, the A cation is selected from at least one of 1-methylpiperazine-1,4-diium, 1-methyl-1,4-diazabicyclo[2.2.2]octane-1,4-diium, and 1,4-diazepane-1,4-diium, and derivatives thereof. In some embodiments, the A cation is selected from at least one of 1-methylpiperazine-1,4-diium, and 1-methyl-1,4-diazabicyclo[2.2.2]octane-1,4-diium, and derivatives thereof. In some embodiments, the A cation is selected from at least one of 1-methylpiperazine-1,4-diium, and 1,4-diazepane-1,4-diium, and derivatives thereof. In some embodiments, the A cation is selected from at least one of 1-methyl-1,4-diazabicyclo[2.2.2]octane-1,4-diium, and 1,4-diazepane-1,4-diium, and derivatives thereof.

In some embodiments, the A cation is selected from at least one of 1,4-diazabicyclo[2.2.2]octane-1,4-diium, and derivatives thereof. In some embodiments, the A cation is selected from at least one of 1-hydroxy-1,4-diazabicyclo[2.2.2]octane-1,4-diium, and derivatives thereof. In some embodiments, the A cation is selected from at least one of 1,4-dihydroxy-1,4-diazabicyclo[2.2.2]octane-1,4-diium, and derivatives thereof. In some embodiments, the A cation is selected from at least one of pyrazine-1,4-diium, and derivatives thereof. In some embodiments, the A cation is selected from at least one of piperazine-1,4-diium, and derivatives thereof. In some embodiments, the A cation is selected from at least one of 1-methylpiperazine-1,4-diium, and derivatives thereof. In some embodiments, the A cation is selected from at least one of 1-methyl-1,4-diazabicyclo[2.2.2]octane-1,4-diium, and derivatives thereof. In some embodiments, the A cation is selected from at least one of 1,4-diazepane-1,4-diium, and derivatives thereof.

In some embodiments, the B cation and/or A' cation is monovalent cation.

In some embodiments, the B cation is selected from at least one of sodium ion, potassium ion, rubidium ion, cesium ion, silver ion, ammonium ion, hydroxylammonium ion, and hydrazinium ion. In some embodiments, the B cation is selected from at least one of sodium ion, potassium ion, rubidium ion, cesium ion, silver ion, and ammonium ion. In some embodiments, the B cation is selected from at least one of potassium ion, silver ion, and ammonium ion. In some embodiments, the B cation is selected from at least one of potassium ion and silver ion. In some embodiments, the B cation is selected from at least one of silver ion and ammonium ion. In some embodiments, the B cation is selected from at least one of potassium ion and ammonium ion. In some embodiments, the B cation is sodium ion. In some embodiments, the B cation is potassium ion. In some embodiments, the B cation is rubidium ion. In some embodiments, the B cation is cesium ion. In some embodiments, the B cation is silver ion. In some embodiments, the B cation is ammonium ion. In some embodiments, the B cation is selected from at least one of hydroxylammonium ion and hydrazinium ion. In some embodiments, the B cation is hydroxylammonium ion. In some embodiments, the A cation is hydrazinium ion.

In some embodiments, the B cation is at least one of alkali metal ion and monovalent nitrogen-containing cation. In some embodiments, the B cation is at least one of alkali metal ion and ammonium ion. In some embodiments, the B cation is monovalent nitrogen-containing cation. In some embodiments, the B cation has the general formula NR₃R₄R₅R₆⁺, wherein R₃, R₄, R₅, R₆ may be selected from at least one of hydrogen, methyl, ethyl, propyl, isopropyl, butyl, isobutyl, sec-butyl, *tert-*butyl, hydroxyl, carbonyl, carboxyl, amino, halogen, sulfhydryl, peroxy, diazenyl, and nitro groups.

In some embodiments, the A' cation is selected from at least one of sodium ion, potassium ion, rubidium ion, cesium ion, silver ion, ammonium ion, hydroxylammonium ion, and hydrazinium ion. In some embodiments, the A' cation is selected from at least one of sodium ion, potassium ion, rubidium ion, cesium ion, silver ion, and ammonium ion. In some embodiments, the A' cation is selected from at least one of potassium ion, silver ion, and ammonium ion. In some embodiments, the A' cation is selected from at least one of potassium ion and silver ion. In some embodiments, the A' cation is selected from at least one of silver ion and ammonium ion. In some embodiments, the A' cation is selected from at least one of potassium ion and ammonium ion. In some embodiments, the A' cation is sodium ion. In some embodiments, the A' cation is potassium ion. In some embodiments, the A' cation is rubidium ion. In some embodiments, the A' cation is cesium ion. In some embodiments, the A' cation is silver ion. In some embodiments, the A' cation is ammonium ion. In some embodiments, the A' cation is selected from at least one of hydroxylammonium ion and hydrazinium ion. In some embodiments, the A' cation is hydroxylammonium ion. In some embodiments, the A' cation is hydrazinium ion.

In some embodiments, the A' cation is at least one of alkali metal ion and monovalent nitrogen-containing cation. In some embodiments, the A' cation is at least one of alkali metal ion and ammonium ion. In some embodiments, the A' cation is divalent nitrogen-containing organic cation. In some embodiments, the A' cation has the general formula NR₃R₄R₅R₆⁺, wherein R₃, R₄, R₅, R₆ may be selected from at least one of hydrogen, methyl, ethyl, propyl, isopropyl, butyl, isobutyl, *sec-*butyl, *tert*-butyl, hydroxyl, carbonyl, carboxyl, amino, halogen, sulfhydryl, peroxy, diazenyl, and nitro groups.

In some embodiments, the B' cation is aliphatic diammonium cation. In some embodiments, the B' cation is saturated aliphatic diammonium cation. In some embodiments, the B' cation is alkyldiammonium cation. In some embodiments, the B' cation is linear alkyl diammonium cation. In some embodiments, the B' cation has the general formula R₇R₈R₉N⁺- (C_{*n*₃}H_{2*n*₃}]-N⁺R₁₀R₁₁R₁₂, where *n*₃ may be a positive integer preferably 1, 2, 3, 4, or 5, more preferably 1, 2, or 3, more preferably 2, and R₇, R₈, R₉, R₁₀, R₁₁, R₁₂ may be selected from at least one of hydrogen, methyl, ethyl, propyl, isopropyl, butyl, isobutyl, *sec*-butyl, and *tert*-butyl. In some embodiments, one, two, three, four, five, or all of R₇, R₈, R₉, R₁₀, R₁₁, R₁₂ in the B' cation is hydrogen. In some embodiments, in the B' cation, *n*₃ is 1. In some embodiments, in the B' cation, *n*₃ is 2. In some embodiments, in the B' cation, *n*₃ is 3. In some embodiments, the B' cation includes ethylene diamine cation. In some embodiments, the B' cation is ethylenediammonium cation.

In some embodiments, the B cation is selected from potassium ion, and the A cation is selected from at least one of piperazine-1,4-diium, and 1,4-diazepane-1,4-diium, and derivatives thereof. In some embodiments, the B cation is selected from potassium ion and the A cation is selected from at least one of piperazine-1,4-diium, and derivatives thereof.

In some embodiments, the B cation is selected from silver ion and the A cation is selected from at least one of piperazine-1,4-diium, 1-methylpiperazine-1,4-diium, 1,4-diazabicyclo[2.2.2]octane-1,4-diium, and 1,4-diazepane-1,4-diium, and derivatives thereof. In some embodiments, the B cation is selected from silver ion and the A cation is selected from at least one of piperazine-1,4-diium, and 1,4-diazepane-1,4-diium, and derivatives thereof. In some embodiments, the B cation is selected from silver ion, and the A cation is selected from at least one of 1-methylpiperazine-1,4-diium, and 1,4-diazepane-1,4-diium, and derivatives thereof.

In some embodiments, the B cation is selected from ammonium ion and the A cation is selected from at least one of 1-methylpiperazine-1,4-diium, 1-hydroxy-1,4-diazabicyclo[2.2.2]octane-1,4-diium, 1-methyl-1,4-diazabicyclo[2.2.2]octane-1,4-diium, and 1,4-diazepane-1,4-diium, and derivatives thereof. In some embodiments, the B cation is selected from ammonium ion, and the A cation is selected from at least one of 1-methylpiperazine-1,4-diium, 1-hydroxy-1,4-diazabicyclo[2.2.2]octane-1,4-diium, and 1,4-diazepane-1,4-diium, and derivatives thereof. In some embodiments, the B cation is selected from ammonium ion and the A cation is selected from at least one of 1-methylpiperazine-1,4-diium, 1-methyl-1,4-diazabicydo[2.2.2]octane-1,4-diium, and 1,4-diazepane-1,4-diium, and derivatives thereof. In some embodiments, the B cation is selected from ammonium ion, and the A cation is selected from at least one of 1-methylpiperazine-1,4-diium, and 1,4-diazepane-1,4-diium, and derivatives thereof.

In some embodiments, the compound has the general formula AB'X₄, the A cation is selected from at least one of piperazine-1,4-diium, 1-methylpiperazine-1,4-diium, and 1,4-diazepane-1,4-diium, and derivatives thereof, and the B' cation is ethylenediammonium cation. In some embodiments, the A cation is selected from at least one of piperazine-1,4-diium, 1-methylpiperazine-1,4-diium, and 1,4-diazepane-1,4-diium, and the B' cation is ethylenediammonium cation. In some embodiments, the A cation is at least one of piperazine-1,4-diium, and 1-methylpiperazine-1,4-diium, and the B' cation is ethylenediammonium cation. In some embodiments, the A cation is at least one of piperazine-1,4-diium, and 1,4-diazepane-1,4-diium, and the B' cation is ethylenediammonium cation. In some embodiments, the A cation is at least one of 1-methylpiperazine-1,4-diium, and 1,4-diazepane-1,4-diium, and the B' cation is ethylenediammonium cation. In some embodiments, the A cation is piperazine-1,4-diium and the B' cation is ethylenediammonium cation. In some embodiments, the A cation is 1-methylpiperazine-1,4-diium and the B' cation is ethylenediammonium cation. In some embodiments, the A cation is 1,4-diazepane-1,4-diium and the B' cation is ethylenediammonium cation.

In some embodiments, the compound has the formula B'₂A'X₅, the A' cation is ammonium ion, and the B' cation is ethylenediamine cation.

In some embodiments, the X anion is an anionic energetic ligand. In some embodiments, the X anion is monovalent anion.

In some embodiments, the X anion is selected from at least one of chlorate ion, bromate ion, iodate ion, perchlorate ion, perbromate ion, periodate ion, nitrate ion, fulminate ion, diazenyl group, and azide ion. In some embodiments, the X anion is selected from at least one of chlorate ion, perchlorate ion, nitrate ion, fulminate ion, diazenyl group, and azide ion.

In some embodiments, the X anion is halogen-containing monovalent anion. In some embodiments, the X anion is halogen-containing monovalent oxyacid anion. In some embodiments, the X anion is at least one of halate ion and perhalate ion. In some embodiments, the X anion is at least one of perhalate ion and nitrate ion. In some embodiments, the X anion is perhalate ion. In some embodiments, the X anion is selected from at least one of chlorate, bromate, iodate, perchlorate, perbromate, periodate, and nitrate. In some embodiments, the X anion is selected from at least one of chlorate ion, bromate ion, iodate ion, perchlorate ion, perbromate ion, and periodate ion. In some embodiments, the X anion is selected from at least one of perchlorate, perbromate and periodate. In some embodiments, the X anion is selected from at least one of perchlorate ion and perbromate ion. In some embodiments, the X anion is selected from at least one of perchlorate ion and periodate ion. In some embodiments, the X anion is selected from at least one of perbromate ion and periodate ion. In some embodiments, the X anion is perchlorate ion. In some embodiments, the X anion is perbromate ion. In some embodiments, the X anion is periodate ion.

In some embodiments, the X anion is perchlorate ion, the B cation is potassium ion, and the A cation are piperazine-1,4-diium, and 1,4-diazepane-1,4-diium. In some of the test examples of these examples, the compound has application advantages as a dynamite or pyrotechnics, the compound having a purple flame reaction; when the A cation is 1,4-dihydro-piperazine ammonium ion, the density of PAP-2 is up to 2.02 g/cm³, the energy density is 3.04 kcal/cm³, the detonation velocity is up to 8.78 km/s, and the detonation pressure is up to 36.6 GPa; when the A cation is 1,4-diazepane-1,4-diium, the density of PAP-H2 is up to 1.96 g/cm³, the energy density is 2.49 kcal/cm³, the detonation velocity is up to 8.17 km/s, and the detonation pressure is up to 31.1 GPa.

In some embodiments, the X anion is perchlorate ion, the B cation is potassium ion, and the A cation is piperazine-1,4-diium.

In some embodiments, the X anion is perchlorate ion, the B cation is silver ion, and the A cation is selected from at least one of piperazine-1,4-diium, 1-methylpiperazine-1,4-diium, 1,4-diazabicyclo[2.2.2]octane-1,4-diium, and 1,4-diazepane-1,4-diium. In some of the test examples of these examples, the compounds have application advantages as dynamites or priming explosives, the energetic compounds of the present invention have a higher crystal density, the crystal density is in the range of 2.35-2.50 g/cm³, and the theoretical heat of detonation can be up to 1.14-1.29 kcal/g; according to the Kamlet-Jacob formula, the theoretical detonation velocity can be up to 8.54-8.96 km/s, and the theoretical detonation pressure can be up to 38.0-42.4 GPa. The thermal decomposition temperatures of the compounds are more than 308.3-341.6°C. The impact sensitivity and friction sensitivity of the compound are more sensitive; when the A cation is piperazine-1,4-diium, the density of PAP-5 is up to 2.50 g/cm³, the energy density is up to 2.91 kcal/cm³, the theoretical detonation velocity is up to 8.96 km/s, and the theoretical detonation pressure is up to 42.4 GPa; when A cation is 1-methylpiperazine-1,4-diium, the density of PAP-M5 is up to 2.35 g/cm³, the energy density is up to 3.05 kcal/cm3, the theoretical detonation velocity is up to 8.73 km/s, and the theoretical detonation pressure is up to 39.2 GPa; where the A cation is 1,4-diazabicyclo[2.2.2]octane-1,4-diium, the impact sensitivity of DAP-5 is 3 J and the friction sensitivity is ≤ 5 N.

In some embodiments, the X anion is perchlorate ion, the B cation is silver ion, and the A cation is selected from at least one of piperazine-1,4-diium, 1-methylpiperazine-1,4-diium, and 1,4-diazepane-1,4-diium. In some embodiments, the X anion is perchlorate ion, the B cation is silver ion, and the A cation is selected from at least one of piperazine-1,4-diium, 1-methylpiperazine-1,4-diium, and 1,4-diazabicyclo[2.2.2]octane-1,4-diium. In some embodiments, the X anion is perchlorate ion, the B cation is silver ion, and the A cation is selected from at least one of 1,4-diium, 1,4-diazabicyclo[2.2.2]octane-1,4-diium, and 1,4-diazepane-1,4-diium. In some embodiments, the X anion is perchlorate ion, the B cation is silver ion, and the A cation is selected from at least one of 1-methylpiperazine-1,4-diium, 1,4-diazabicyclo[2.2.2]octane-1,4-diium, and 1,4-diazepane-1,4-diium. In some embodiments, the X anion is perchlorate ion, the B cation is silver ion, and the A cation is selected from at least one of piperazine-1,4-diium, and 1,4-diazepane-1,4-diium. In some embodiments, the X anion is perchlorate ion, the B cation is silver ion, and the A cation is selected from at least one of 1-methylpiperazine-1,4-diium, and 1,4-diazepane-1,4-diium. In some embodiments, the X anion is perchlorate ion, the B cation is silver ion, and the A cation is selected from at least one of piperazine-1,4-diium, and 1-methylpiperazine-1,4-diium. In some embodiments, the X anion is perchlorate ion, the B cation is silver ion, and the A cation is selected from at least one of piperazine-1,4-diium, and 1,4-diazabicycio[2.2.2]octane-1,4-diium. In some embodiments, the X anion is perchlorate ion, the B cation is silver ion, and the A cation is selected from at least one of 1,4-diazabicyclo[2.2.2]octane-1,4-diium, and 1,4-diazepane-1,4-diium. In some embodiments, the X anion is perchlorate ion, the B cation is silver ion, and the A cation is selected from at least one of 1-methylpiperazine-1,4-diium, and 1,4-diazabicyclo[2.2.2]octane-1,4-diium.

In some embodiments, the X anion is perchlorate ion, the B cation is ammonium ion, and the A cation is selected from at least one of 1-methylpiperazine-1,4-diium, 1-hydroxy-1,4-diazabicyclo[2.2.2]octane-1,4-diium, 1-methyl-1,4-diazabicyclo[2.2.2]octane-1,4-diium, and 1,4-diazepane-1,4-diium. Among the test examples of these examples, the compounds have application advantages as dynamites or solid propellants, the energetic compounds of the present invention have a high energy density, a theoretical heat of detonation up to 1.19-1.48 kcal/g, and a crystal density in the range of 1.74-1.85 g/cm³; according to the Kamlet-Jacob formula, the theoretical detonation velocity can be up to 8.08-8.90 km/s, and the theoretical detonation pressure can be up to 28.8-35.7 GPa. The thermal stability is good and the decomposition temperature is more than 288.2-364.0°C. There is a higher theoretical specific impulse, the formation enthalpies of the compounds were deduced according to DFT and the designed explosive decomposition reaction, and the theoretical specific impulse calculated by EXPLO5 can be up to 225. 3-264. 2 s. When the A cation is 1-hydroxy-4-hydro-1,4-diazabicyclo[2.2.2]octane ammonium ion, the theoretical heat of detonation of DAP-O4 compound is up to 1.48 kcal/g, and the corresponding energy density is up to 2.74 kcal/cm³. When the A cation is 1,4-dihydro-piperazine ammonium ion, the theoretical specific impulse of PAP-4 compound is up to 264.2 s.

In some embodiments, the X anion is perchlorate ion, the B cation is ammonium ion, and the A cation is selected from at least one of 1-methylpiperazine-1,4-diium, 1-hydroxy-1,4-diazabicyclo[2.2.2]octane-1,4-diium, and 1,4-diazepane-1,4-diium. In some embodiments, the X anion is perchlorate ion, the B cation is ammonium ion, and the A cation is selected from at least one of 1-methylpiperazine-1,4-diium, 1-methyl-1,4-diazabicyclo[2.2.2]octane-1,4-diium, and 1,4-diazepane-1,4-diium. In some embodiments, the X anion is perchlorate ion, the B cation is ammonium ion, and the A cation is selected from at least one of 1-methylpiperazine-1,4-diium, and 1,4-diazepane-1,4-diium.

In some embodiments, A is selected from at least one of 1-methyl-1,4-diazabicyclo[2.2.2]octane-1,4-diium, and derivatives thereof; B is selected from at least one of sodium ion, potassium ion, rubidium ion, cesium ion, silver ion, and ammonium ion. X is selected from at least one of chlorate ion, bromate ion, iodate ion, perchlorate ion, perbromate ion, and periodate ion. In some embodiments, A is selected from at least one of 1-methyl-1,4-diazabicyclo[2.2.2]octane-1,4-diium, and derivatives thereof; B is selected from at least one of potassium ion, rubidium ion, silver ion, and ammonium ion. X is selected from at least one of chlorate ion, bromate ion, iodate ion, perchlorate ion, perbromate ion, and periodate ion. In some embodiments, A is selected from at least one of 1-methyl-1,4-diazabicyclo[2.2.2]octane-1,4-diium, and derivatives thereof; B is selected from at least one of potassium ion, rubidium ion, silver ion, and ammonium ion. X is selected from chlorate ion.

In some embodiments, the A cation is 1,4-diazabicyclo[2.2.2]octane-1,4-diium, the B cation is selected from at least one of hydroxylammonium ion and hydrazinium ion,and the X anion is selected from perchlorate ion. In some embodiments, the A cation is 1,4-diazabicyclo[2.2.2]octane-1,4-diium, the B cation is hydrazinium ion, and the X anion is selected from perchlorate ion. In some embodiments, the A cation is 1,4-diazabicyclo[2.2.2]octane-1,4-diium, the B cation is hydroxylammonium ion, and the X anion is selected from perchlorate ion. In some test examples of these examples, hydroxylammonium ion and hydrazinium ion have higher enthalpies of formation and are expected to form more hydrogen bonding interaction in the crystal, or will contribute to a stronger binding force to increase both enthalpy of formation and crystal density of the compound. In some test examples of these examples, the theoretical heat of detonation is up to 1.52, 1.43 kcal/g, the room temperature densities are up to 1.90, 1.87 g/cm³, respectively, and the corresponding volumetric energy densities are up to 2.89, 2.67 kcal/cm³, respectively; according to the Kamlet-Jacob formula, the theoretical detonation velocities are up to 9.12 km/s and 8.88 km/s, and the theoretical detonation pressures are up to 38.1 GPa and 35.8 GPa. The decomposition peak temperature is 246.1°C and 376.1°C, and the theoretical specific impulse is 265.3 s and 256.9 s.

In some embodiments, the compound has the general formula AB'X₄, the A cation is selected from at least one of piperazine-1,4-diium, 1-methylpiperazine-1,4-diium, and 1,4-diazepane-1,4-diium, and derivatives thereof, the B' cation is ethylenediammonium cation, and the Xanion is perchlorate ion. In some embodiments, the A cation is selected from at least one of piperazine-1,4-diium, 1-methylpiperazine-1,4-diium, and 1,4-diazepane-1,4-diium, the B' cation is ethylenediammonium cation, and the X anion is perchlorate ion. In some embodiments, the A cation is at least one of piperazine-1,4-diium, and 1-methylpiperazine-1,4-diium, the B' cation is ethylenediammonium cation, and the X anion is perchlorate ion. In some embodiments, the A cation is at least one of piperazine-1,4-diium and 1,4-diazepane-1,4-diium, the B' cation is ethylenediammonium cation, and the X anion is perchlorate ion. In some embodiments, the A cation is at least one of 1-methylpiperazine-1,4-diium, and 1,4-diazepane-1,4-diium, the B' cation is ethylenediammonium cation, and the X anion is perchlorate ion. In some embodiments, the A cation is piperazine-1,4-diium, the B' cation is an ethylene diaminium cation, and the X anion is perchlorate ion. In some embodiments, the A cation is 1-methylpiperazine-1,4-diium, the B' cation is ethylenediammonium cation, and the X anion is perchlorate ion. In some embodiments, the A cation is 1,4-diazepane-1,4-diium,the B' cation is ethylenediammonium cation, and the Xanion is perchlorate ion. In some embodiments, the compound has the general formula B'₂A'X₅, the A' cation is ammonium ion, the B' cation is an ethylene diaminium cation, and the X anion is perchlorate ion. In some test examples of these examples, the theoretical heat of detonation and volumetric energy density are high, the oxygen balance is high, the crystallographic density value is high, the detonation performance is superior, it is sensitive to friction but insensitive to impact, the safety performance is good, the moisture absorption is small and the thermal stability is good, it can be stored for a long time without decomposition; the room temperature crystallinity is single, the raw materials are cheap and readily available, the production process is simple, and can be prepared in large quantities safely. Specifically, in some test examples of these examples, the theoretical heat of detonation is up to 1.40 kcal/g, the crystal density at room temperature is up to 1.91 g/cm³, and the oxygen balance parameter value is up to 6.25%; according to Kamlet-Jacob formula, the theoretical detonation velocity is up to 9.09 km/s. The theoretical burst pressure is up to 37.6 GPa. The decomposition peak temperature is up to 376.0°C, and the specific impulse is up to 264.2 s. More sensitive to friction but less sensitive to impact, friction sensitivity (FS) is between 9 N and 36 N and impact sensitivity (IS) is between 9 J and 20 J.

In some embodiments, the compounds of the present application include optionally one, two or more of the A cations described above or optionally one, two or more of the A' cations described above, optionally one, two or more of the B cations described above or optionally one, two or more of the B' cations described above, and/or optionally one, two or more of the X anions described above.

In some embodiments, in the compound ABX₃, the sum of the valency of the A cation and the valency of the B cation is three times the valency of the X anion. In some embodiments, the sum of the valency of the A cation and the valency of the B' cation in the compound AB'X₄ is four times the valency of the X anion. In some embodiments, in the compound B'₂A'X₅, the sum of the valency of the A' cation and twice the valency of the B' cation is five times the valency of the X anion.

In some embodiments, a compound of the present application is compound ABX₃, compound AB'X₄, or is compound B'₂A'X₅ consisting of an A cation or A' cation, a B cation or B' cation, and an X anion, wherein the A cation is a nitrogen-containing organic cation, the B cation and the A' cation are cation, the B' cation is saturated aliphatic diammonium cation, and the X anion is an anionic energetic ligand.

In some embodiments, compound of the present application is compound ABX₃ consisting of A cation, B cation, and X anion, wherein the A cation is nitrogen-containing organic cation, the B cation is cation, and the X anion is anionic energetic ligand; or is compound AB'X₄ consisting of A cation, B' cation and X anion or is compound B'₂A'X₅ consisting of A' cation, B' cation and X anion, wherein the A cation is a divalent nitrogen-containing monocyclic heterocyclic cation, the A' cation is at least one of alkali metal ion and monovalent nitrogen-containing cation, the B' cation is saturated aliphatic diammonium cation, and the X anion is an anionic energetic ligand.

In at least some examples of the present application, the compounds of the present application, such as compound ABX₃, compound AB'X₄, or is compound B'₂A'X₅, may be obtained by reacting the corresponding component A or component A', component B or component B', and component X in any order in a liquid reaction system; the liquid reaction system is preferably a polar solvent that may dissolve the component A or component A', the component B or component B', and the component X. Or obtained by referencing known synthesis methods. The reaction temperature is not particularly limited and may be adjusted within a wide range, for example, from 0 to 100°C.

In some embodiments, a method of preparing a compound may include the steps of:
1) mixing the component A or component A', the component B or component B', and the component X in any order in a liquid reaction system; and
2) obtaining a solid product produced in the liquid reaction system; wherein further purification is preferred.

The liquid reaction system is preferably a polar solvent that may dissolve the component A or component A', the component B or component B', and the component X. Or obtained by referencing known synthesis methods. The reaction temperature may range, for example, from 0°C to 100°C, e.g. may be room temperature or 25°C, e.g. may range from 15°C to 40°C, or from 20°C to 30°C, *etc.*

In some embodiments, the component A or component A', the component B or component B', and the component X may be mixed by thorough stirring in the liquid reaction system. In some embodiments, purification is carried out by filtering the solid product produced in the liquid reaction system and by washing the filter residue with ethanol or a similar organic solvent and drying under vacuum.

In some embodiments, a method of preparing a compound may include the steps of:
1) synthesizing the solution of component A or component A', *i.e.* the solution containing the A cation or A' cation in the compound to be synthesized;
2) mixing the solution of component A or component A', the component B or component B', and the component X in any order in a liquid reaction system; and
3) taking the solid product produced in the liquid reaction system and purifying same.

In some embodiments, a method of preparing a compound may include the steps of:
1) synthesizing the solution of component B or component B', *i.e.* the solution containing the B cation or B cation in the compound to be synthesized;
2) mixing the solution of component A or component A', the component B or component B', and the component X in any order in a liquid reaction system; and
3) taking the solid product produced in the liquid reaction system and purifying same.

In some embodiments, a method of preparing a compound may include the steps of:
1) synthesizing the solution of component A or component A', *i.e.* the solution containing the A cation or A' cation in the compound to be synthesized;
2) synthesizing the solution of component B or component B', *i.e.* the solution containing the B cation or B cation in the compound to be synthesized;
3) mixing the solution of component A or component A', the component B or component B', and the component X in any order in a liquid reaction system; and
4) taking the solid product produced in the liquid reaction system and purifying same.

In some embodiments, the component A or component A', and the component B or component B', are solids dissolved with a polar solvent, and the method of preparing the compound may include the steps of:
1) dissolving the component A or component A', and the component B or component B', separately or together with a polar solvent to obtain a solution of component A or component A' and a solution of component B or component B';
2) mixing the solution of component A or component A', the solution of component B or component B', and the component X in any order; and
3) taking the solid product produced in the liquid reaction system and purifying same.

In some embodiments, a method of preparing a compound may include the steps of:
1) adding the component A or component A' into a polar solvent, then adding the component X, and stirring until uniform to obtain a solution of the component A or component A' and the component X;
2) dissolving the component B or component B' in a polar solvent to obtain a solution of component B or component B'; and
3) mixing the component A or component A' and component X solution and the solution of component B or component B', thoroughly stirring same, filtering, washing the filter residue with ethanol and drying same under vacuum to obtain the compound as a white powder.

In some embodiments, the component A, the component A', the component B, and/or the component B' is a salt that includes the A cation, the A' cation, the B cation, and/or the B' cation in the compound to be synthesized, or a solution that contains the A cation in the compound to be synthesized, or the component A, component A', component B, and/or component B' is a deprotonated product of the A cation, the A' cation, the B cation, and/or the B' cation, *i.e*. the protonated product of the component A, the component A', the component B and/or the component B' is the A cation, the A' cation, the B cation and/or the B' cation.

In some embodiments, the A component is a nitrogen-containing heterocyclic compound. In some embodiments, the A component is a divalent nitrogen-containing organic compound or a salt thereof. In some embodiments, the A component is a divalent nitrogen-containing heterocyclic compound or a salt thereof. In some embodiments, the A component is selected from at least one of a divalent nitrogen-containing five-membered ring, a divalent nitrogen-containing six-membered ring, and a divalent nitrogen-containing seven-membered ring, or salts thereof, including onium salts thereof.

In some embodiments, the A component is selected from at least one of compounds of Formula (XI), compounds of Formula (XII), salts of ions of Formula (I) including onium salts thereof, and salts of ions of Formula (II) including onium salts thereof, and derivatives thereof, wherein *n*₁, *n*₂*, n*₃, *n*₄, and *n*₅ each may be a positive integer of preferably 1, 2, 3, 4, or 5, more preferably 1, 2, or 3, R₁, R₂, R₃, and R₄ may be selected from at least one of hydrogen, methyl, ethyl, propyl, isopropyl, butyl, isobutyl, *sec*-butyl, *tert*-butyl, hydroxyl, carbonyl, carboxyl, amino, halogen, mercapto, peroxy, diazenyl, and nitro groups, the derivatives refer to the organic cationic moiety in which hydrogen atoms are substituted, simultaneously or not, with substituents, and common substituents include methyl, ethyl, propyl, isopropyl, butyl, isobutyl, *sec*-butyl, *tert*-butyl, hydroxyl, carbonyl, carboxyl, amino, halogen, mercapto, peroxy, diazenyl, nitro, *etc.*

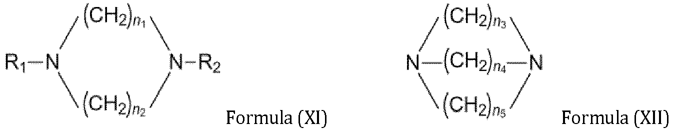

In some embodiments,
the A component is selected from at least one of compounds of Formula (XI), and salts of Formula (I) including onium salts thereof, and derivatives thereof, and
any one of *n*₁ and *n*₂ is greater than 2, or any one of R₁ and R₂ includes at least one carbon atom;
or
the A component is selected from at least one of compounds of Formula (XII), and salts of Formula (I) including onium salts thereof, and derivatives thereof, and
any one of *n*₃, *n*₄, and *n*₅ is greater than 2, or any one of R₃ and R₄ includes at least one carbon atom.

In some embodiments, the A component is selected from at least one of compounds of Formula (XI), and salts of ions of Formula (I) including onium salts thereof, and derivatives thereof, and any one of *n*₁ and *n*₂ is greater than 2, or any one of R₁ and R₂ includes at least one carbon atom. In some embodiments, the A component is selected from at least one of compounds of Formula (XII), and salts of ions of Formula (II) including onium salts thereof, and derivatives thereof, and any one of *n*₃, *n*₄, and *n*₅ is greater than 2, or any one of R₃ and R₄ includes at least one carbon atom. In some embodiments, the A component is selected from at least one of compounds of Formula (XI), and salts of ions of Formula (I) including onium salts thereof, and derivatives thereof, and any one of *n*₁ and *n*₂ is greater than 2; or the A component is selected from at least one of compounds of Formula (XII), and salts of ions of Formula (II) including onium salts thereof, and derivatives thereof, and any one of *n*₃, *n*₄, and *n*₅ is greater than 2. In some embodiments, the A component is selected from at least one of compounds of Formula (XI), and salts of ions of Formula (I) including onium salts thereof, and derivatives thereof, and any one of R₁ and R₂ includes at least one carbon atom; or the A component is selected from at least one of compounds of Formula (XII), and salts of ions of Formula (II) including onium salts thereof, and derivatives thereof, and any one of R₃ and R₄ includes at least one carbon atom. In some embodiments, the A component is selected from at least one of compounds of Formula (XI), and salts of ions of Formula (I) including onium salts thereof, and derivatives thereof, and any one of R₁ and R₂ is methyl; or the A component is selected from at least one of compounds of Formula (XII), and salts of ions of Formula (II) including onium salts thereof, and derivatives thereof, and any one of R₃ and R₄ is methyl.

In some embodiments, the A component is selected from at least one of compounds of Formula (XI), and salts of ions of Formula (I) including onium salts thereof, and derivatives thereof, and any one of *n*₁ and *n*₂ is 2. In some embodiments, the A component is selected from at least one of compounds of Formula (XI), and salts of ions of Formula (I) including onium salts thereof, and derivatives thereof, and *n*₁ and *n*₂ are both 2. In some embodiments, the A component is selected from at least one of compounds of Formula (XI), and salts of ions of Formula (I) including onium salts thereof, and derivatives thereof, and *n*₁ is 2, *n*₂ is greater than or equal to 2. In some embodiments, the A component is selected from compounds of Formula (XI) or salts of ions of Formula (I) including onium salts thereof, or derivatives thereof, and *n*₁ is 2, *n*₂ is greater than 2. In some embodiments, the A component is selected from at least one of compounds of Formula (XI), and salts of ions of Formula (I) including onium salts thereof, and derivatives thereof, and any one of *n*₁ and *n*₂ is 3. In some embodiments, the A component is selected from at least one of compounds of Formula (XII), and salts of ions of Formula (II) including onium salts thereof, and derivatives thereof, and any one of *n*₃, *n*₄, and *n*₅ is 2. In some embodiments, the A component is selected from at least one of compounds of Formula (XI), and salts of ions of Formula (II) including onium salts thereof, and derivatives thereof, and *n*₃, *n*₄, and *n*₅ are both 2.

In some embodiments, the A component is selected from at least one of 1,4-diazabicyclo[2.2.2]octane, a reaction product of 1,4-diazabicyclo[2.2.2]octane with hydrogen peroxide, pyrazine, piperazine, 1-methylpiperazine, 1,4-diazepane, 1,4-diazabicyclo[2.2.2]octane-1,4-diium, 1-hydroxy-1,4-diazabicyclo[2.2.2]octane-1,4-diium, 1,4-dihydroxy-1,4-diazabicyclo[2.2.2]octane-1,4-diium, pyrazine-1,4-diium, piperazine-1,4-diium, 1-methylpiperazine-1,4-diium, 1-methyl-1,4-diazabicyclo[2.2.2]octane-1,4-diium, and 1,4-diazepane-1,4-diium, and derivatives thereof.

In some embodiments, the A component is selected from at least one of piperazine, 1-methylpiperazine, 1,4-diazabicyclo[2.2.2]octane, a reaction product of 1,4-diazabicyclo[2.2.2]octane with hydrogen peroxide, and 1,4-diazepane, and derivatives thereof.

In some embodiments, the A component is selected from at least one of piperazine, 1,4-diazepane, piperazine-1,4-diium, and 1,4-diazepane-1,4-diium, and derivatives thereof.

In some embodiments, the A component is selected from at least one of piperazine, 1-methylpiperazine, 1,4-diazabicyclo[2.2.2]octane, 1,4-diazepane, piperazine-1,4-diium, 1-methylpiperazine-1,4-diium, 1,4-diazabicyclo[2.2.2]octane-1,4-diium, and 1,4-diazepane-1,4-diium, and derivatives thereof.

In some embodiments, the A component is selected from at least one of 1-methylpiperazine, 1,4-diazabicyclo[2.2.2]octane and hydrogen peroxide reaction products, 1,4-diazepane, 1-methylpiperazine-1,4-diium, 1-hydroxy-1,4-diazabicyclo[2.2.2]octane-1,4-diium, 1-methyl-1,4-diazabicyclo[2.2.2]octane-1,4-diium, and 1,4-diazepane-1,4-diium, and derivatives thereof. In some embodiments, the A component is selected from at least one of 1-methylpiperazine, 1,4-diazabicyclo[2.2.2]octane and hydrogen peroxide reaction products, 1,4-diazepane, 1-methylpiperazine-1,4-diium, 1-hydroxy-1,4-diazabicyclo[2.2.2]octane-1,4-diium, and 1,4-diazepane-1,4-diium, and derivatives thereof.

In some embodiments, the A component is selected from at least one of 1-methylpiperazine, 1,4-diazepane, 1-methylpiperazine-1,4-diium, 1-methyl-1,4-diazabicyclo[2.2.2]octane-1,4-diium, and 1,4-diazepane-1,4-diium, and derivatives thereof. In some embodiments, the A component is selected from at least one of 1-methylpiperazine, 1-methylpiperazine-1,4-diium, and 1-methyl-1,4-diazabicyclo[2.2.2]octane-1,4-diium, and derivatives thereof. In some embodiments, the A component is selected from at least one of 1-methylpiperazine, 1,4-diazepane, 1-methylpiperazine-1,4-diium, and 1,4-diazepane-1,4-diium, and derivatives thereof. In some embodiments, the A component is selected from at least one of 1,4-diazepane, 1-methyl-1,4-diazabicyclo[2.2.2]octane-1,4-diium, and 1,4-diazepane-1,4-diium, and derivatives thereof.

In some embodiments, the A component is selected from at least one of 1,4-diazabicyclo[2.2.2]octane, and derivatives thereof. In some embodiments, the A component is selected from at least one of 1-hydroxy-1,4-diazabicyclo[2.2.2]octane, and derivatives thereof. In some embodiments, the A component is selected from at least one of 1,4-dihydroxy-1,4-diazabicyclo[2.2.2]octane, and derivatives thereof. In some embodiments, the A component is selected from at least one of pyrazine, and derivatives thereof. In some embodiments, the A component is selected from at least one of piperazine, and derivatives thereof. In some embodiments, the A component is selected from at least one of 1-methylpiperazine, and derivatives thereof. In some embodiments, the A component is selected from at least one of 1,4-diazepane, and derivatives thereof. In some embodiments, the A component is selected from at least one of 1,4-diazabicyclo[2.2.2]octane-1,4-diium, and derivatives thereof. In some embodiments, the A component is selected from at least one of 1-hydroxy-1,4-diazabicyclo[2.2.2]octane-1,4-diium, and derivatives thereof. In some embodiments, the A component is selected from at least one of 1,4-dihydroxy-1,4-diazabicyclo[2.2.2]octane-1,4-diium, and derivatives thereof. In some embodiments, the A component is selected from at least one of pyrazine -1,4-diiums and derivatives thereof. In some embodiments, the A component is selected from at least one of piperazine-1,4-diium, and derivatives thereof. In some embodiments, the A component is selected from at least one of 1-methylpiperazine-1,4-diium, and derivatives thereof. In some embodiments, the A component is selected from at least one of 1-methyl-1,4-diazabicyclo[2.2.2]octane-1,4-diium, and derivatives thereof. In some embodiments, the A component is selected from at least one of 1,4-diazepane-1,4-diium, and derivatives thereof.

In some embodiments, the B or A' component is selected from at least one of salts and hydroxides including a B or A' cation in the compound to be synthesized, and/or the B or A' component produces the B or A' cation when dissolved in the liquid reaction system. In some embodiments, the B or A' component is selected from a base including a B or A' cation in the compound to be synthesized. In some embodiments, the B or A' component is selected from at least one of salts and hydroxides including a monovalent cation.

In some embodiments, the B component is selected from at least one of salts and hydroxides of potassium ion, silver ion, and ammonium ion. In some embodiments, the B component is selected from at least one of salts and hydroxides of potassium ion and silver ion. In some embodiments, the B component is selected from at least one of salts and hydroxides of silver ion and ammonium ion. In some embodiments, the B component is selected from at least one of salts and hydroxides of potassium ion and ammonium ion. In some embodiments, the B component is selected from at least one of potassium salts, potassium hydroxide, ammonium salts, ammonia, and silver salts. In some embodiments, the B component is at least one of alkali metal salts and hydroxides, and monovalent nitrogen-containing cation salts, and monovalent nitrogen-containing bases. In some embodiments, the B component is at least one of alkali metal salts and hydroxides, ammonium salts, and ammonia. In some embodiments, the B component is selected from at least one of potassium salts, potassium hydroxide, ammonium salts, ammonia, and silver salts. In some embodiments, the B component is selected from at least one of potassium salts, potassium hydroxide, ammonium salts, and ammonia. In some embodiments, the B component is selected from at least one of ammonium salts, ammonia, and silver salts. In some embodiments, the B component is selected from at least one of potassium salts, potassium hydroxide, and silver salts. In some embodiments, the B component is at least one of ammonium salts and ammonia. In some embodiments, the B component is at least one of sodium salts and sodium hydroxide. In some embodiments, the B component is at least one of potassium salts and potassium hydroxide. In some embodiments, the B component is at least one of rubidium salts and rubidium hydroxide. In some embodiments, the B component is at least one of cesium salts and cesium hydroxide. The ammonia may be selected from at least one of ammonia gas, ammonia water, and ammonia dissolved in other solvents. In some embodiments, the B component is a silver salt.

In some embodiments, the B component is at least one of hydroxylamine, hydroxylammonium ion-containing salts, hydroxylammonium ion-containing bases, hydrazine, ion-containing salts, and ion-containing bases. In some embodiments, the B component is at least one of hydroxylamine, hydroxylammonium salts, hydrazine, hydrazinium salts. In some embodiments, the B component is at least one of hydroxylamine and hydroxylammonium salts. In some embodiments, the B component is at least one of hydrazine and hydrazinium salts. In some embodiments, the B component is hydroxylamine. In some embodiments, the B component is hydrazine.

In some embodiments, the B component is at least one of monovalent nitrogen-containing cationic salts and monovalent nitrogen-containing bases. In some embodiments, the B component is at least one of salts, bases, and hydroxides of cations of the general formula NR₃R₄R₅R₆⁺, wherein R₃, R₄, R₅, R₆ may be selected from at least one of hydrogen, methyl, ethyl, propyl, isopropyl, butyl, isobutyl, *sec*-butyl, *tert*-butyl, hydroxyl, carbonyl, carboxyl, amino, halogen, mercapto, peroxy, diazenyl, and nitro groups; or a compound of a general formula NR₃R₄R₅.

In some embodiments, the A' component is selected from at least one of salts and hydroxides of potassium ion, silver ion, and ammonium ion. In some embodiments, the A' component is selected from at least one of salts and hydroxides of potassium ion and silver ion. In some embodiments, the A' component is selected from at least one of salts and hydroxides of silver ion and ammonium ion. In some embodiments, the A' component is selected from at least one of salts and hydroxides of potassium ion and ammonium ion. In some embodiments, the A' component is selected from at least one of potassium salts, potassium hydroxide, ammonium salts, ammonia, and silver salts. In some embodiments, the A' component is at least one of alkali metal salts and hydroxides, or at least one of monovalent nitrogen-containing cation salts and monovalent nitrogen-containing bases. In some embodiments, the A' component is at least one of alkali metal salts and hydroxides, ammonium salts, and ammonia. In some embodiments, the A' component is selected from at least one of potassium salts, potassium hydroxide, ammonium salts, ammonia, and silver salts. In some embodiments, the A' component is selected from at least one of potassium salts, potassium hydroxide, ammonium salts, and ammonia. In some embodiments, the A' component is selected from at least one of ammonium salts, ammonia, and silver salts. In some embodiments, the A' component is selected from at least one of potassium salts, potassium hydroxide, and silver salts. In some embodiments, the A' component is at least one of ammonium salts and ammonia. In some embodiments, the A' component is at least one of sodium salts and sodium hydroxide. In some embodiments, the A' component is at least one of potassium salts and potassium hydroxide. In some embodiments, the A' component is at least one of rubidium salts and rubidium hydroxide. In some embodiments, the A' component is at least one of cesium salts and cesium hydroxide. The ammonia may be selected from at least one of ammonia gas, ammonia water, and ammonia dissolved in other solvents. In some embodiments, the A' component is a silver salt.

In some embodiments, the A' component is at least one of hydroxylamine, hydroxylammonium ion-containing salts, hydroxylammonium ion-containing bases, hydrazine, ion-containing salts, and ion-containing bases. In some embodiments, the A' component is at least one of hydroxylamine, hydroxylammonium salts, hydrazine, hydrazinium salts. In some embodiments, the A' component is at least one of hydroxylamine and hydroxylammonium salts. In some embodiments, the A' component is at least one of hydrazine and hydrazinium salts. In some embodiments, the A' component is hydroxylamine. In some embodiments, the A' component is hydrazine.

In some embodiments, the A' component is at least one of monovalent nitrogen-containing cationic salts and monovalent nitrogen-containing bases. In some embodiments, the A' component is at least one of salts, bases, and hydroxides of cations of a general formula NR₃R₄R₅R₆⁺, wherein R₃, R₄, R₅, R₆ may be selected from at least one of hydrogen, methyl, ethyl, propyl, isopropyl, butyl, isobutyl,sec-butyl, tert-butyl, hydroxyl, carbonyl, carboxyl, amino, halogen, mercapto, peroxy, diazenyl, and nitro groups; or a compound of a general formula NR₃R₄R₅.

In some embodiments, the B' component is selected from at least one of salts and hydroxides including a B' cation in the compound to be synthesized, and/or the B' component produces the B' cation when dissolved in the liquid reaction system. In some embodiments, the B' component is selected from a base including a B' cation in the compound to be synthesized.

In some embodiments, the B' component is at least one of aliphatic diamine and aliphatic diammonium salts. In some embodiments, the B' component is at least one of saturated aliphatic diamine and saturated aliphatic diammonium salts. In some embodiments, the B' component is at least one of alkyldiamine and alkyldiammonium salts. In some embodiments, the B' component is at least one of linear alkyl diammonium and linear alkyl diamine salts. In some embodiments, the B' component is at least one of salts and hydroxides of ions of a general formula R₇R₈R₉N⁺-(C_{*n*₃}H_{2*n*₃})-N⁺R₁₀R₁₁R₁₂, wherein n₃ may be a positive integer preferably 1, 2, 3,4, or 5, more preferably 1, 2, or 3, more preferably 2, and R₇, R₈, R₉, R₁₀, R₁₁, R₁₂ may be selected from at least one of hydrogen, methyl, ethyl, propyl, isopropyl, butyl, isobutyl, *sec*-butyl, and *tert*-butyl. In some embodiments, the B' component is amines of a general formula R₇R₈N-(C_{*n*₃}H_{2*n*₃})-NR₁₀R₁₁, wherein n₃ may be a positive integer preferably 1, 2, 3, 4, or 5, more preferably 1, 2, or 3, more preferably 2, and R₇, R₈, R₉, R₁₀, R₁₁, R₁₂ may be selected from at least one of hydrogen, methyl, ethyl, propyl, isopropyl, butyl, isobutyl, *sec*-butyl, and *tert*-butyl. In some embodiments, one, two, three, four, five, or all of R₇, R₈, R₉, R₁₀, R₁₁, R₁₂ in the B' component is hydrogen. In some embodiments, in the B' component, n₃ is 1. In some embodiments, in the B' component, n₃ is 2. In some embodiments, in the B' component, n₃ is 3.

In some embodiments, the B' component includes at least one of ethylenediamine and ethylenediammonium salts. In some embodiments, the B' component includes ethylenediamine. In some embodiments, the B' component includes ethylenediammonium salts. In some embodiments, the B' component is ethylenediamine.

In some embodiments, the B component is selected from at least one of potassium salts and potassium hydroxide, and the A component is selected from at least one of piperazine, 1,4-diazepane, piperazine-1,4-diium, and 1,4-diazepane-1,4-diium, and derivatives thereof. In some embodiments, the B component is selected from at least one of potassium salt and potassium hydroxide, and the A component is selected from at least one of piperazine and piperazine-1,4-diium, and derivatives thereof.

In some embodiments, the B component is selected from a silver salt and the A component is selected from at least one of piperazine, 1-methylpiperazine, 1,4-diazabicyclo[2.2.2]octane, 1,4-diazepane, piperazine-1,4-diium, 1-methylpiperazine-1,4-diium, 1,4-diazabicyclo[2.2.2]octane-1,4-diium, and 1,4-diazepane-1,4-diium, and derivatives thereof. In some embodiments, the B component is selected from a silver salt and the A component is selected from at least one of piperazine, 1,4-diazepane, piperazine-1,4-diium, and 1,4-diazepane-1,4-diium, and derivatives thereof. In some embodiments, the B component is selected from a silver salt and the A component is selected from at least one of 1-methylpiperazine, 1,4-diazepane, 1-methylpiperazine-1,4-diium, and 1,4-diazepane-1,4-diium, and derivatives thereof.

In some embodiments, the B component is selected from at least one of ammonium salts and ammonia, and the A component is selected from 1-methylpiperazine, 1,4-diazabicyclo[2.2.2]octane reaction products with hydrogen peroxide, 1,4-diazepane, 1-methylpiperazine-1,4-diium, 1-hydroxy-1,4-diazabicyclo[2.2.2]octane-1,4-diium, 1-methyl-1,4-diazabicyclo[2.2.2]octane-1,4-diium, and 1,4-diazepane-1,4-diium, and derivatives thereof. In some embodiments, the B component is selected from at least one of ammonium salts and ammonia, and the A component is selected from at least one of 1-methylpiperazine, 1,4-diazabicyclo[2.2.2]octane reaction product with hydrogen peroxide, 1,4-diazepane, 1-methylpiperazine-1,4-diium, 1-hydroxy-1,4-diazabicyclo[2.2.2]octane-1,4-diium, and 1,4-diazepane-1,4-diium, and derivatives thereof. In some embodiments, the B component is selected from at least one of ammonium salts and ammonia, and the A component is selected from at least one of 1-methylpiperazine, 1,4-diazepane, 1-methylpiperazine-1,4-diium, 1-methyl-1,4-diazabicyclo[2.2.2]octane-1,4-diium, and 1,4-diazepane-1,4-diium, and derivatives thereof. In some embodiments, the B component is selected from at least one of ammonium salts and ammonia, and the A component is selected from at least one of 1-methylpiperazine, 1,4-diazepane, 1-methylpiperazine-1,4-diium, and 1,4-diazepane-1,4-diium, and derivatives thereof.

In some embodiments, the A component is selected from at least one of piperazine, piperazine-1,4-diium, 1-methylpiperazine, 1-methylpiperazine-1,4-diium, and derivatives thereof, and the B' component includes at least one of ethylenediamine and ethylenediammonium salts. In some embodiments, the A component is selected from at least one of piperazine, piperazine-1,4-diium, 1,4-diazepane, 1,4-diazepane-1,4-diium, and derivatives thereof, and the B' component includes at least one of ethylenediamine and ethylenediammonium salts. In some embodiments, the A component is selected from at least one of 1-methylpiperazine, 1-methylpiperazine-1,4-diium, 1,4-diazepane, 1,4-diazepane-1,4-diium, and derivatives thereof, and the B' component includes at least one of ethylene diamine and ethylenediammonium salts.

In some embodiments, the A component is selected from at least one of piperazine, piperazine-1,4-diium, and derivatives thereof, and the B' component includes at least one of ethylenediamine and ethylenediammonium salts. In some embodiments, the A component is selected from at least one of 1-methylpiperazine, 1-methylpiperazine-1,4-diium, and derivatives thereof, and the B' component includes at least one of ethylenediamine and ethylenediammonium salts. In some embodiments, the A component is selected from at least one of 1,4-diazepane, 1,4-diazepane-1,4-diium, and derivatives thereof, and the B' component includes at least one of ethylenediamine and ethylenediammonium salts.

In some embodiments, the A component is selected from at least one of piperazine, piperazine-1,4-diium, 1-methylpiperazine, 1-methylpiperazine-1,4-diium, 1,4-diazepane, and 1,4-diazepane-1,4-diium, and the B' component includes at least one of ethylenediamine and ethylenediammonium salts. In some embodiments, the A component is selected from at least one of piperazine, piperazine-1,4-diium, 1-methylpiperazine, 1-methylpiperazine-1,4-diium, and the B' component includes at least one of ethylenediamine and ethylenediammonium salts. In some embodiments, the A component is selected from at least one of piperazine, piperazine-1,4-diium, 1,4-diazepane, and 1,4-diazepane-1,4-diium, and the B' component includes at least one of ethylenediamine and ethylenediammonium salts. In some embodiments, the A component is selected from at least one of 1-methylpiperazine, 1-methylpiperazine-1,4-diium, 1,4-diazepane, and 1,4-diazepane-1,4-diium, and the B' component includes at least one of ethylenediamine and ethylenediammonium salts. In some embodiments, the A component includes at least one of piperazine and piperazine-1,4-diium and the B' component includes at least one of ethylenediamine and ethylenediammonium salts. In some embodiments, the A component includes at least one of 1-methylpiperazine and 1-methylpiperazine-1,4-diium, and the B' component includes at least one of ethylenediamine and ethylenediammonium salts. In some embodiments, the A component includes at least one of 1,4-diazepane and 1,4-diazepane-1,4-diium, and the B' component includes at least one of ethylenediamine and ethylenediammonium salts.

In some embodiments, the X component is selected from at least one of acids and salts including an X anion in the compound to be synthesized, and/or the X component produces the X anion when dissolved in the liquid reaction system.

In some embodiments, the X component is selected from at least one of acids and salts including anionic energetic ligands.

In some embodiments, the X component is selected from at least one of acids and salts including monovalent anions.

In some embodiments, the X component is selected from at least one of chloric acid, chlorate, perchloric acid, perchlorate, bromic acid, bromate, perbromic acid, perbromate, iodic acid, iodate, periodic acid, periodate, nitric acid, nitrate, fulminic acid, fulminate, azo salts, and azide salts.

In some embodiments, the X component is selected from at least one of chloric acid, chlorate, perchloric acid, perchlorate, nitric acid, nitrate, fulminic acid, fulminate, azo salts, and azide salts.

In some embodiments, the X component includes at least one of halogen-containing acids and salts.

In some embodiments, the X component includes at least one of halogen-containing oxyacids and salts thereof, nitric acid, and nitrate.

In some embodiments, the X component includes at least one of halogen-containing oxyacids and salts thereof.

In some embodiments, the X component includes at least one of haloacid, halate, perhaloacid, perhalate, nitric acid, and nitrate. In some embodiments, the X component includes at least one of haloacid, halate, perhaloacid, and perhalate. In some embodiments, the X component includes at least one of perhalic acid, perhalate, nitric acid, and nitrate. In some embodiments, the X component includes at least one of perhalic acid and perhalate.

In some embodiments, the X component includes at least one of perchloric acid, perchlorate, perbromic acid, perbromate, periodic acid, periodate, nitric acid, and nitrate. In some embodiments, the X component includes at least one of perchloric acid, perchlorate, perbromic acid, perbromate, periodic acid, and periodate. In some embodiments, the X component includes at least one of perchloric acid, perchlorate, perbromic acid, and perbromate. In some embodiments, the X component includes at least one of perchloric acid, perchlorate, periodic acid, and periodate. In some embodiments, the X component includes at least one of perbromic acid, perbromate, periodic acid, and periodate.

In some embodiments, the X component includes at least one of perchloric acid and perchlorate. In some embodiments, the X component includes at least one of perbromic acid and perbromate. In some embodiments, the X component includes at least one of periodic acid and periodate.

In some embodiments, the X component is selected from at least one of perchloric acid and perchlorate; the B component is selected from at least one of potassium salt and psotassium hydroxide; and the A component is selected from at least one of piperazine, 1,4-diazepane, piperazine-1,4-diium, and 1,4-diazepane-1,4-diium.

In some embodiments, the X component is selected from at least one of perchloric acid and perchlorate; the B component is selected from at least one of potassium salts and potassium hydroxide; and the A component is selected from at least one of piperazine, and piperazine-1,4-diium.

In some embodiments, the X component is selected from at least one of perchloric acid and perchlorate; the B component is selected from silver salts; and the A component is selected from at least one of piperazine, 1-methylpiperazine, 1,4-diazabicyclo[2.2.2]octane, 1,4-diazepane, piperazine-1,4-diium, 1-methylpiperazine-1,4-diium, 1,4-diazabicycio[2.2.2]octane-1,4-diium, and 1,4-diazepane-1,4-diium.

In some embodiments, the X component is selected from at least one of perchloric acid and perchlorate; the B component is selected from silver salts; and the A component is selected from at least one of piperazine, 1-methylpiperazine, 1,4-diazepane, piperazine-1,4-diium, 1-methylpiperazine-1,4-diium, and 1,4-diazepane-1,4-diium.

In some embodiments, the X component is selected from at least one of perchloric acid and perchlorate; the B component is selected from silver salts; and the A component is selected from at least one of piperazine, 1-methylpiperazine, 1,4-diazabicyclo[2.2.2]octane, piperazine-1,4-diium, 1-methylpiperazine-1,4-diium, and 1,4-diazabicyclo[2.2.2]octane-1,4-diium.

In some embodiments, the X component is selected from at least one of perchloric acid and perchlorate; the B component is selected from silver salts; and the A component is selected from at least one of piperazine, 1,4-diazabicyclo[2.2.2]octane, 1,4-diazepane, piperazine-1,4-diium, 1,4-diazabicyclo[2.2.2]octane-1,4-diium, and 1,4-diazepane-1,4-diium.

In some embodiments, the X component is selected from at least one of perchloric acid and perchlorate; the B component is selected from silver salts; and the A component is selected from at least one of 1-methylpiperazine, 1,4-diazabicyclo[2.2.2]octane, 1,4-diazepane, 1-methylpiperazine-1,4-diium, 1,4-diazabicyclo[2.2.2]octane-1,4-diium, and 1,4-diazepane-1,4-diium.

In some embodiments, the X component is selected from at least one of perchloric acid and perchlorate; the B component is selected from silver salts; and the A component is selected from at least one of piperazine, 1,4-diazepane, piperazine-1,4-diium, and 1,4-diazepane-1,4-diium.

In some embodiments, the X component is selected from at least one of perchloric acid and perchlorate; the B component is selected from silver salts; and the A component is selected from at least one of 1-methylpiperazine, 1,4-diazepane, 1-methylpiperazine-1,4-diium, and 1,4-diazepane-1,4-diium.

In some embodiments, the X component is selected from at least one of perchloric acid and perchlorate; the B component is selected from silver salts; and the A component is selected from at least one of piperazine, 1-methylpiperazine, piperazine-1,4-diium, and 1-methylpiperazine-1,4-diium.

In some embodiments, the X component is selected from at least one of perchloric acid and perchlorate; the B component is selected from silver salts; and the A component is selected from at least one of piperazine, 1,4-diazabicyclo[2.2.2]octane, piperazine-1,4-diium, and 1,4-diazabicyclo[2.2.2]octane-1,4-diium.

In some embodiments, the X component is selected from at least one of perchloric acid and perchlorate; the B component is selected from silver salts; and the A component is selected from at least one of 1,4-diazabicyclo[2.2.2]octane, 1,4-diazepane, 1,4-diazabicyclo[2.2.2]octane-1,4-diium, and 1,4-diazepane-1,4-diium.

In some embodiments, the X component is selected from at least one of perchloric acid and perchlorate; the B component is selected from silver salts; and the A component is selected from at least one of 1-methylpiperazine, 1,4-diazabicyclo[2.2.2]octane, 1-methylpiperazine-1,4-diium, and 1,4-diazabicyclo[2.2.2]octane-1,4-diium.

In some embodiments, the X component is selected from at least one of perchloric acid and perchlorate; the B component is selected from at least one of ammonium salts and ammonia; and the A component is selected from at least one of 1-methylpiperazine, 1,4-diazepane, 1-methylpiperazine-1,4-diium, 1-hydroxy-1,4-diazabicyclo[2.2.2]octane-1,4-diium, 1-methyl-1,4-diazabicyclo[2.2.2]octane-1,4-diium, and 1,4-diazepane-1,4-diium.

In some embodiments, the X component is selected from at least one of perchloric acid and perchlorate; the B component is selected from at least one of ammonium salts and ammonia; and the A component is selected from at least one of 1-methylpiperazine, 1,4-diazepane, 1-methylpiperazine-1,4-diium, 1-hydroxy-1,4-diazabicyclo[2.2.2]octane-1,4-diium, and 1,4-diazepane-1,4-diium.

In some embodiments, the X component is selected from at least one of perchloric acid and perchlorate; the B component is selected from at least one of ammonium salts and ammonia; and the A component is selected from at least one of 1-methylpiperazine, 1,4-diazepane, 1-methylpiperazine-1,4-diium, 1-methyl-1,4-diazabicyclo[2.2.2]octane-1,4-diium, and 1,4-diazepane-1,4-diium

In some embodiments, the X component is selected from at least one of perchloric acid and a perchlorate; the B component is selected from at least one of an ammonium salt and ammonia; the A component is selected from at least one of 1-methylpiperazine, 1,4-diazepane, a 1-methylpiperazine-1,4-diium, and a 1,4-diazepane-1,4-diium.

In some embodiments, the X component is at least one of a perchlorate group-containing acid and a perchlorate group-containing salt; the polar solvent is selected from at least one of water, ethanol, and methanol; the B component is at least one of hydroxylamine, a hydroxylammonium ion-containing salt, a hydroxylammonium ion-containing base, hydrazine, a hydrazinium ion-containing salt, and an hydrazinium ion-containing base; the A component is at least one of 1,4-diazabicyclo[2.2.2]octane and an organic salt including 1,4-diazabicyclo[2.2.2]octane-1,4-diium.

In some embodiments, the X component is at least one of a perchlorate group-containing acid, a perchlorate group-containing salt; the B component is at least one of hydroxylamine, a hydroxylammonium ion-containing salt, and a hydroxylammonium ion-containing base; the A component is at least one of 1,4-diazabicyclo[2.2.2]octane and an organic salt including 1,4-diazabicyclo[2.2.2]octane-1,4-diium.

In some embodiments, the X component is at least one of a perchlorate group-containing acid, a perchlorate group-containing salt; the B component is at least one of a hydrazine, a hydrazinium ion -containing salt, and a hydrazinium ion - containing base; the A component is at least one of 1,4-diazabicyclo[2.2.2]octane and an organic salt including 1,4-diazabicyclo[2.2.2] octane- 1,4-diium.

In some embodiments, the A component is selected from at least one of piperazine, piperazine-1,4-diium, 1-methylpiperazine, 1-methylpiperazine-1,4-diium, and derivatives thereof; the B' component includes at least one of ethylenediamine and an ethylenediammonium salt; the X component includes perchloric acid and a perchlorate. In some embodiments, the A component is selected from at least one of piperazine, piperazine-1,4-diium, 1,4-diazepane, 1,4-diazepane-1,4-diium, and derivatives thereof; the B' component includes at least one of ethylenediamine and an ethylenediammonium salt; the X component includes at least one of perchloric acid and a perchlorate. In some embodiments, the A component is selected from at least one of 1-methylpiperazine, 1-methylpiperazine-1,4-diium, 1,4-diazepane, 1,4-diazepane-1,4-diium, and derivatives thereof; the B' component includes at least one of ethylenediamine and an ethylenediammonium salt; the X component includes at least one of perchloric acid and a perchlorate.

In some embodiments, the A component is selected from at least one of piperazine, piperazine-1,4-diium, and derivatives thereof; the B' component includes at least one of ethylenediamine and ethylenediammonium salts; the X component includes at least one of perchloric acid and perchlorate. In some embodiments, the A component is selected from at least one of 1-methylpiperazine, 1-methylpiperazine-1,4-diium, and derivatives thereof; the B' component includes at least one of ethylenediamine and ethylenediammonium salts; the X component includes at least one of perchloric acid and a perchlorate. In some embodiments, the A component is selected from at least one of 1,4-diazepane, 1,4-diazepane-1,4-diium, and derivatives thereof; the B' component includes at least one of ethylenediamine and ethylenediammonium salts; the X component includes at least one of perchloric acid and perchlorate.

In some embodiments, the A component is selected from at least one of piperazine, piperazine-1,4-diium, 1-methylpiperazine, 1-methylpiperazine-1,4-diium, 1,4-diazepane, and 1,4-diazepane-1,4-diium; the B' component includes at least one of ethylenediamine and ethylenediammonium salts; the X component includes at least one of perchloric acid and perchlorate. In some embodiments, the A component is selected from at least one of piperazine, piperazine-1,4-diium, 1-methylpiperazine, 1-methylpiperazine-1,4-diium; the B' component includes at least one of ethylenediamine and ethylenediammonium salts; the X component includes perchloric acid and perchlorate. In some embodiments, the A component is selected from at least one of piperazine, piperazine-1,4-diium, 1,4-diazepane, and 1,4-diazepane-1,4-diiumt; the B' component includes at least one of ethylenediamine and ethylenediammonium salts; the X component includes at least one of perchloric acid and perchlorate. In some embodiments, the A component is selected from at least one of 1-methylpiperazine,a 1-methylpiperazine-1,4-diium, 1,4-diazepane, and 1,4-diazepane-1,4-diium; the B' component includes at least one of ethylenediamine and ethylenediammonium; the X component includes at least one of perchloric acid and perchlorate. In some embodiments, the A component includes at least one of piperazine and piperazine-1,4-diium; the B' component includes at least one of ethylenediamine and ethylenediammonium salts; the X component includes at least one of perchloric acid and perchlorate. In some embodiments, the A component includes at least one of 1-methylpiperazine and 1-methylpiperazine-1,4-diium; the B' component includes at least one of ethylenediamine and ethylenediammonium salts; the X component includes at least one of perchloric acid and perchlorate. In some embodiments, the A component includes at least one of 1,4-diazepane and 1,4-diazepane-1,4-diium; the B' component includes at least one of ethylenediamine and ethylenediammonium; the X component includes at least one of perchloric acid and perchlorate.

In some embodiments, the A component is selected from optionally one, two, or more of the A components described above; the A' component is selected from optionally one, two, or more of the A' components described above; the B component is selected from optionally one, two, or more of the B components described above; the B' component is selected from optionally one, two, or more of the B' components described above; and/or the X component is selected from optionally one, two, or more of the X components described above.

In some embodiments, the compound to be synthesized is compound ABX₃; wherein the A component is selected from the salts including the A cation in the compound to be synthesized; the B component is selected from at least one of salts and hydroxides including the B cation in the compound to be synthesized; the X component is selected from at least one of acids and salts including the X anion in the compound to be synthesized; the sum of the valency of the A cation and the valency of the B cation is three times the valency of the X anion.

In some embodiments, the compound to be synthesized is compound AB'X₄; wherein the A component is selected from the salts including the A cation in the compound to be synthesized; the B' component is selected from at least one of salts and hydroxides comprising the B' cation in the compound to be synthesized; the X component is selected from at least one of acids and salts comprising the X anion in the compound to be synthesized; the sum of the valency of the A cation and the valency of the B' cation is four times the valency of the X anion.

In some embodiments, the compound to be synthesized is compound B'₂A'X₅; wherein the A' component is selected from the salts including the A' cation in the compound to be synthesized; the B' component is selected from at least one of salts and hydroxides comprising the B' cation in the compound to be synthesized; the X component is selected from at least one of acids and salts comprising the Xanion in the compound to be synthesized; the sum of the valency of the A' cation and two times the valency of the B' cation is five times the valency of the X anion.

In some embodiments, a method for preparing a compound includes: mixing A component or A' component, B component or B' component, and X component in a liquid reaction system in any order to obtain a solid product produced in the liquid reaction system. Wherein the A component is selected from at least one of a nitrogen-containing organic compound and salts thereof; the B component and A' component are selected from at least one of an alkali metal salt and hydroxide, an ammonium salt, ammonia, a silver salt, a monovalent nitrogen-containing cation salt, and a monovalent nitrogen-containing base; the B' component is selected from at least one of saturated an aliphatic diammonium salt and a saturated aliphatic diamine; the X component is selected from at least one of an acid and salt including an anionic energetic ligand; the liquid reaction system is a polar solvent that may dissolve the A component or A' component, the B component or B' component, and the X component.

In some embodiments, the compound to be synthesized by the method for preparing a compound is a compound ABX₃ consisting of an A cation, a B cation, and an X anion. Wherein the A cation is a nitrogen-containing organic cation; the B cation is a cation; the Xanion is an anionic energetic ligand; the A component is at least one of a nitrogen-containing organic compound and a salt thereof; the B component is at least one of a salt including a cation, a hydroxide, and ammonia; the X component is at least one of an acid and a salt including an anion. Alternatively, the compound is a compound AB'X₄ consisting of an A cation, a B' cation, and an X anion or a compound B'₂A'X₅ consisting of an A' cation, a B' cation, and an X anion, wherein the A cation is a divalent nitrogen-containing monocyclic heterocycle cation; the A' cation is at least one of an alkali metal ion and a monovalent nitrogen-containing cation; the B' cation is a saturated aliphatic diammonium cation; the X anion is an anionic energetic ligand; the A component is selected from at least one of a divalent nitrogen-containing monocyclic heterocycle and a salt thereof, including an onium salt; the A' component is selected from at least one of an alkali metal salt and hydroxide, a monovalent nitrogen-containing cation salt, and a monovalent nitrogen-containing base; the B component is selected from at least one of a saturated aliphatic diammonium salt and a saturated aliphatic diamine; the X component is selected from at least one of an acid and salt including an anion.

In some embodiments, the polar solvent may be selected from at least one of water and an alcohol. In some embodiments, the polar solvent may be selected from at least one of water, ethanol, and methanol. In some embodiments, the polar solvent may be water.

In some embodiments of the present application, there is provided an energetic material including a compound of any one of the above embodiments, a combination of compounds of any two or more of the above embodiments, or a compound prepared by a method of preparing a compound of any one of the above embodiments, and a combination of methods of preparing a compound of any two or more of the above embodiments. In some embodiments of the present application, provided is a compound of any one ofthe above embodiments, a combination of compounds of any two or more ofthe above embodiments, or a compound prepared by a method of preparing a compound of any one of the above embodiments, a combination of methods of preparing a compound of any two or more of the above embodiments, and use as an energetic material. In some embodiments ofthe present application, provided is a compound of any one of the above embodiments, a combination of compounds of any two or more of the above embodiments, or a compound prepared by a method of preparing a compound of any one of the above embodiments, a combination of methods of preparing a compound of any two or more ofthe above embodiments, and use for manufacturing an energetic material therefrom.

In some ofthe embodiments described above, the energetic material may be a primer, an explosive, a propellant, or a pyrotechnic composition. In some embodiments, the B cation is selected from at least one of ammonium ion, hydroxylammonium ion, and hydrazinium ion, and the energetic material is an explosive or propellant, such as a solid propellant. In some embodiments, the energetic material is a metal-free energetic material.

In some embodiments, the B cation is silver ion, and the energetic material is a primer or an explosive. In some embodiments, the B cation is potassium ion and the energetic material is an explosive or a pyrotechnic composition. In some embodiments, the B cation is selected from at least one of ammonium ion, hydroxylammonium ion, and hydrazinium ion; the A cation is selected from piperazine-1,4-diium, 1-methylpiperazine-1,4-diium, 1,4-diazepane-1,4-diium, 1-hydroxy-1,4-diazabicyclo[2.2.2]octane ammonium ion, or 1-methyl-1,4-diazabicyclo[2.2.2]octane-1,4-diium; the X ion is perchlorate ion; the energetic material is an explosive or a propellant, e.g. a solid propellant. In some embodiments, the B cation is silver ion; the A cation is selected from a piperazine-1,4-diium, a 1-methylpiperazine-1,4-diium, a 1,4-diazepane-1,4-diium, or a 1,4-diazabicyclo[2.2.2]octane-1,4-diium; the X ion is perchlorate ion; the energetic material is a primer or an explosive. In some embodiments, the B cation is potassium ion; the A cation is selected from a piperazine-1,4-diium or a 1,4-diazepane-1,4-diium; the X ion is perchlorate ion; and the energetic material is an explosive or a pyrotechnic composition.

At least one compound in at least some embodiments of the present disclosure has no volatility, can be stored for a long time without decomposing, and does not absorb moisture; the crystallinity at room temperature is single. The raw materials are cheap and easy to obtain, and the production process is simple. The compound can be prepared safely and in large quantities.

### BRIEF DESCRIPTION OF THE DRAWINGS

FIG. 1 is a schematic diagram of the crystal structure of a compound ABX₃.
FIG. 2 is a schematic diagram of the crystal structure of a compound ABX₃.
FIG. 3 is a schematic diagram of the crystal structure of a compound ABX₃.
FIG. 4 is a schematic diagram of the crystal structure of a compound ABX₃.
FIG. 5 is a schematic diagram of the crystal structure of a compound ABX₃.
FIG. 6 is a schematic diagram of the crystal structure of a compound ABX₃.
FIG. 7 is a schematic diagram of the crystal structure of a compound AB'X₄.
FIG. 8 is a schematic diagram of the crystal structure of a compound B'₂A'X₅.
FIG. 9 is a powder X-ray diffraction pattern of compound PAP-4 of Example 1.
FIG. 10 is a differential thermal analysis curve of compound PAP-4 of Example 1.
FIG. 11 is a powder X-ray diffraction pattern of a compound PAP-M4 of Example 2.
FIG. 12 is a differential thermal analysis curve of compound PAP-M4 of Example 2.
FIG. 13 is a powder X-ray diffraction pattern of compound PAP-H4 of Example 3.
FIG. 14 is a differential thermal analysis curve of compound PAP-H4 of Example 3.
FIG. 15 is a powder X-ray diffraction pattern of compound DAP-O4 of Example 4.
FIG. 16 is a differential thermal analysis curve of compound DAP-O4 of Example 4.
FIG. 17 is a powder X-ray diffraction pattern of compound DAP-M4 of Example 5.
FIG. 18 is a differential thermal analysis curve of compound DAP-M4 of Example 5.
FIG. 19 is a powder X-ray diffraction pattern of compound PAP-5 of Example 6.
FIG. 20 is a differential thermal analysis curve of compound PAP-5 of Example 6.
FIG. 21 is a powder X-ray diffraction pattern of compound PAP-M5 of Example 7.
FIG. 22 is a differential thermal analysis curve of compound PAP-M5 of Example 7.
FIG. 23 is a powder X-ray diffraction pattern of compound PAP-H5 of Example 8.
FIG. 24 is a differential thermal analysis curve of compound PAP-H5 of Example 8.
FIG. 25 is a powder X-ray diffraction pattern of compound DAP-5 of Example 9.
FIG. 26 is a differential thermal analysis curve of compound DAP-5 of Example 9.
FIG. 27 is a powder X-ray diffraction pattern of compound PAP-H2 of Example 11.
FIG. 28 is a differential thermal analysis curve of compound PAP-H2 of Example 11.
FIG. 29 is a powder X-ray diffraction pattern of compound DAP-6 of Example 12.
FIG. 30 is a differential thermal analysis map of compound DAP-6 of Example 12.
FIG. 31 is a powder X-ray diffraction pattern of compound DAP-7 of Example 13.
FIG. 32 is a differential thermal analysis map of compound DAP-7 of Example 13.
FIG. 33 is a powder X-ray diffraction pattern of compound EAP of Example 14.
FIG. 34 is a differential thermal analysis map of compound EAP of Example 14.
FIG. 35 is a powder X-ray diffraction pattern of compound PEP of Example 15.
FIG. 36 is a differential thermal analysis map of compound PEP of Example 15.
FIG. 37 is a powder X-ray diffraction pattern of compound MPEP of Example 16.
FIG. 38 is a differential thermal analysis map of compound MPEP of Example 16.
FIG. 39 is a powder X-ray diffraction pattern of compound HPEP of Example 17.
FIG. 40 is a differential thermal analysis map of compound HPEP of Example 17.

### DETAILED DESCRIPTION

The inventor designs a series of compounds with specific chemical general formulas and does correlational researches on application of the compounds as simple substance energetic materials in the field of energetic materials. In some embodiments, the compounds are ternary crystalline state energetic compounds. In some embodiments, the energetic compounds may be expressed as the general formula ABX₃. In some embodiments, the compounds may be expressed as the general formula AB'X₄. In some embodiments, the compounds may be expressed as the general formula B'₂A'X₅. In some embodiments, the crystalline state compounds have the characteristics of a perovskite type structure. For example, the crystalline state compounds have the characteristics of a perovskite type structure of the chemical general formula ABX₃.

In some embodiments, X in ABX₃, AB'X₄ or B'₂A'X₅ is an anion energetic ligand. The energetic ligand refers to a group with explosiveness. Common explosive groups include but not limited to ClO₃⁻, ClO₄⁻, IO₄⁻, NO₃⁻, ONC⁻, an diazenyl group, an azide ion, nitryl and the like.

In some embodiments, in ABX₃, AB'X₄ or B'₂A'X₅, for example, X may include one or more ions, and 2, 3, 4, 5, 6, 7, 8, 9, 10 ... types of ions may exist simultaneously. A, A' and B are also the same. When perovskite includes more than one A cation or one A' cation, different A cations or A' cations may be distributed on an A site or an A' site orderly or disorderly. When perovskite includes more than one B cations, different B cations may be distributed on a B site orderly or disorderly. When perovskite includes more than one X anion, different X anions may be distributed on an X site orderly or disorderly.

Based on the properties, as described herein, "X is ... group/ion", "A is ... group/ion", "A' is ... group/ion"; "B is ... group/ion", "B' is ... group/ion", "X is selected from at least one of...","A is selected from at least one of...","A' is selected from at least one of...", 'B is selected from at least one of...","B' is selected from at least one of..." and the like should be understood as, for example, for X, there are a lot of X sites in three-dimensional crystal structures of ABX₃, AB'X₄ or B'₂A'X₅; each X site is composed of ions; in the three-dimensional crystal structures, a plurality of X sites may be composed of the same type of ions, and may also be composed of various different ions; and when the plurality of X sites are composed of different ions, there are at least some sites (or most sites) therein are composed of ... groups/ions. At the moment, it is not exclusive that, in the whole three-dimensional crystal structures of ABX₃, AB'X₄ or B'₂A'X₅, there are a few sites that are not composed on the ... groups/ions or are some other foreign ions, so long as the quantity of these sites does not influence the whole performance to a great extent. The few sites may be, for example, under 50% of the mole number, which is not more than 40%, 30%, 25%, 20%, 15%, 10%, 9%, 8%, 7%, 6%, 5%, 4%, 3%, 2% or 1%. The same applies to A and B.

In some embodiments, the present application provides various identification and characterization methods comprising characterizing and testing of powder X-ray single-crystal structures, identifying of X-ray powder diffraction, testing and characterizing of a DTA (Differential Thermal Analysis), testing and characterizing of impact and friction sensitivity, calculating of detonation parameters (values of detonation heat/detonation pressure/detonation velocity) and the like.

In some embodiments, the synthesis of the compounds ABX₃, AB'X₄ or B'₂A'X₅ may be carried out according to a synthesis method disclosed by the present application.

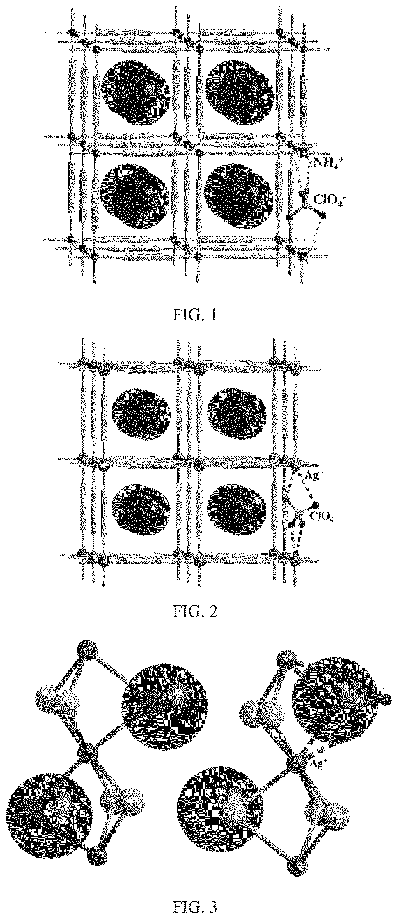

A schematic diagram of a crystal structure of a type of perovskite compound ABX₃ is shown in Fig. 1. As shown in Fig. 1, it can be seen that a cation on each B site (such as an ammonium ion) is connected with six adjacent anions on X sites, and the anion on each X site is connected with two adjacent cations on the B sites, so as to form a three-dimensional anion skeleton formed by cubic cage-shaped units; and an organic cation on each A site is filled in a hole of each cubic cage-shaped unit.

A schematic diagram of a crystal structure of a type of perovskite compound ABX₃ is shown in Fig. 2. As shown in Fig. 2, it can be seen that a cation on each B site (such as a silver ion) is connected with six adjacent anions on X sites, and the anion on each X site is connected with two adjacent ions on the B sites, so as to form a three-dimensional anion skeleton formed by cubic cage-shaped units; and an organic cation on each A site is filled in a hole of each cubic cage-shaped unit.

A schematic diagram of a crystal structure of a type of perovskite compound ABX₃ is shown in Fig. 3. As shown in Fig. 3, it can be seen that six anions on X sites are around a cation (such as a silver ion) on each B site, so as to form a BX₆ octahedron; the adjacent cations are connected in a *b*-axis direction through three µ₂-anions, so as to form a one-dimensional chain; and an organic cation on each A site is filled between the chains.

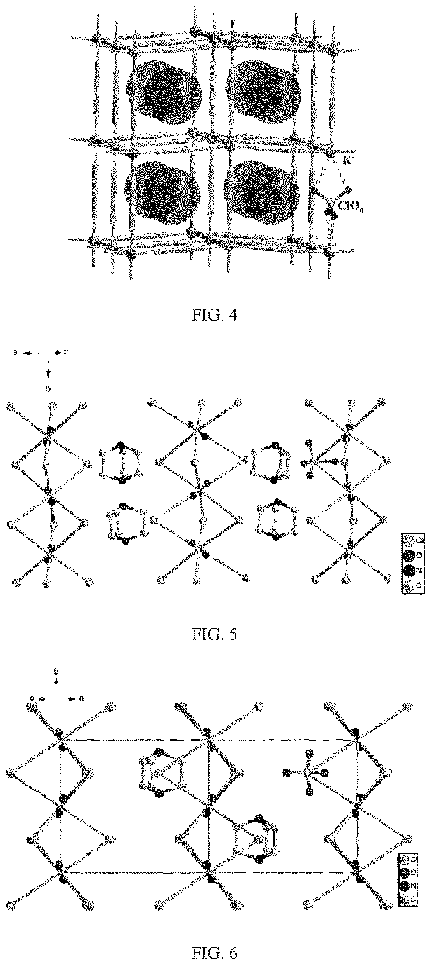

A schematic diagram of a crystal structure of a type of perovskite compound ABX₃ is shown in Fig. 4. As shown in Fig. 4, it can be seen that a cation (such as a potassium ion) on each B site is connected with six adjacent anions on X sites, and the anion on each X site is connected with two adjacent cations on the B sites, so as to form a three-dimensional anion skeleton formed by cubic cage-shaped units; and an organic cation on each A site is filled in a hole of each cubic cage-shaped unit.

A schematic diagram of a crystal structure of a type of perovskite compound ABX₃ is shown in Fig. 5. As shown in Fig. 5, it can be seen that six anions (such as ClO₄⁻ ions) on X sites are around a cation (such as a NH₃OH⁺ ion) on each B site, so as to form a squashed octahedron; the adjacent cations on the B sites are connected in a *b*-axis direction through three µ₂-anions on the X sites, so as to form a one-dimensional chain; and an organic cation (such as a 1,4-diazabicyclo[2.2.2]octane-1,4-diium (H₂dabco²⁺) cation) on each A site is filled between the chains.

A schematic diagram of a crystal structure of a type of perovskite compound ABX₃ is shown in Fig. 6. As shown in Fig. 6, it can be seen that six anions (such as ClO₄⁻ ions) on X sites are around a cation (such as a NH₂NH₃⁺ ion) on each B site, so as to form a squashed octahedron; the adjacent cations on the B sites are connected in a *b*-axis direction through three µ₂-anions on the X sites, so as to form a one-dimensional chain; and an organic cation (such as a 1,4-diazabicyclo[2.2.2]octane-1,4-diium (H₂dabco²⁺) cation) on each A site is filled between the chains.

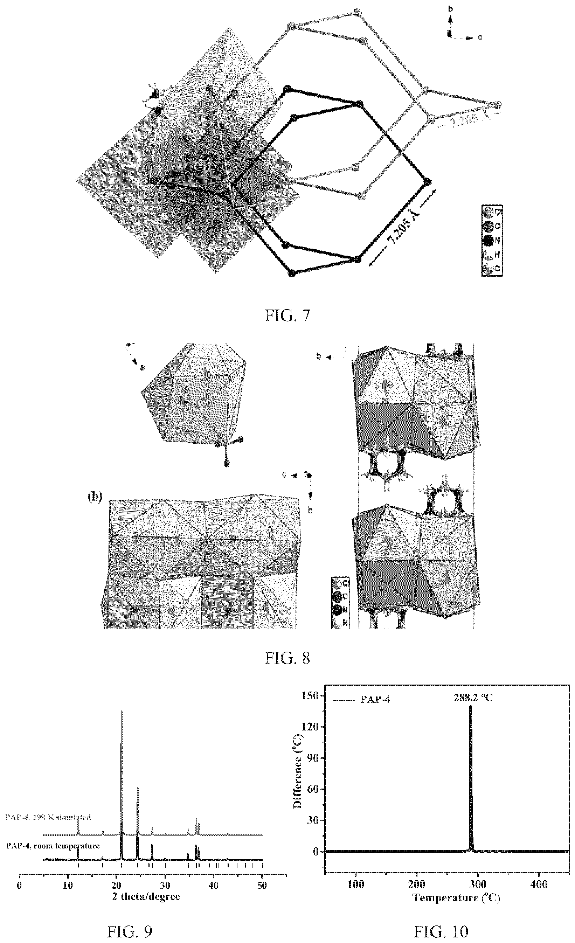

A schematic diagram of a crystal structure of a type of perovskite compound AB'X₄ is shown in Fig. 7. As shown in Fig. 7, it can be seen that ten anions (such as ClO₄⁻ ions) on X sites are around a cation (such as a H₂EA²⁺ ion) on each B' site, so as to form an irregular dodecahedron (as shown in Fig. 7a); the adjacent cations on the B' sites are connected in a *b*-axis direction and a c-axis direction through four *µ*₄-anions on the X sites and six *µ*₂-anions on the X sites, so as to form a layered structure (as shown in Fig. 7b); and organic cations (such as piperazine cations) on A sites between two adjacent layers and anions on the X sites are arranged alternately (as shown in Fig. 7c).

A schematic diagram of a crystal structure of a type of perovskite compound B'₂A'X₅ is shown in Fig. 8. As shown in Fig. 8, it can be seen that adjacent cations (such as ammonium ions) on A' sites are connected with each other to form a diamond mesh structure; an independent unit of the structure has anions on X sites (such as ClO₄⁻ ions), wherein one type of anions are connected to form a diamond mesh structure, the length of one side of which is the same as the length of one side of the diamond mesh structure formed by the adjacent cations on the A' sites; two sets of diamond meshes are crosses; six cations (such as diaminoethane cations) on B' sites are around the cation on each A' site and the anion on the type of X site, so as to form an octahedral structure; three octahedrons are connected through a *µ*₃-cation on the B' site; and an anion on another X site is among the three octahedrons.

In one embodiment, a compound (C₄H₁₂N₂)(NH₄)(ClO₄)₃ (denoted by PAP-4) is provided as an energetic material. The compound is crystallized at an *Fm*-3c space group of a cubic crystal system under 298 K; cell parameters are *a*=*b*=*c*=14.5631(3) Å; and the powder X-ray diffraction (a Cu-*K_{α}* ray) at room temperature occurs but not limited to the diffraction angle 2*θ*, which is about 12.10±0.2°, 17.17±0.2°, 21.03±0.2°, 24.33±0.2°, 27.27±0.2°, 29.95±0.2°, 34.70±0.2°, 36.30±0.2°, 36.89±0.2°, 40.95±0.2°, 42.81±0.2°, 44.67±0.2° and 47.95±0.2°. A test result of a DTA indicates that the peak temperature of thermal decomposition of the compound is 288.2 °C. The values of detonation heat, detonation velocity and detonation pressure ofthe energetic compound, which are obtained by adoptinga detonation parameter calculating method reported by literatures and applying a DFT (Density Functional Theory) and a K-J empirical formula are: 6.00 kJ/g, 8.63 km/s and 32.4 GPa respectively. The values ofthe enthalpy of formation and the theoretical specific impulse ofthe energetic compound, which are obtained by applying a DFT and EXPLO5 software are: -537.7 kJ/mol and 264.2 s respectively.

In one embodiment, a compound (C₅H₁₄N₂)(NH₄)(ClO₄)₃ (denoted by PAP-M4) is provided as an energetic material. The compound is crystallized at a *Pnma* space group of an orthorhombic crystal system under 298 K; cell parameters are a=10.2673(2) Å, b=14.7004(2) Å, and c=20.9914(3) Å; and the powder X-ray diffraction (a Cu-*K_{α}* ray) at room temperature occurs but not limited to the diffraction angle 2*θ*, which is about 7.45±0.2°, 9.68±0.2°, 12.15±0.2°, 13.57±0.2°, 15.45±0.2°, 16.53±0.2°, 17.35±0.2°, 18.34±0.2°, 18.86±0.2°, 20.86±0.2°, 21.88±0.2°, 22.95±0.2°, 23.72±0.2°, 24.31±0.2°, 25.15±0.2°, 26.97±0.2°, 27.46±0.2°, 27.66±0.2°, 28.21±0.2°, 29.13±0.2°, 29.95±0.2°, 30.51±0.2°, 31.13±0.2°, 33.05±0.2°, 34.56±0.2°, 35.52±0.2°, 36.56±0.2°, 37.93±0.2°, 38.83±0.2° and 39.93±0.2°. A test result of a DTA indicates that the peak temperature of thermal decomposition ofthe compound is 323.1 °C. A characterization result of friction sensitivity indicates that PAP-M4 is more sensitive to the friction sensitivity (*FS*≤6N), and a characterization result of impact sensitivity indicates that PAP-M4 is more insensitive to the impact sensitivity (*IS*=30 J). The values of detonation heat, detonation velocity and detonation pressure ofthe energetic compound, which are obtained by adopting a detonation parameter calculating method reported by literatures and applying a DFT and a K-J empirical formula are: 5.14 kJ/g, 8.31 km/s and 30.3 GPa respectively. The values ofthe enthalpy of formation and the theoretical specific impulse ofthe energetic compound, which are obtained by applying a DFT and EXPLO5 software are: -859.9 and 241.2 s respectively.

In one embodiment, a compound (C₅H₁₄N₂)(NH₄)(ClO₄)₃ (denoted by PAP-H4) is provided as an energetic material. The compound is crystallized at a *P2*₁/*n* space group of a monoclinic crystal system under 223 K; cell parameters are a=19.7404(5) Å b=14.3294(5) Å, c=21.2948(8) Å, and *β*=90.075(3)°; and the powder X-ray diffraction (a Cu-*K_{α}* ray) at room temperature occurs but not limited to the diffraction angle 2*θ*, which is about 6.14±0.2°, 7.66±0.2°, 12.25±0.2°, 13.70±0.2°, 14.86±0.2°, 16.58±0.2°, 17.41±0.2°, 18.29±0.2°, 19.05±0.2°, 20.70±0.2°, 21.86±0.2°, 22.27±0.2°, 22.99±0.2°, 23.37±0.2°, 24.50±0.2°, 24.80±0.2°, 25.29±0.2°, 25.93±0.2°, 26.50±0.2°, 27.74±0.2°, 28.31±0.2°, 29.32±0.2°, 29.95±0.2°, 30.48±0.2°, 30.83±0.2°, 31.52±0.2°, 33.36±0.2°, 34.07±0.2°, 35.13±0.2°, 35.64±0.2°, 36.20±0.2°, 36.62±0.2°, 37.11±0.2°, 37.91±0.2°, 39.42±0.2° and 39.97±0.2°. A test result of a DTA indicates that the peak temperature of thermal decomposition of the compound is 348.9 °C. A characterization result of friction sensitivity indicates that PAP-H4 is more sensitive to the friction sensitivity (*FS*=6N), and a characterization result of impact sensitivity indicates that PAP-H4 is more insensitive to the impact sensitivity (*IS*=27.5 J). The values of detonation heat, detonation velocity and detonation pressure of the energetic compound, which are obtained by adopting a detonation parameter calculating method reported by literatures and applying a DFT and a K-J empirical formula are: 5.76 kj/g, 8.76 km/s and 34.3 GPa respectively. The values of the enthalpy of formation and the theoretical specific impulse of the energetic compound, which are obtained by applying a DFT and EXPLO5 software are: -600.4 and 255.4 s respectively.

In one embodiment, a compound (C₆H₁₄N₂O)(NH₄)(ClO₄)₃ (denoted by DAP-O4) is provided as an energetic material. The compound is crystallized at an *Fm*-3*c* space group of a cubic crystal system under 298 K; cell parameters are *a*=*b*=*c*=14.7627(1) Å, and *β* = 90°; and the powder X-ray diffraction (a Cu-*K_{α}* ray) at room temperature occurs but not limited to the diffraction angle 2*θ*, which is about 12.06±0.2°, 20.90±0.2°, 24.15±0.2°, 27.05±0.2°, 29.7±0.2°, 34.38±0.2°, 35.99±0.2°, 36.52±0.2°, 38.60±0.2°, 40.52±0.2°, 42.42±0.2° and 49.36±0.2°. A test result of a DTA indicates that the peak temperature of thermal decomposition ofthe compound is 352.0 °C. A characterization result of friction sensitivity indicates that DAP-O4 is more sensitive to the friction sensitivity (*FS*<5N), and a characterization result of impact sensitivity indicates that DAP-O4 is more insensitive to the impact sensitivity (*IS*=17.5 J). The values of detonation heat, detonation velocity and detonation pressure ofthe energetic compound, which are obtained by adopting a detonation parameter calculating method reported by literatures and applying a DFT and a K-J empirical formula are: 6.21 kJ/g, 8.90 km/s and 35.7 GPa respectively. The values of the enthalpy of formation and the theoretical specific impulse of the energetic compound, which are obtained by applying a DFT and EXPLO5 software are: -436.1 and 262.4 s respectively.

In one embodiment, a compound (C₇H₁₆N₂)(NH₄)(ClO₄)₃ (denoted by DAP-M4) is provided as an energetic material. The compound is crystallized at a *P2*₁ space group of a monoclinic crystal system under 298 K; cell parameters are *a*=10.1520(2) Å, *b*=10.9824(2) Å, *c*=14.7774(2) Å, and *β*=89.856(1)°; and the powder X-ray diffraction (a Cu-*K_{α}* ray) at room temperature occurs but not limited to the diffraction angle 2*θ*, which is about 9.94±0.2°, 11.81±0.2°, 11.44±0.2°, 17.07±0.2°, 18.29±0.2°, 20.00±0.2°, 21.03±0.2°, 23.90±0.2°, 24.39±0.2°, 25.71±0.2°, 26.64±0.2°, 27.35±0.2°, 28.00±0.2°, 28.43±0.2°, 29.83±0.2°, 31.42±0.2°, 32.43±0.2°, 33.06±0.2°, 33.88±0.2°, 34.61±0.2°, 35.03±0.2°, 35.38±0.2°, 35.89±0.2°, 36.15±0.2°, 36.60±0.2°, 37.29±0.2°, 38.35±0.2°, 39.97±0.2° and 40.70±0.2°. A test result of a DTA indicates that the peak temperature of thermal decomposition of the compound is 364.0 °C. A characterization result of friction sensitivity indicates that DAP-M4 is more sensitive to the friction sensitivity (*FS*=10N), and a characterization result of impact sensitivity indicates that DAP-M4 is a bit sensitive to the impact sensitivity (*IS*=7.5 J). The values of detonation heat, detonation velocity and detonation pressure ofthe energetic compound, which are obtained by adoptinga detonation parameter calculating method reported by literatures and applying a DFT and a K-J empirical formula are: 4.99 kj/g, 8.09 km/s and 28.8 GPa respectively. The values of the enthalpy of formation and the theoretical specific impulse of the energetic compound, which are obtained by applying a DFT and EXPLO5 software are: -839.1 and 225.2 s respectively.

In one embodiment, a compound (C₄H₁₂N₂)[Ag(ClO₄)₃] (denoted by PAP-5) is provided as an energetic material. The compound is crystallized at a *P2*₁/*c* space group of a monoclinic crystal system under 223 K; cell parameters are a=10.1221(2) Å, b=9.6470(2) Å, c=13.2672(3) Å, and *β*=91.7815(19]°; and the powder X-ray diffraction (a Cu-*K_{α}* ray) at room temperature occurs but not limited to the diffraction angle 2*θ*, which is about 12.88±0.2, 13.43±0.2°, 14.64±0.2°, 17.68±0.2°, 18.35±0.2°, 18.73±0.2°, 19.78±0.2°, 21.82±0.2°, 22.54±0.2°, 22.90±0.2°, 24.07±0.2°, 25.66±0.2°, 26.44±0.2°, 26.80±0.2°, 28.11±0.2°, 28.74±0.2°, 29.32±0.2°, 30.01±0.2°, 30.83±0.2°, 31.56±0.2°, 32.40±0.2°, 32.70±0.2°, 35.50±0.2°, 36.89±0.2°, 37.73±0.2°, 38.52±0.2°, 38.79±0.2°, 39.24±0.2°, 39.83±0.2° and 40.24±0.2°. A test result of a DTA indicates that the peaktemperature of thermal decomposition of the compound is 341.6 °C. A characterization result of friction sensitivity indicates that PAP-5 is more sensitive to the friction sensitivity (*FS*≤5N). The values of detonation heat, detonation velocity and detonation pressure of the energetic compound, which are obtained by adopting a detonation parameter calculating method reported by literatures and applying a DFT and a K-J empirical formula are: 4.88 kj/g, 8.96 km/s and 42.4 GPa respectively.

In one embodiment, a compound (C₅H₁₄N₂)[Ag(ClO₄)₃] (denoted by PAP-M5) is provided as an energetic material. The compound is crystallized at a *Pnma* space group of an orthorhombic crystal system under 298 K; cell parameters are a=10.1827(3]Å, b=13.8975(4) Å, and c=20.2734(5) Å; and the powder X-ray diffraction (a Cu-*K_{α}* ray) at room temperature occurs but not limited to the diffraction angle 2*θ*, which is about 8.74±0.2°, 9.72±0.2°, 11.61±0.2°, 12.33±0.2°, 12.76±0.2°, 14.60±0.2°, 15.76±0.2°, 17.02±0.2°, 17.43±0.2°, 17.78±0.2°, 18.56±0.2°, 19.60±0.2°, 20.60±0.2°, 24.82±0.2°, 25.68±0.2°, 26.64±0.2°, 27.52±0.2°, 27.99±0.2°, 28.56±0.2°, 29.46±0.2°, 30.62±0.2°, 31.03±0.2°, 31.54±0.2°, 32.21±0.2°, 33.97±0.2°, 34.48±0.2°, 35.50±0.2°, 36.13±0.2°, 37.40±0.2°, 37.93±0.2°, 38.24±0.2°, 38.91±0.2°, 39.46±0.2° and 40.99±0.2°. A test result of a DTA indicates that the peak temperature of thermal decomposition of the compound is 308.3 °C. A test result of sensitivity indicates that PAP-M5 is more sensitive to friction (*FS*≤5N). The values of detonation heat, detonation velocity and detonation pressure of the energetic compound, which are obtained by adopting a detonation parameter calculating method reported by literatures and applying a DFT and a K-J empirical formula are: 5.42 kj/g, 8.73 km/s and 39.2 GPa respectively.

In one embodiment, a compound (C₅H₁₄N₂)[Ag(ClO₄)₃] (denoted by DAP-H5) is provided as an energetic material. The compound is crystallized at a *P2*₁/*n* space group of a monoclinic crystal system under 298 K; cell parameters are *a*=10.7045(2) Å, *b*=8.60470(10) Å, *c*=15.6254(3) Å, and *β*=90.345(2)°; and the powder X-ray diffraction (a Cu-*K_{α}* ray) at room temperature occurs but not limited to the diffraction angle 2*θ*, which is about 10.00±0.2°, 11.33±0.2°, 14.37±0.2°, 16.56±0.2°, 18.98±0.2°, 20.39±0.2°, 20.66±0.2°, 21.66±0.2°, 22.62±0.2°, 23.01±0.2°, 23.62±0.2°, 25.05±0.2°, 25.60±0.2°, 26.09±0.2°, 26.60±0.2°, 27.21±0.2°, 27.66±0.2°, 28.19±0.2°, 29.03±0.2°, 29.85±0.2°, 30.93±0.2°, 31.81±0.2°, 32.09±0.2°, 32.87±0.2°, 33.15±0.2°, 33.5±0.2°, 34.68±0.2°, 35.54±0.2°, 36.58±0.2°, 37.07±0.2°, 38.20±0.2°, 38.56±0.2°, 39.89±0.2° and 40.52±0.2°. A test result of a DTA indicates that the peak temperature of thermal decomposition of the compound is 328.7 °C. A test result of sensitivity indicates that DAP-H5 is more sensitive to friction (*FS*≤N). The values of detonation heat, detonation velocity and detonation pressure of the energetic compound, which are obtained by adopting a detonation parameter calculating method reported by literatures and applying a DFT and a K-J empirical formula are: 5.36 kJ/g, 8.69 km/s and 38.7 GPa respectively.

In one embodiment, a compound (C₆H₁₄N₂)[Ag(ClO₄)₃] (denoted by DAP-5) is provided as an energetic material. The compound is crystallized at a *Pa-3* space group of a cubic crystal system under 223 K; a cell parameter is *a*=14.1379(3) Å; and the powder X-ray diffraction (a Cu-*K_{α}* ray) at room temperature occurs but not limited to the diffraction angle 2*θ*, which is about 12.46±0.2°, 13.90±0.2°, 17.62±0.2°, 18.72±0.2°, 21.64±0.2°, 23.41±0.2°, 25.06±0.2°, 25.83±0.2°, 28.06±0.2°, 28.75±0.2°, 30.83±0.2°, 31.48±0.2°, 33.90±0.2°, 34.53±0.2°, 35.72±0.2°, 36.29±0.2°, 37.41±0.2°, 37.96±0.2°, 39.06±0.2°, 40.11±0.2°, 40.62±0.2°, 41.15±0.2°, 41.68±0.2°, 42.13±0.2°, 42.76±0.2°, 44.12±0.2°, 44.58±0.2°, 45.09±0.2°, 46.03±0.2°, 46.15±0.2°, 46.64±0.2°, 47.88±0.2°, 47.98±0.2°, 48.32±0.2° and 49.24±0.2°. A test result of a DTA indicates that the peak temperature of thermal decomposition of the compound is 313.7 °C. A test result of sensitivity indicates that DAP-5 is more sensitive to impact and friction (*IS*=3J, and *FS≤*N). The values of detonation heat, detonation velocity and detonation pressure of the energetic compound, which are obtained by adopting a detonation parameter calculating method reported by literatures and applying a DFT and a K-J empirical formula are: 4.76 kj/g, 8.59 km/s and 38.5 GPa respectively.

In one embodiment, a compound (C₄H₁₂N₂)[K(ClO₄)₃] (denoted by PAP-2) is provided as an energetic material. The compound is crystallized at a *Pbcm* space group of an orthorhombic crystal system under 284 (17) K; cell parameters are a=10.3353(6) Å, b=9.6777(6) Å, c=13.9862(10) Å, and *α*=*β*=*γ*=90°; and the powder X-ray diffraction (a Cu-*K_{α}* ray) at room temperature occurs but not limited to the diffraction angle 2*θ*, which is about 8.55±0.2, 12.52±0.2°, 12.65±0.2°, 14.03±0.2°, 15.29±0.2°, 17.15±0.2°, 17.83±0.2°, 18.32±0.2°, 19.40±0.2°, 20.25±0.2°, 20.48±0.2°, 21.23±0.2°, 21.36±0.2°, 22.32±0.2°, 22.84±0.2°, 23.27±0.2°, 23.94±0.2°, 25.19±0.2°, 25.45±0.2°, 26.53±0.2°, 26.90±0.2°, 27.34±0.2°, 27.46±0.2°, 27.92±0.2°, 28.20±0.2°, 28.28±0.2°, 28.46±0.2°, 28.87±0.2°, 28.97±0.2°, 29.68±0.2°, 30.34±0.2°, 30.85±0.2°, 31.54±0.2°, 31.73±0.2°, 31.76±0.2°, 31.86±0.2°, 32.24±0.2°, 32.51±0.2°, 32.70±0.2°, 33.34±0.2°, 33.62±0.2°, 34.41±0.2°, 34.69±0.2°, 35.19±0.2°, 35.95±0.2°, 36.12±0.2°, 36.53±0.2°, 37.06±0.2°, 37.37±0.2°, 37.70±0.2°, 38.10±0.2°, 38.25±0.2°, 38.59±0.2°, 38.73±0.2°, 38.94±0.2°, 39.38±0.2°, 39.51±0.2° and 40.05±0.2°. The values of detonation heat, detonation velocity and detonation pressure of the energetic compound, which are obtained by adopting a detonation parameter calculating method reported by literatures and applying a DFT and a K-J empirical formula are: 6.29 kj/g, 8.78 km/s and 36.6 GPa respectively.

In one embodiment, a compound (C₅H₁₄N₂)[K(ClO₄)₃] (denoted by PAP-H2) is provided as an energetic material. The compound is crystallized at a *Pbca* space group of an orthorhombic crystal system under 298 K; cell parameters are a=9.6351(3) Å, b=14.7473(4) Å, and c=20.9607(8) Å; and the powder X-ray diffraction (a Cu-*K_{α}* ray) at room temperature occurs but not limited to the diffraction angle 2*θ*, which is about 8.44±0.2°, 11.96±0.2°, 12.461±0.2°, 12.68±0.2°, 13.84±0.2°, 14.66±0.2°, 16.78±0.2°, 17.46±0.2°, 18.40±0.2°, 19.36±0.2°, 19.82±0.2°, 20.76±0.2°, 22.00±0.2°, 22.40±0.2°, 22.74±0.2°, 24.04±0.2°, 24.42±0.2°, 25.08±0.2°, 25.46±0.2°, 25.82±0.2°, 26.14±0.2°, 26.48±0.2°, 27.16±0.2°, 27.88±0.2°, 28.24±0.2°, 28.86±0.2°, 29.04±0.2°, 29.54±0.2°, 29.76±0.2°, 30.44±0.2°, 30.72±0.2°, 31.02±0.2°, 31.20±0.2°, 31.64±0.2°, 32.24±0.2°, 32.76±0.2°, 33.08±0.2°, 33.24±0.2°, 33.60±0.2°, 34.20±0.2°, 34.36±0.2°, 35.02±0.2°, 35.32±0.2°, 35.50±0.2°, 35.72±0.2°, 35.84±0.2°, 36.04±0.2°, 36.38±0.2°, 36.58±0.2°, 37.38±0.2°, 37.54±0.2°, 37.80±0.2°, 38.04±0.2°, 38.30±0.2°, 38.52±0.2°, 38.68±0.2°, 38.78±0.2°, 39.28±0.2°, 39.64±0.2°, 39.84±0.2° and 40.26±0.2°. A test result of a DTA indicates that the peak temperature of thermal decomposition of the compound is 367.4 °C. A test result of sensitivity indicates that PAP-H2 is insensitive to impact and is more sensitive to friction, and the impact sensitivity and the friction sensitivity of PAP-H2 are *IS=27.5* J and *FS*=7N respectively. The values of detonation heat, detonation velocity and detonation pressure of the energetic compound, which are obtained by adopting a detonation parameter calculating method reported by literatures and applying a DFT and a K-J empirical formula are: 5.32 kj/g, 8.17 km/s and 31.1 GPa respectively.

In one embodiment, a compound (C₆H₁₄N₂)(NH₃OH)(ClO₄)₃ (denoted by DAP-6) is provided as an energetic material.

The compound is crystallized at a *P2*₁ space group of a monoclinic crystal system under 223 K; cell parameters are *a*=20.740(1) Å, *b*=8.2366(2) Å, *c*=20.790(1) Å, and *β*=119.65(1)°; and the powder X-ray diffraction (a Cu-*K*_{α} ray) at room temperature occurs but not limited to the diffraction angle 2*θ*, which is about 9.81±0.2°, 14.56±0.2°, 19.68±0.2°, 21.56±0.2°, 22.46±0.2°, 27.62±0.2°, 29.36±0.2°, 34.12±0.2°, 37.00±0.2° and 49.34±0.2°. A test result of a DTA indicates that the peak temperature of thermal decomposition of the compound is 246.1 °C. A characterization result of friction sensitivity indicates that DAP-6 is more sensitive to friction (*FS*≤5N), and a characterization result of impact sensitivity indicates that DAP-6 is more insensitive to impact (*IS*=15 J). The values of detonation heat, detonation velocity and detonation pressure of the energetic compound, which are obtained by adopting a detonation parameter calculating method reported by literatures and applying a DFT and a K-J empirical formula are: 1.52 kcal/g, 9.12 km/s and 38.1 GPa respectively.

In one embodiment, a compound (C₆H₁₄N₂)(NH₃NH₂)(ClO₄)₃ (denoted by DAP-7) is provided as an energetic material. The compound is crystallized at a *P2*₁/*m* space group of a monoclinic crystal system under 223 K; cell parameters are *a*=10.378(2) Å, *b*=8.0505(7) Å, *c*=10.587(2) Å, and *β*=117.99(2)°; and the powder X-ray diffraction (a Cu-*K*_{α} ray) at room temperature occurs but not limited to the diffraction angle 2*θ*, which is about 14.52±0.2°, 19.36±0.2°, 19.76±0.2°, 21.98±0.2°, 22.30±0.2°, 22.65±0.2°, 29.78±0.2°, 35.74±0.2°, 37.40±0.2° and 49.68±0.2°. A test result of a DTA indicates that the peak temperature of thermal decomposition of the compound is 376.1 °C. A characterization result of friction sensitivity indicates that DAP-7 is more sensitive to friction (*FS*≤5N), and a characterization result of impact sensitivity indicates that DAP-7 is more insensitive to impact (*IS*=27.5 J). The values of detonation heat, detonation velocity and detonation pressure of the energetic compound, which are obtained by adopting a detonation parameter calculating method reported by literatures and applying a DFT and a K-J empirical formula are: 1.43 kcal/g, 8.89 km/s and 35.80 GPa respectively.

In one embodiment, a compound (H₂EA)₂(NH₄)(ClO₄)₅ (denoted by EAP) is provided as an energetic material. The compound is crystallized at an *I*4₁/*a* space group of a tetragonal crystal system under 298 K; cell parameters are *a*=*b*=10.7731(1) Å, *c*=19.1420(3) Å, and *α*=*β*=*γ*=90°; and the powder X-ray diffraction (a Cu-*K*_{α} ray) at room temperature occurs but not limited to the diffraction angle 2*θ*, which is about 9.29±0.2°, 14.73±0.2°, 16.30±0.2°, 18.86±0.2°, 21.78±0.2°, 23.25±0.2°, 24.77±0.2°, 25.10±0.2°, 32.11±0.2°, 38.16±0.2° and 48.28±0.2°. Atest result of a DTA indicates that EAP has two decomposition exothermic peaks, and the peak temperatures are 304.2 °C and 376.0 °C respectively. A characterization result of friction sensitivity indicates that EAP is more sensitive to friction (*FS*=36N), and a result of impact sensitivity indicates that EAP is more insensitive to impact (*IS*=12 J). The values of detonation heat, detonation velocity and detonation pressure of the energetic compound, which are obtained by adopting a detonation parameter calculating method reported by literatures and applying a DFT and a Kamlet-Jacob empirical formula are: 5.07 kJ/g, 8.97 km/s and 37.0 GPa respectively.

In one embodiment, a compound (C₄H₁₂N₂)(H₂EA)(ClO₄)₄ (denoted by PEP) is provided as an energetic material. The compound is crystallized at a *Pbca* space group of an orthorhombic crystal system under 298 K; cell parameters are a=23.9304(2) Å, b=10.3226(1) Å, c=31.4590(4) Å, and *α*=*β*=*γ*=90°; and the powder X-ray diffraction (a Cu-*K*_{α} ray) at room temperature occurs but not limited to the diffraction angle 2*θ*, which is about 11.31±0.2°, 11.84 ± 0.2°, 14.78±0.2°, 15.82±0.2°, 18.70±0.2°, 20.56±0.2°, 21.72±0.2°, 22.90±0.2°, 23.44±0.2°, 25.44±0.2°, 26.95±0.2°, 28.76±0.2°, 34.75±0.2°, 41.46±0.2° and 49.69±0.2°. A test result of a DTA indicates that the peak temperature of thermal decomposition of the compound is 311.6 °C. A characterization result of friction sensitivity indicates that PEP is more sensitive to friction (FS=12N), and a characterization result of impact sensitivity indicates that PEP is more insensitive to impact (IS=9 J). The values of detonation heat, detonation velocity and detonation pressure of the energetic compound, which are obtained by adopting a detonation parameter calculating method reported by literatures and applying a DFT and a Kamlet-Jacob empirical formula are: 6.10 kJ/g, 9.09 km/s and 37.6 GPa respectively.

In one embodiment, a compound (C₅H₁₄N₂)(H₂EA)(ClO₄)₄ (denoted by MPEP) is provided as an energetic material. The compound is crystallized at a *P2₁*/*c* space group of a monoclinic crystal system under 298 K; cell parameters are *a*=12.7872(5) Å, *b*=10.3107(3) Å, *c*=15.9171(5) Å, *α*=*γ*=90°, and *β*=99.987(4)°; and the powder X-ray diffraction (a Cu-*K*_{α} ray) at room temperature occurs but not limited to the diffraction angle 2*θ*, which is about 6.99 ± 0.2°, 14.04±0.2°, 14.94±0.2°, 16.68±0.2°, 18.57±0.2°, 19.53±0.2°, 21.13±0.2°, 22.42±0.2°, 23.88±0.2°, 26.13±0.2°, 26.85±0.2°, 33.33±0.2°, 34.69±0.2° and 47.76±0.2°. A test result of a DTA indicates that MPEP has three decomposition exothermic peaks, and the peak temperatures are 300.0 °C, 323.0 °C and 368.0 °C respectively. A characterization result of friction sensitivity indicates that MPEP is more sensitive to friction (*FS*=9N), and a characterization result of impact sensitivity indicates that MPEP is more insensitive to impact (*IS*=20 J). The values of detonation heat, detonation velocity and detonation pressure of the energetic compound, which are obtained by adopting a detonation parameter calculating method reported by literatures and applying a DFT and a Kamlet-Jacob empirical formula are: 5.86 kJ/g, 8.73 km/s and 34.0 GPa respectively.

In one embodiment, a compound (C₅H₁₄N₂)(H₂EA)(ClO₄)₄ (denoted by HPEP) is provided as an energetic material. The compound is crystallized at a *P2*₁/*n* space group of a monoclinic crystal system under 298 K; cell parameters are *a*=10.7045(2] Å, *b*=8.60470(10) Å, *c*=15.6254(3) Å, *α*=*γ*=90°, and *β*=90.345(2)°; and the powder X-ray diffraction (a Cu-*K*_{α} ray) at room temperature occurs but not limited to the diffraction angle 2*θ*, which is about 7.02±0.2°, 10.98±0.2°, 14.11±0.2°, 15.47±0.2°, 18.35±0.2°, 21.01±0.2°, 22.43±0.2°, 23.54±0.2°, 25.37±0.2°, 26.46±0.2°, 27.50±0.2°, 42.10±0.2° and 48.46±0.2°. A test result of a DTA indicates that the peak temperature of thermal decomposition of HPEP is 324.6 °C. A characterization result of friction sensitivity indicates that HPEP is more sensitive to friction (*FS*=12N), and a characterization result of impact sensitivity indicates that HPEP is more insensitive to impact (*IS*=17.5 J). The values of detonation heat, detonation velocity and detonation pressure of the energetic compound, which are obtained by adopting a detonation parameter calculating method reported by literatures and applying a DFT and a Kamlet-Jacob empirical formula are: 5.87 kJ/g, 8.76 km/s and 34.4 GPa respectively.

Data of the single-crystal structures of PAP-4, PAP-M4 and DAP-O4 is determined on a Rigaku XtaLAB P300DS single-crystal diffractometer (Cu-*K*ₐ, and *λ*=1.54184 Å). Data of the single-crystal structures of PAP-H4 and DAP-M4 is determined on an Agilent SuperNova single-crystal diffractometer (Mo-*K*_{α}, and *λ*=0.71073 Å). Data of the X-ray powder diffraction is tested on an Advance D8 diffractometer (a *θ*-2*θ* scanning manner, and Cu-*K*_{α}). Data of the DTA is tested and determined on a DTA 552-EX anti-explosion DTA of Idea Science Instrument Company of America. The impact sensitivity and the friction sensitivity are determined respectively on a BFH 10 BAM drop hammer impact sensitivity instrument and an FSKM10 BAM friction sensitivity instrument according to the dangerous goods transport standard of the United Nations.

Wherein data of the single-crystal structures of PAP-5 and DAP-5 is determined on the Rigaku XtaLAB P300DS single-crystal diffractometer (Mo-*K*_{α}, and *λ*=0.71073 Å] at the temperature of 223 K; and data of the single-crystal structures of PAP-M5 and PAP-H5 is determined on the Rigaku XtaLAB P300DS single-crystal diffractometer (Cu-*K*ₐ, and *λ*=1.54184 Å) at the temperature of 298 K. Data of the X-ray powder diffraction is tested on the Advance D8 diffractometer (a *θ*-2*θ* scanning manner, and Cu-*K*_{α}). Data of the DTA is tested and determined on the DTA 552-EX anti-explosion DTA of Idea Science Instrument Company of America. The impact sensitivity and the friction sensitivity are determined respectively on the BFH 10 BAM drop hammer impact sensitivity instrument and the FSKM10 BAM friction sensitivity instrument according to the dangerous goods transport standard of the United Nations.

Wherein data of the single-crystal structure of PAP-2 is determined on the Agilent SuperNova single-crystal diffractometer (Cu-*K*ₐ, and *λ*=1.54178 Å); and data of the single-crystal structure of PAP-H2 is determined on the Rigaku XtaLAB P300DS single-crystal diffractometer (Cu-*K*ₐ, and *λ*=1.54184 Å). Data of the X-ray powder diffraction is tested on the Advance D8 diffractometer (a *θ*-2*θ* scanning manner, and Cu-*K*_{α}). Data of the DTA is tested and determined on the DTA 552-EX anti-explosion DTA of Idea Science Instrument Company of America. The impact sensitivity and the friction sensitivity are determined respectively on the BFH 10 BAM drop hammer impact sensitivity instrument and the FSKM10 BAM friction sensitivity instrument according to the dangerous goods transport standard of the United Nations.

Wherein data of the single-crystal structures of DAP-6 and DAP-7 is determined on the Agilent SuperNova single-crystal diffractometer (Cu-*K*ₐ, and *λ*=1.54184 Å) at the temperature of 223 K. Data ofthe X-ray powder diffraction is tested on the Advance D8 diffractometer (a *θ*-2*θ* scanning manner, and Cu-*K*_{α}). Data of the DTA is tested and determined on the DTA 552-EX anti-explosion DTA of Idea Science Instrument Company of America. The impact sensitivity and the friction sensitivity are determined respectively on the BFH 10 BAM drop hammer impact sensitivity instrument and the FSKM10 BAM friction sensitivity instrument according to the dangerous goods transport standard of the United Nations.

Data of the single-crystal structures of EAP, PEP, MPEP and HPEP is determined on the Rigaku XtaLAB P300DS single-crystal diffractometer (Cu-*K*ₐ, and *λ*=1.54184 Å) at room temperature. Data of the X-ray powder diffraction is tested on the Advance D8 diffractometer (a *θ*-2*θ* scanning manner, and Cu-*K*_{α}). Data of the DTA is tested and determined on the DTA 552-EX anti-explosion DTA of Idea Science Instrument Company of America. The impact sensitivity and the friction sensitivity are determined respectively on the BFH 10 BAM drop hammer impact sensitivity instrument and the FSKM10 BAM friction sensitivity instrument according to the dangerous goods transport standard of the United Nations.

### Example 1

Synthesis and testing of (C₄H₁₂N₂)(NH₄)(ClO₄)₃ (PAP-4) (general formula ABX₃; A is a piperazine-1,4-diium ion; B is NH₄⁺; and X is ClO₄⁻.)

### Synthesis method:

1) 5.74 g of perchloric acid solution with the mass percent of 70%-72% was added into 15 mL of water, 2.35 g of ammonium perchlorate was added into the mixed solution while stirring, and the obtained mixed solution was stirred at normal temperature for 5 min;
2) 1.72 g of piperazine anhydrous was added into 5 mL of water for dissolving; and
3) the solution obtained in the step 1) was mixed with the solution obtained in the step 2); the obtained mixed solution was heated to 80 °C, then was stirred for 10 min and was filtered; precipitates were washed by ethanol and then were dried under a vacuum condition, so as to obtain solid powder; and the solid powder was identified as a PAP-4 pure phase through X-ray powder diffraction, and the yield was 80%.

**Powder X-ray diffraction identification pattern:** a powder X-ray diffraction pattern at room temperature is shown in FIG. 9.

**Single crystal structure characterization test:** detailed crystal determination data is shown in Table 1.

**Table 1 Crystal determination data of PAP-4**

| Complex | PAP-4 |
|---|---|
| Formula | C₄H₁₆Cl₃N₃O₁₂ |
| Formula weight | 404.55 |
| *T*/K | 298(2) |
| *λ*/Å | 1.54184 |
| Crystal system | cubic |
| Space group | Fm-3c |
| *a*/Å | 14.5631(3) |
| *V*/Å³ | 3088.60(19) |
| Z | 8 |
| *D*_{c} /g cm⁻³ | 1.737 |
| reflections collected | 3025 |
| unique reflections | 143 |
| *R*ᵢₙₜ | 0.0889 |
| *R*₁ [*I* > 2*σ*(*I*)]^{[a]} | 0.0711 |
| *wR*₂ [*I* > 2*σ*(*I*)^{[b]} | 0.2570 |
| *R*₁ (all data) | 0.0733 |
| *wR*₂ (all data) | 0.2647 |
| GOF on *F*² | 1.242 |

| | |
|---|---|
| ^{[a]} *R*₁ = ∑\|\|*F*_{ο}\| - \|*F*_{c}\|\|/∑\|*F*ₒ\|; ^{[b]} *wR*₂ = {∑w[(*F*ₒ)² - (*F*_{c})²]²/∑w[(*F*ₒ)²]²}^{1/2}; | |

**Differential thermal analysis (DTA) characterization of PAP-4:** a DTA curve of PAP-4 is shown as Fig. 10. It can be known from Fig. 10 that the powder-state energetic compound PAP-4 is decomposed at the peak temperature of decomposition of 288.2 °C, the decomposition peak type is sharp, and the decomposition is rapid.

**Detonation heat, detonation pressure and detonation velocity of energetic compound PAP-4 obtained according to density functional theory (DFT):** the value of decomposition heat of PAP-4 (the value of the decomposition enthalpy is Δ*H*_{det}) is calculated to be about 1.43 kcal/g by adopting the DFT (J. Am. Chem. Soc. 2012, 134, 1422); and according to a Kamlet-Jacob formula, the detonation velocity of PAP-4 is calculated to be about 8.63 km/s, and the detonation pressure of PAP-4 is calculated to be about 32.4 GPa.

**Theoretical specific impulse of energetic compound PAP-4 calculated by DFT and EXPLO5 software:** the enthalpy of formation of PAP-4 is calculated to be about -537.7 kJ/mol by adopting the DFT (J. Am. Chem. Soc. 2012,134, 1422), and the enthalpy of formation is substituted into EXPLO5 v.6.04.02 for calculation to obtain that the value of the theoretical specific impulse of PAP-4 is 264.2 s. As a contrast, by taking a 1,4-diazabicyclo[2.2.2] octane-1,4-diium ion as (C₆H₁₄N₂)[NH₄(ClO₄)₃] (DAP-4) of an A cation, the theoretical enthalpy of formation is -484.0 kJ/mol under the same condition, and the enthalpy of formation is substituted into EXPLO5 v.6.04.02 for calculation to obtain that the value of the theoretical specific impulse of DAP-4 is 253.5 s.

**Volume of gas produced per mole of PAP-4:** a product of complete explosion of an energetic material in an oxygen-free environment is judged according to literatures (J. Am. Chem. Soc.2012, 134, 1422; J. Phys. Chem. A. 2014,118,4575; Chem. Eur. J. 2016, 22, 1141), and final decomposition products are: gaseous substances, such as nitrogen, hydrogen halide, water, carbon dioxide and the like, and solid substances, such as metallic chlorate, a simple substance carbon (if oxygen atoms are not enough to completely translate all carbon atoms into carbon dioxide) and the like. Therefore, after 1 mol of PAP-4 is completely exploded in the oxygen-free environment, 13.75 mol of gas substances are generated, and 3.25 mol of simple substances carbon are left. Under the condition that an enough oxidizing agent (such as common NH₄ClO₄) is mixed, no residue is left after PAP-4 is completely exploded.

### Example 2

Synthesis and testing of (C₅H₁₄N₂)(NH₄)(ClO₄)₃ (PAP-M4) (general formula ABX₃; A is a 1-methylpiperazine-1,4-diium ion; B is NH₄⁺; and X is ClO₄⁻.)

### Synthesis method:

1) 5.74 g of perchloric acid solution with the mass percent of 70%-72% was added into 15 mL of water, 2.35 g of ammonium perchlorate was added into the mixed solution while stirring, and the obtained mixed solution was stirred at normal temperature for 5 min;
2) 2.00 g of 1-methylpiperazine was added into 5 mL of water for dissolving; and
3) the solution obtained in the step 1) was mixed with the solution obtained in the step 2); the obtained mixed solution was stirred for 10 min and was filtered; precipitates were washed by ethanol and then were dried under a vacuum condition, so as to obtain solid powder; and the solid powder was identified as a PAP-M4 pure phase through X-ray powder diffraction, and the yield was 80%.

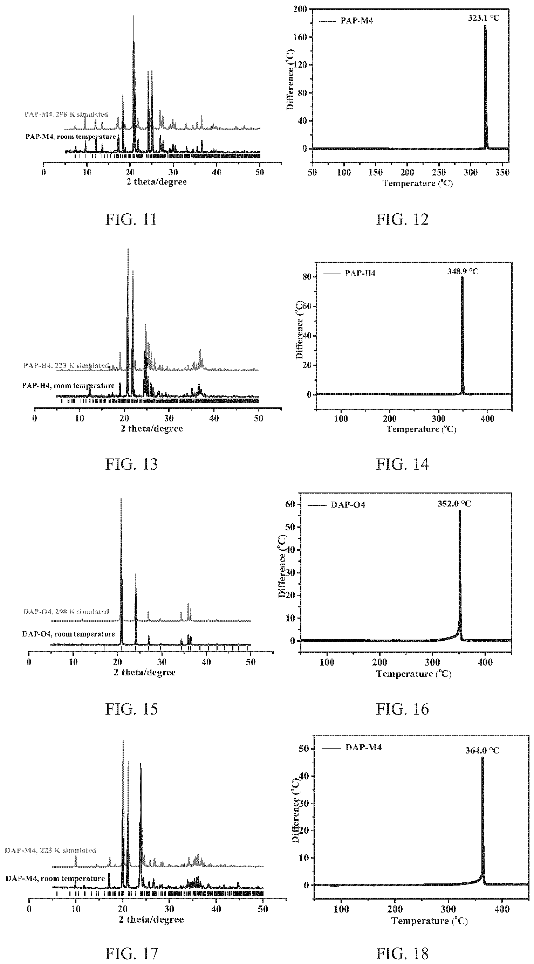

**Powder X-ray diffraction identification pattern:** a powder X-ray diffraction pattern at room temperature is shown in FIG. 11.

**Single crystal structure characterization test:** detailed crystal determination data is shown in Table 2.

**Table 2 Crystal determination data of PAP-M4**

| Complex | PAP-M4 |
|---|---|
| Formula | C_{S}H₁₈Cl₃N₃O₁₂ |
| Formula weight | 418.57 |
| *T*/K | 298(2) |
| *λ*/Å | 1.54178 |
| Crystal system | orthorhombic |
| Space group | Pnma |
| *a*/Å | 10.26733(15) |
| *b*/Å | 14.7004(2) |
| *c*/Å | 20.9914(3) |
| *V*/Å³ | 3168.32(8) |
| Z | 8 |
| *D*_{c} /g cm⁻³ | 1.755 |
| reflections collected | 15561 |
| unique reflections | 3399 |
| *R*ᵢₙₜ | 0.0381 |
| *R*₁ [*I* > 2*σ*(*I*)]^{[a]} | 0.0563 |
| *wR*₂ [*I* > 2*σ*(*I*)]^{[b]} | 0.1611 |
| *R*₁ (all data) | 0.0593 |
| *wR*₂ (all data) | 0.1633 |
| GOF on *F*² | 1.078 |

| | |
|---|---|
| ^{[a]} *R*₁ = ∑\|\|*F*_{ο}\| - \|*F*_{c}\|\|/∑\|*F*ₒ\|; ^{[b]} *wR*₂ = {∑w[(*F*ₒ)² - (*F*_{c})²]²/∑w[(*F*ₒ)²]²}^{1/2}; | |

**Differential thermal analysis (DTA) characterization of PAP-M4:** a DTA curve of PAP-M4 is shown as FIG. 12. It can be known from FIG. 12 that the powder-state energetic compound PAP-M4 is decomposed at the peak temperature of decomposition of 323.1 °C, the decomposition peak type is sharp, and the decomposition is rapid.

**Detonation heat, detonation pressure and detonation velocity of energetic compound PAP-M4 obtained according to density functional theory (DFT):** the value of decomposition heat of PAP-M4 (the value of the decomposition enthalpy is Δ*H*_{det}) is calculated to be about 1.23 kcal/g by adopting the DFT (J. Am. Chem. Soc. 2012, 134, 1422); and according to a Kamlet-Jacob formula, the detonation velocity of PAP-M4 is calculated to be about 8.31 km/s, and the detonation pressure of PAP-M4 is calculated to be about 30.3 GPa.

**Theoretical specific impulse of energetic compound PAP-M4 calculated by DFT and EXPLO5 software:** the enthalpy of formation of PAP-M4 is calculated to be about -859.9 kJ/mol by adopting the DFT (J. Am. Chem. Soc. 2012, 134, 1422), and the enthalpy of formation is substituted into EXPLO5 v.6.04.02 for calculation to obtain that the value of the theoretical specific impulse of PAP-M4 is 241.2 s.

**Volume of gas produced per mole of PAP-M4:** a product of complete explosion of an energetic material in an oxygen-free environment is judged according to literatures (J. Am. Chem. Soc.2012, 134, 1422; J. Phys. Chem. A. 2014, 118, 4575; Chem. Eur. J. 2016, 22, 1141), and final decomposition products are: gaseous substances, such as nitrogen, hydrogen halide, water, carbon dioxide and the like, and solid substances, such as metallic chlorate, a simple substance carbon (if oxygen atoms are not enough to completely translate all carbon atoms into carbon dioxide) and the like. Therefore, after 1 mol of PAP-M4 is completely exploded in the oxygen-free environment, 14.25 mol of gas substances are generated, and 2.75 mol of simple substances carbon are left. Under the condition that an enough oxidizing agent (such as common NH₄ClO₄) is mixed, no residue is left after PAP-M4 is completely exploded.

### Example 3

Synthesis and testing of (C₅H₁₄N₂)(NH₄)(ClO₄)₃ (PAP-H4) (general formula ABX₃; A is a 1,4-diazepane-1,4-diium ion; B is NH₄⁺; and X is ClO₄⁻.)

### Synthesis method:

1) 5.74 g of perchloric acid solution with the mass percent of 70%-72% was added into 15 mL of water, 2.35 g of ammonium perchlorate was added into the mixed solution while stirring, and the obtained mixed solution was stirred at normal temperature for 5 min;
2) 2.00 g of homopiperazine was added into 5 mL of water for dissolving; and
3) the solution obtained in the step 1) was mixed with the solution obtained in the step 2); the obtained mixed solution was stirred for 10 min and was filtered; precipitates were washed by ethanol and then were dried under a vacuum condition, so as to obtain solid powder; and the solid powder was identified as a PAP-H4 pure phase through X-ray powder diffraction, and the yield was 80%.

**Powder X-ray diffraction identification pattern:** a powder X-ray diffraction pattern at room temperature is shown in FIG. 13.

**Single crystal structure characterization test:** detailed crystal determination data is shown in Table 3.

**Table 3 Crystal determination data of PAP-H4**

| Complex | PAP-H4 |
|---|---|
| Formula | C₅H₁₈Cl₃N₃O₁₂ |
| Formula weight | 418.57 |
| *T*/K | 223(2) |
| *λ*/Å | 0.71073 |
| Crystal system | monoclinic |
| Space group | P2₁/n |
| *a*/Å | 19.7404(7) |
| *b*/Å | 14.3294(5) |
| *c*/Å | 21.2948(8) |
| *β*/° | 90.075(3) |
| *V*/Å³ | 6023.6(4) |
| Z | 16 |
| *D*_{c} /g cm⁻³ | 1.846 |
| reflections collected | 32844 |
| unique reflections | 19613 |
| *R*ᵢₙₜ | 0.0487 |
| *R*₁ [*I* > 2*σ*(*I*)]^{[a]} | 0.0795 |
| *wR*₂ [*I* > 2*σ*(*I*)]^{[b]} | 0.1680 |
| *R*₁ (all data) | 0.1417 |
| *wR*₂ (all data) | 0.2062 |
| GOF on *F*² | 1.045 |

| | |
|---|---|
| ^{[a]} *R*₁ = ∑\|\|*F*_{ο}\| - \|*F*_{c}\|\|/∑\|*F*ₒ\|; ^{[b]} *wR*₂ = {∑w[(*F*ₒ)² - (*F*_{c})²]²/∑w[(*F*ₒ)²]²}^{1/2}; | |

**Differential thermal analysis (DTA) characterization of PAP-H4:** a DTA curve of PAP-H4 is shown as Fig. 14. It can be known from Fig. 14 that the powder-state energetic compound PAP-H4 is decomposed at the peak temperature of decomposition of 348.9 °C, the decomposition peak type is sharp, and the decomposition is rapid.

**Detonation heat, detonation pressure and detonation velocity of energetic compound PAP-H4 obtained according to density functional theory (DFT):** the value of decomposition heat of PAP-H4 (the value of the decomposition enthalpy is Δ*H*_{det}) is calculated to be about 1.38 kcal/g by adopting the DFT (J. Am. Chem. Soc. 2012, 134, 1422); and according to a Kamlet-Jacob formula, the detonation velocity of PAP-H4 is calculated to be about 8.76 km/s, and the detonation pressure of PAP-H4 is calculated to be about 34.3 GPa.

**Theoretical specific impulse of energetic compound PAP-H4 calculated by DFT and EXPLO5 software:** the enthalpy of formation of PAP-H4 is calculated to be about -600.4 kJ/mol by adopting the DFT (J. Am. Chem. Soc. 2012, 134, 1422), and the enthalpy of formation is substituted into EXPLO5 v.6.04.02 for calculation to obtain that the value of the theoretical specific impulse of PAP-H4 is 255.4 s.

**Volume of gas produced per mole of PAP-H4:** a product of complete explosion of an energetic material in an oxygen-free environment is judged according to literatures (J. Am. Chem. Soc.2012, 134, 1422; J. Phys. Chem. A. 2014, 118, 4575; Chem. Eur. J. 2016, 22, 1141), and final decomposition products are: gaseous substances, such as nitrogen, hydrogen halide, water, carbon dioxide and the like, and solid substances, such as metallic chlorate, a simple substance carbon (if oxygen atoms are not enough to completely translate all carbon atoms into carbon dioxide) and the like. Therefore, after 1 mol of PAP-H4 is completely exploded in the oxygen-free environment, 14.25 mol of gas substances are generated, and 2.75 mol of simple substances carbon are left. Under the condition that an enough oxidizing agent (such as common NH₄ClO₄) is mixed, no residue is left after PAP-H4 is completely exploded.

### Example 4

Synthesis and test of (C₆H₁₄N₂O)(NH₄)(ClO₄)₃ (DAP-O4) (general formula ABX₃, where A is 1-hydroxy-1,4-diazabicyclo[2.2.2]octane-1,4-diium, B is NH₄⁺ and X is ClO₄⁻)

### Synthesis method:

1) 5.74 g of a perchloric acid solution with a mass fraction of 70-72% was added into 15 mL of water, 2.35 g of ammonium perchlorate was added again while the solution was stirred, and the mixture was stirred for 5 min at normal temperature;
2) 2.24g of 1,4-diazabicyclo[2.2.2]octane was slowly added into 5.9 mL of 30% hydrogen peroxide in an ice-water bath, the mixture was stirred for 5 min, then the temperature was recovered to room temperature, and the mixture was stirred for 30 min; and
3) the solutions in step 1) and step 2) were mixed, stirred for 10 min and filtered, a precipitate was washed with ethanol, and the precipitate was subjected to vacuum drying to obtain a solid powder which was identified as a DAP-O4 pure phase through X-ray powder diffraction, with a yield of 85%.

**Powder X-ray diffraction identification pattern:** a powder X-ray diffraction pattern at room temperature is shown in FIG. 15.

**Single crystal structure characterization test:** detailed crystal determination data is shown in Table 4.

**Table 4 Crystal determination data of the DAP-O4**

| Complex | DAP-O4 |
|---|---|
| Formula | C₆H₁₈Cl₃N₃O₁₃ |
| Formula weight | 446.55 |
| *T*/K | 298(2) |
| *λ*/Å | 1.54178 |
| Crystal system | Cubic |
| Space group | *Fm-3c* |
| *a*/Å | 14.76270 (10) |
| *V*/Å³ | 3217.34(7) |
| Z | 8 |
| *D*_{c} /g cm⁻³ | 1.844 |
| reflections collected | 5939 |
| unique reflections | 168 |
| *R*ᵢₙₜ | 0.0373 |
| R₁[*I*>2*σ*(*I*)]^{[a]} | 0.0298 |
| *wR*₂[*I*>2*σ*(*I*)^{[b]} | 0.0852 |
| *R*₁ (all data) | 0.0306 |
| *wR*₂ (all data) | 0.0860 |
| GOF on *F*² | 1.151 |

| | |
|---|---|
| ^{[a]} *R*₁ = ∑\|\|*F*_{ο}\| - \|*F*_{c}\|\|/∑\|*F*ₒ\|; ^{[b]} *wR*₂ = {∑w[(*F*ₒ)² - (F_{c})²]²/∑w[(*F*ₒ)²]²}^{1/2}; | |

**Differential thermal analysis (DTA) characterization of DAP-O4:** a DTA curve of the DAP-O4 is shown in FIG. 16. It can be known from FIG. 16 that the energetic compound DAP-O4 in a powder state is decomposed at a decomposition peak temperature of 352.0 °C, the decomposition peak pattern is sharp, and the energetic compound is decomposed rapidly.

**Detonation heat, detonation pressure and detonation velocity of energetic compound DAP-O4 obtained according to density functional theory (DFT):** a decomposition heat value (decomposition enthalpy value Δ*H*_{det}) of the DAP-O4 calculated (J. Am. Chem. Soc. 2012, 134, 1422) based on the DFT is about 1.48 kcal/g, and calculated according to a Kamlet-Jacob formula, an explosive velocity of the DAP-O4 is about 8.90 km/s, and an explosive pressure is about 35.7 GPa.

**Theoretical specific impulse of energetic compound DAP-O4 calculated by DFT and EXPLO5 software:** the enthalpy of formation of the DAP-O4 calculated (J. Am. Chem. Soc. 2012, 134, 1422) based on the DFT is about -436.1 kJ/mol, and the theoretical specific impulse value of the energetic compound DAP-O4 calculated by substituting the enthalpy of formation into EXPLO5 v.6.04.02 is 262.4 s.

**Volume of gas produced per mole of DAP-O4:** with respect to judgment on products due to complete explosion of the energetic material in an oxygen-free environment, decomposition products thereof are finally gaseous substances such as nitrogen, halogen hydride, water and carbon dioxide, and solid substances such as chlorates of metals and elemental carbon (in a case that oxygen atoms are not enough to convert all carbon atoms completely into carbon dioxide) according to literatures (J. Am. Chem. Soc.2012, 134, 1422;J. Phys. Chem. A. 2014, 118,4575; Chem. Eur. J. 2016, 22, 1141). Therefore, 1 mol of the DAP-O4 can generate 14.75 mol of gas substances after complete explosion in the oxygen-free environment with 3.25 mol of elemental carbon left. Under a condition that enough oxidants (for example, common NH₄ClO₄) are mixed, the DAP-O4 is free of solid residues after complete explosion.

### Example 5

Synthesis and test of (C₇H₁₆N₂)(NH₄)(ClO₄)₃ (DAP-M4) (general formula ABX₃, where A is 1-methyl-1,4-diazabicyclo[2.2.2]octane-1,4-diium, B is NH₄⁺ and X is ClO₄⁻)

### Synthesis method:

1) 5.74 g of a perchloric acid solution with a mass fraction of 70-72% was added into 15 mL of water, 2.35 g of ammonium perchlorate was added again while the solution was stirred, and the mixture was stirred for 5 min at normal temperature;
2) 5.08 g of a 1-methyl-1,4-diazabicyclo[2.2.2]octane iodide was added into 5 mL of water to be dissolved; and
3) the solutions in step 1) and step 2) were mixed, stirred for 10 min and filtered, a precipitate was washed with ethanol, and the precipitate was subjected to vacuum drying to obtain a solid powder which was identified as a DAP-M4 pure phase through X-ray powder diffraction, with a yield of 70%.

**Powder X-ray diffraction identification pattern:** a powder X-ray diffraction pattern at room temperature is shown is shown in FIG. 17.

**Single crystal structure characterization test:** detailed crystal determination data is shown in Table 5.

**Table 5 Crystal determination data of the DAP-M4**

| Complex | DAP-M4 |
|---|---|
| Formula | C₇H₂₀Cl₃N₃O₁₂ |
| Formula weight | 444.61 |
| *T*/K | 223(2) |
| *λ*/Å | 0.71073 |
| Crystal system | monoclinic |
| Space group | *P*2₁ |
| *a*/Å | 10.15203 (16) |
| *b*/Å | 10.9824 (2) |
| *c*/Å | 14.7774(2) |
| *β*/° | 89.8562 (14) |
| *V*/Å³ | 1647.58 (5) |
| Z | 4 |
| *D*_{c} /g cm⁻³ | 1.792 |
| reflections collected | 27589 |
| unique reflections | 12392 |
| *R*ᵢₙₜ | 0.0324 |
| *R*₁ [*I* > 2*σ*(*I*)]^{[a]} | 0.0364 |
| *wR*₂[*I* > 2*σ*(*I*)]^{[b]} | 0.0909 |
| *R*₁ (all data) | 0.0414 |
| *wR*₂ (all data) | 0.0952 |
| GOF on *F*² | 1.040 |

| | |
|---|---|
| ^{[a]} *R*₁ = ∑\|\|*F*_{ο}\| - \|*F*_{c}\|\|/∑\|*F*ₒ\|; ^{[b]} *wR*₂ = {∑w[(*F*ₒ)² - (*F*_{c})²]²/∑w[(*F*ₒ)²]²}^{1/2}; | |

**Differential thermal analysis (DTA) characterization of DAP-M4:** a DTA curve of the DAP-M4 is shown in FIG. 18. It can be known from FIG. 18 that the energetic compound DAP-M4 in a powder state is decomposed at a decomposition peak temperature of 364.0°C, the decomposition peak pattern is sharp, and the energetic compound is decomposed rapidly.

**Detonation heat, detonation pressure and detonation velocity of energetic compound DAP-M4 obtained according to density functional theory (DFT):** a decomposition heat value (decomposition enthalpy value Δ*H*_{det}) of the DAP-M4 calculated (J. Am. Chem. Soc. 2012, 134, 1422) based on the DFT is about 1.20 kcal/g, and calculated according to a Kamlet-Jacob formula, an explosive velocity of the DAP-M4 is about 8.08 km/s, and an explosive pressure is about 28.8 GPa.

**Theoretical specific impulse of energetic compound DAP-M4 calculated by DFT and EXPLO5 software:** the enthalpy of formation of the DAP-M4 calculated (J. Am. Chem. Soc. 2012, 134, 1422) based on the DFT is about -839.1 kJ/mol, and the theoretical specific impulse value of the energetic compound DAP-M4 calculated by substituting the enthalpy of formation into EXPLO5 v.6.04.02 is 225.2 s.

### Volume of gas produced per mole of DAP-M4:

with respect to judgment on products due to complete explosion of the energetic material in an oxygen-free environment, decomposition products thereof are finally gaseous substances such as nitrogen, halogen hydride, water and carbon dioxide, and solid substances such as chlorates of metals and elemental carbon (in a case that oxygen atoms are not enough to convert all carbon atoms completely into carbon dioxide) according to literatures (. Am. Chem. Soc .2012, 134, 1422; J. Phys. Chem. A. 2014, 118, 4575; Chem. Eur. J. 2016, 22, 1141). Therefore, 1 mol of the DAP-M4 can generate 14.75 mol of gas substances after complete explosion in the oxygen-free environment with 5.25 mol of elemental carbon left. Under a condition that enough oxidants (for example, common NH₄ClO₄) are mixed, the DAP-M4 is free of solid residues after complete explosion.

### Example 6

Synthesis and test of (C₄H₁₂N₂)[Ag(ClO₄)₃] (PAP-5) (general formula ABX₃, where A is piperazine-1,4-diium, B is Ag⁺ and X is ClO₄⁻)

### Synthesis method:

1) 5.74 g of a perchloric acid solution with a mass fraction of 70-72% was added into 5 mL of water, 4.14 g of silver perchlorate was added again while the solution was stirred, and the mixture was stirred for 5 min at normal temperature;
2) 2.19 g of piperazine was added into 5 mL of water to be dissolved; and
3) the solutions in step 1) and step 2) were mixed, stirred for 10 min and filtered, a precipitate was washed with ethanol, and the precipitate was subjected to vacuum drying to obtain a solid powder which was identified as a PAP-5 pure phase through X-ray powder diffraction, with a yield of 75%.

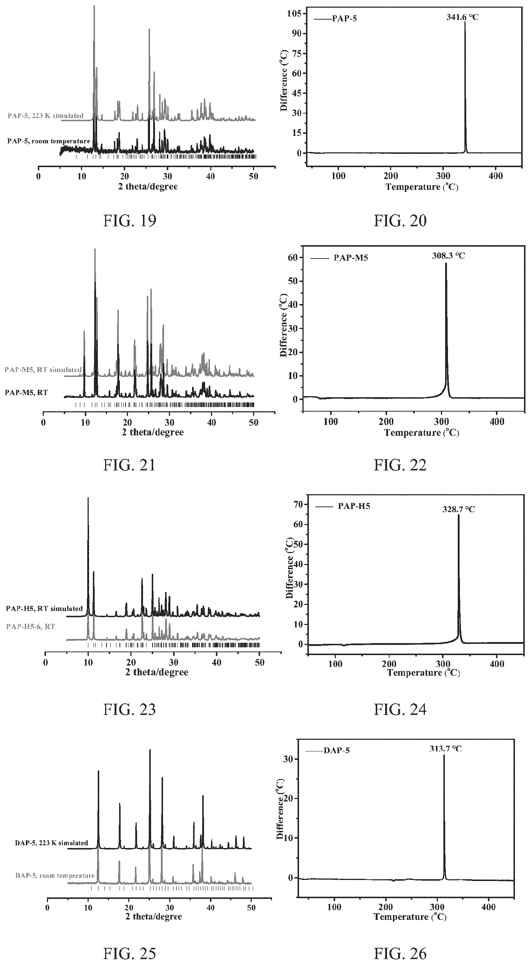

**Powder X-ray diffraction identification pattern:** a powder X-ray diffraction pattern at room temperature is shown in FIG. 19.

**Single crystal structure characterization test:** detailed crystal determination data is shown in Table 6.

**Table 6 Crystal determination data of the PAP-5**

| Complex | PAP-5 |
|---|---|
| Formula | C₄H₁₂AgCl₃N₂O₁₂ |
| Formula weight | 494.38 |
| *T*/K | 223(2) |
| *λ*/Å | 0.71073 |
| Crystal system | Monoclinic |
| Space group | *P*2₁/*c* |
| *a*/Å | 10.1221(2) |
| *b*/Å | 9.6470 (2) |
| *c*/Å | 13.2672 (3) |
| *β*/° | 91.7815 (19) |
| *V*/Å³ | 1294.89 (5) |
| Z | 4 |
| *D*_{c} /g cm⁻³ | 2.536 |
| reflections collected | 16140 |
| unique reflections | 3744 |
| *R*ᵢₙₜ | 0.0544 |
| *R*₁ [*I* > 2*σ*(*I*)^{[a]} | 0.0301 |
| *wR*₂[*I* > 2*σ*(*I*)]^{[b]} | 0.0692 |
| *R*₁ (all data) | 0.0329 |
| *wR*₂ (all data) | 0.0715 |
| GOF on *F*² | 1.026 |

| | |
|---|---|
| ^{[a]} *R*₁ = ∑\|\|*F*_{ο}\| - \|*F*_{c}\|\|/∑\|*F*ₒ\|; ^{[b]} *wR*₂ = {∑w[(*F*ₒ)² - (F_{c})²]²/∑w[(*F*ₒ)²]²}^{1/2}; | |

**Differential thermal analysis (DTA) characterization of PAP-5:** a DTA curve of the PAP-5 is shown in FIG. 20. It can be known from FIG. 20 that the energetic compound PAP-5 in a powder state is decomposed at a decomposition peak temperature of 341.6°C, the decomposition peak pattern is sharp, and the energetic compound is decomposed rapidly.

**Detonation heat, detonation pressure and detonation velocity of energetic compound PAP-5 obtained according to density functional theory (DFT):** a decomposition heat value (decomposition enthalpy value Δ*H*_{det}) of the PAP-5 calculated (J. Am. Chem. Soc. 2012, 134, 1422) based on the DFT is about 1.17 kcal/g, and calculated according to a Kamlet-Jacob formula, an explosive velocity of the PAP-5 is about 8.96 km/s, and an explosive pressure is about 42.4 GPa.

**Volume of** gas **produced per mole of PAP-5:** with respect to judgment on products due to complete explosion of the energetic material in an oxygen-free environment, decomposition products thereof are finally gaseous substances such as nitrogen, halogen hydride, water and carbon dioxide, and solid substances such as chlorates of metals and elemental carbon (in a case that oxygen atoms are not enough to convert all carbon atoms completely into carbon dioxide) according to literatures (J. Am. Chem. Soc.2012, 134, 1422;J. Phys. Chem. A. 2014, 118,4575; Chem. Eur. J. 2016, 22, 1141). Therefore, 1 mol of the PAP-5 can generate 11.5 mol of gas substances after complete explosion in the oxygen-free environment with 0.5 mol of elemental carbon and 1 mol of a silver chloride solid left. Under a condition that enough oxidants (for example, common NH₄ClO₄) are mixed, there is 1 mol of the silver chloride solid after complete explosion of 1 mol of PAP-5.

### Example 7

Synthesis and test of (C₅H₁₄N₂)[Ag(ClO₄)₃] (PAP-M5) (general formula ABX₃, where A is 1-methyl piperazine-1,4-diium, B is Ag⁺ and X is ClO₄⁻)

### Synthesis method:

1) 5.74 g of a perchloric acid solution with a mass fraction of 70-72% was added into 2 mL of water, 4.14 g of silver perchlorate was added again while the solution was stirred, and the mixture was stirred for 5 min at normal temperature;
2) 2.00 g of 1-methyl piperazine was added into 2 mL of water to be dissolved; and
3) the solutions in step 1) and step 2) were mixed, stirred for 10 min and filtered, a precipitate was washed with ethanol, and the precipitate was subjected to vacuum drying to obtain a solid powder which was identified as a PAP-M5 pure phase through X-ray powder diffraction, with a yield of 75%.

**Powder X-ray diffraction identification pattern:** a powder X-ray diffraction pattern at room temperature is shown in FIG. 21.

**Single crystal structure characterization test:** detailed crystal determination data is shown in Table 7.

**Table 7 Crystal determination data of the PAP-M5**

| Complex | PAP-M5 |
|---|---|
| Formula | C₅H₁₄AgCl₃N₃O₁₂ |
| Formula weight | 508.4 |
| *T*/K | 298(2) |
| *λ*/Å | 1.54184 |
| Crystal system | Orthorhombic |
| Space group | *Pnma* |
| *a*/Å | 10.1827 (3) |
| *b*/Å | 13.8975 (4) |
| *c*/Å | 20.2734 (5) |
| *V*/Å³ | 2868.97 (14) |
| Z | 8 |
| *D*_{c}/g cm⁻³ | 2.354 |
| reflections collected | 18231 |
| unique reflections | 3122 |
| *R*ᵢₙₜ | 0.0574 |
| *R*₁ [*I* > 2σ(*I*)]^{[a]} | 0.1003 |
| *wR*₂[*I* > 2*σ*(*I*)]^{[b]} | 0.3118 |
| *R*₁ (all data) | 0.1100 |
| *wR*₂ (all data) | 0.3259 |
| GOF on *F*² | 1.178 |

| | |
|---|---|
| ^{[a]} *R*₁ = ∑\|\|*F*_{ο}\| - \|*F*_{c}\|\|/∑\|*F*ₒ\|; ^{[b]} *wR*₂ = {∑w[(*F*ₒ)² - (*F*_{c})²]²/∑w[(*F*ₒ)²]²}^{1/2}; | |

**Differential thermal analysis (DTA) characterization of PAP-M5:** a DTA curve of the PAP-M5 is shown in FIG. 22. It can be known from FIG. 22 that the energetic compound PAP-M5 in a powder state is decomposed at a decomposition peak temperature of 308.3°C, the decomposition peak pattern is sharp, and the energetic compound is decomposed rapidly.

**Detonation heat, detonation pressure and detonation velocity of energetic compound PAP-M5 obtained according to density functional theory (DFT):** a decomposition heat value (decomposition enthalpy value Δ*H*_{det}) of the PAP-M5 calculated (J. Am. Chem. Soc. 2012, 134, 1422) based on the DFT is about 1.29 kcal/g, and calculated according to a Kamlet-Jacob formula, an explosive velocity of the PAP-M5 is about 8.73 km/s, and an explosive pressure is about 39.2 GPa.

**Volume of gas produced per mole of PAP-M5:** with respect to judgment on products due to complete explosion of the energetic material in an oxygen-free environment, decomposition products thereof are finally gaseous substances such as nitrogen, halogen hydride, water and carbon dioxide, and solid substances such as chlorates of metals and elemental carbon (in a case that oxygen atoms are not enough to convert all carbon atoms completely into carbon dioxide) according to literatures (J. Am. Chem. Soc.2012, 134, 1422;J. Phys. Chem. A. 2014, 118,4575; Chem. Eur. J. 2016, 22, 1141). Therefore, 1 mol of the PAP-M5 can generate 12 mol of gas substances after complete explosion in the oxygen-free environment with 2 mol of elemental carbon and 1 mol of a silver chloride solid left. Under a condition that enough oxidants (for example, common NH₄ClO₄) are mixed, there is 1 mol of the silver chloride solid after complete explosion of 1 mol of PAP-M5.

### Example 8

Synthesis and test of (C₅H₁₄N₂)[Ag(ClO₄)₃] (PAP-H5) (general formula ABX₃, where A is 1,4-diazepane-1,4-diium, B is Ag⁺ and X is ClO₄⁻)

### Synthesis method:

1) 5.74 g of a perchloric acid solution with a mass fraction of 70-72% was added into 2 mL of water, 4.14 g of silver perchlorate was added again while the solution was stirred, and the mixture was stirred for 5 min at normal temperature;
2) 2.00 g of homopiperazine was added into 2 mL of water to be dissolved; and
3) the solutions in step 1) and step 2) were mixed, stirred for 10 min and filtered, a precipitate was washed with ethanol, and the precipitate was subjected to vacuum drying to obtain a solid powder which was identified as a PAP-H5 pure phase through X-ray powder diffraction, with a yield of 75%.

**Powder X-ray diffraction identification pattern:** a powder X-ray diffraction pattern at room temperature is shown in FIG. 23.

**Single crystal structure characterization test:** detailed crystal determination data is shown in Table 8.

**Table 8 Crystal determination data of the PAP-H5**

| Complex | PAP-H5 |
|---|---|
| Formula | C₅H₁₄AgCl₃N₃O₁₂ |
| Formula weight | 508.4 |
| *T*/K | 298(2) |
| *λ*/Å | 1.54184 |
| Crystal system | Monoclinic |
| Space group | P2₁/n |
| *a*/Å | 10.7045 (2) |
| *b*/Å | 8.60470 (10) |
| *c*/Å | 15.6254 (3) |
| *β*/° | 90.345 (2) |
| *V*/Å³ | 1439.21 (4) |
| Z | 4 |
| *D*_{c} /g cm⁻³ | 2.346 |
| reflections collected | 12166 |
| unique reflections | 2977 |
| *R*ᵢₙₜ | 0.0401 |
| *R*₁ [*I* > 2*σ*(*I*)]^{[a]} | 0.0419 |
| *wR*₂[*I* > 2*σ*(*I*)]^{[b]} | 0.1142 |
| *R*₁ (all data) | 0.0447 |
| *wR*₂ (all data) | 0.1237 |
| GOF on *F*² | 1.091 |

| | |
|---|---|
| ^{[a]} *R*₁ = ∑\|\|*F*_{ο}\| - \|*F*_{c}\|\|/∑\|*F*ₒ\|; ^{[b]} *wR*₂ = {∑w[(*F*ₒ)² - (*F*_{c})²]²/∑w[(*F*ₒ)²]²}^{1/2}; | |

**Differential thermal analysis (DTA) characterization of PAP-H5:** a DTA curve of the PAP-H5 is shown in FIG. 24. It can be known from FIG. 24 that the energetic compound PAP-H5 in a powder state is decomposed at a decomposition peak temperature of 328.7°C, the decomposition peak pattern is sharp, and the energetic compound is decomposed rapidly.

**Detonation heat, detonation pressure and detonation velocity of energetic compound PAP-H5 obtained according to density functional theory (DFT):** a decomposition heat value (decomposition enthalpy value Δ*H*_{det}) of the PAP-H5 calculated (J. Am. Chem. Soc. 2012, 134, 1422) based on the DFT is about 1.28 kcal/g, and calculated according to a Kamlet-Jacob formula, an explosive velocity of the PAP-H5 is about 8.69 km/s, and an explosive pressure is about 38.7 GPa.

**Volume of gas produced per mole of PAP-H5:** with respect to judgment on products due to complete explosion of the energetic material in an oxygen-free environment, decomposition products thereof are finally gaseous substances such as nitrogen, halogen hydride, water and carbon dioxide, and solid substances such as chlorates of metals and elemental carbon (in a case that oxygen atoms are not enough to convert all carbon atoms completely into carbon dioxide) according to literatures (J. Am. Chem. Soc.2012, 134, 1422;J. Phys. Chem. A. 2014, 118,4575; Chem. Eur. J. 2016, 22, 1141). Therefore, 1 mol of the PAP-H5 can generate 12 mol of gas substances after complete explosion in the oxygen-free environment with 2 mol of elemental carbon and 1 mol of a silver chloride solid left. Under a condition that enough oxidants (for example, common NH₄ClO₄) are mixed, there is 1 mol of the silver chloride solid after complete explosion of 1 mol of PAP-H5.

### Example 9

Synthesis and test of (C₆H₁₄N₂)[Ag(ClO₄)₃] (DAP-5) (general formula ABX₃, where A is 1,4-diazabicyclo[2.2.2]octane-1,4-diium, B is Ag⁺ and X is ClO₄⁻)

### Synthesis method:

1) 5.74 g of a perchloric acid solution with a mass fraction of 70-72% was added into 5 mL of water, 4.14 g of silver perchlorate was added again while the solution was stirred, and the mixture was stirred for 5 min at normal temperature;
2) 2.24 g of 1,4-diazabicyclo[2.2.2]octane was added into 5 mL of water to be dissolved; and
3) the solutions in step 1) and step 2) were mixed, stirred for 10 min and filtered, a precipitate was washed with ethanol, and the precipitate was subjected to vacuum drying to obtain a solid powder which was identified as a DAP-5 pure phase through X-ray powder diffraction, with a yield of 90%.

**Powder X-ray diffraction identification pattern:** a powder X-ray diffraction pattern at room temperature is shown in FIG. 25.

**Single crystal structure characterization test:** detailed crystal determination data is shown in Table 9.

**Table 9 Crystal determination data of the DAP-5**

| Complex | DAP-5 |
|---|---|
| Formula | C₆H₁₄AgCl₃N₃O₁₂ |
| Formula weight | 520.41 |
| *T*/K | 223(2) |
| *λ*/Å | 0.71073 |
| Crystal system | Cubic |
| Space group | *Pa*-3 |
| *a*/Å | 14.1379 (3) |
| *V*/Å³ | 2825.86 (16) |
| Z | 8 |
| *D*_{c}/g cm⁻³ | 2.446 |
| reflections collected | 14902 |
| unique reflections | 929 |
| *R*ᵢₙₜ | 0.0677 |
| *R*₁ [*I* > 2*σ*(*I*)]^{[a]} | 0.0212 |
| *wR*₂[*I* > 2*σ*(*I*)]^{[b]} | 0.0583 |
| *R*₁ (all data) | 0.0226 |
| *wR*₂ (all data) | 0.0595 |
| GOF on *F*² | 1.110 |

| | |
|---|---|
| ^{[a]} *R*₁ = ∑\|\|*F*_{ο}\| - \|*F*_{c}\|\|/∑\|*F*ₒ\|; ^{[b]} *wR*₂ = {∑w[(*F*ₒ)² - (*F*_{c})²]²/∑w[(*F*ₒ)²]²}^{1/2}; | |

**Differential thermal analysis (DTA) characterization of DAP-5:** a DTA curve of the **DAP-5** is shown in FIG. 26. It can be known from FIG. 26 that the energetic compound DAP-5 in a powder state is decomposed at a decomposition peak temperature of 313.7 °C, the decomposition peak pattern is sharp, and the energetic compound is decomposed rapidly.

**Detonation heat, detonation pressure and detonation velocity of energetic compound DAP-5 obtained according to density functional theory (DFT):** a decomposition heat value (decomposition enthalpy value Δ*H*_{det}) of the DAP-5 calculated (J. Am. Chem. Soc. 2012, 134, 1422) based on the DFT is about 1.14 kcal/g, and calculated according to a Kamlet-Jacob formula, an explosive velocity of the DAP-5 is about 8.59 km/s, and an explosive pressure is about 38.5 GPa.

**Volume of gas produced per mole of DAP-5:** with respect to judgment on products due to complete explosion of the energetic material in an oxygen-free environment, decomposition products thereof are finally gaseous substances such as nitrogen, halogen hydride, water and carbon dioxide, and solid substances such as chlorates of metals and elemental carbon (in a case that oxygen atoms are not enough to convert all carbon atoms completely into carbon dioxide) according to literatures (J. Am. Chem. Soc.2012, 134, 1422;J. Phys. Chem. A. 2014, 118,4575; Chem. Eur. J. 2016, 22, 1141). Therefore, 1 mol of the DAP-5 could generate 12 mol of gas substances after complete explosion in the oxygen-free environment with 3 mol of elemental carbon and 1 mol of a silver chloride solid left. Under a condition that enough oxidants (for example, common NH₄ClO₄) are mixed, there is 1 mol of the silver chloride solid after complete explosion of 1 mol of DAP-5.

### Example 10

Synthesis and test of (C₄H₁₂N₂)[K(ClO₄)₃] (PAP-2) (general formula ABX₃, where A is piperazine-1,4-diium, B is K⁺ and X is ClO₄⁻)

### Synthesis method:

1) 1.72 g of piperazine and 0.25 g of homopiperazine was added into 5 mL of water to be dissolved, 8.61 g of a perchloric acid solution with a mass fraction of 70-72% was added, and the mixture was stirred for 5 min at normal temperature;
2) 2.77 g of potassium perchlorate was added to 5 mL of water and heated and stirred to dissolve; and
3) the solutions in step 1) and step 2) were mixed, stirred for 30 min and filtered, a precipitate was washed with ethanol, and the precipitate was subjected to vacuum drying to obtain a solid powder which was identified as a PAP-2 pure phase through X-ray powder diffraction, with a yield of 70%.

**Single crystal structure characterization test:** detailed crystal determination data is shown in Table 10.

**Table 10 Crystal determination data of the PAP-2**

| Complex | PAP-2 |
|---|---|
| Formula | C₄H₁₂KCl₃N₂O₁₂ |
| Formula weight | 425.61 |
| *T*/K | 284 (17) |
| *λ*/Å | 1.54178 |
| Crystal system | Orthorhombic |
| Space group | *Pbcm* |
| *a*/Å | 10.3353 (6) |
| *b*/Å | 9.6777 (6) |
| *c*/Å | 13.9862 (10) |
| *V*/Å³ | 1398.93 (16) |
| Z | 4 |
| *D*_{c} /g cm⁻³ | 2.021 |
| reflections collected | 7003 |
| unique reflections | 1317 |
| *R*ᵢₙₜ | 0.0715 |
| *R*₁ [*I* > 2σ(*I*)]^{[a]} | 0.0704 |
| *wR*₂ [*I* > 2*σ*(*I*)]^{[b]} | 0.1849 |
| *R*₁ (all data) | 0.0804 |
| *wR*₂ (all data) | 0.1998 |
| GOF on *F*² | 1.056 |

| | |
|---|---|
| ^{[a]} *R*₁ = ∑\|\|*F*_{ο}\| - \|*F*_{c}\|\|/∑\|*F*ₒ\|; ^{[b]} *wR*₂ = {∑w[(*F*ₒ)² - (*F*_{c})²]²/∑w[(*F*ₒ)²]²}^{1/2}; | |

**Detonation heat, detonation pressure and detonation velocity of energetic compound PAP-2 obtained according to density functional theory (DFT):** a decomposition heat value (decomposition enthalpy value Δ*H*_{det}) of the PAP-2 calculated (J. Am. Chem. Soc. 2012, 134, 1422) based on the DFT is about 1.29 kcal/g, and calculated according to a Kamlet-Jacob formula, an explosive velocity of the PAP-2 is about 8.78 km/s, and an explosive pressure is about 36.6 GPa.

**Volume of gas produced per mole of PAP-2:** with respect to judgment on products due to complete explosion of the energetic material in an oxygen-free environment, decomposition products thereof were finally gaseous substances such as nitrogen, halogen hydride, water and carbon dioxide, and solid substances such as chlorates of metals and elemental carbon (in a case that oxygen atoms are not enough to convert all carbon atoms completely into carbon dioxide) according to literatures (J. Am. Chem. Soc.2012, 134, 1422; J. Phys. Chem. A. 2014, 118,4575; Chem. Eur. J. 2016, 22, 1141). Therefore, 1 mol of the PAP-2 could generate 11.5 mol of gas substances after complete explosion in the oxygen-free environment with 0.5 mol of elemental carbon and 1 mol of a potassium chloride solid left. Under a condition that enough oxidants (for example, common NH₄ClO₄) were mixed, there was 1 mol of the potassium chloride solid after complete explosion of 1 mol of PAP-2.

### Example 11

Synthesis and test of (C₅H₁₄N₂)[K(ClO₄)₃] (PAP-H2) (general formula ABX₃, A is 1,4-diazepane-1,4-diium, B is K⁺, and X is ClO₄⁻)

### Synthesis method:

1) 2.00 g of homopiperazine was dissolved in 5 mL of water, and 8.61 g of a perchloric acid solution with a mass fraction of 70%-72% was added and stirred at a room temperature for 5 min;
2) 2.77 g of potassium perchlorate was added to 5 mL of water and heated and stirred to dissolve; and
3) the solutions in step 1) and step 2) were mixed, stirred for 30 min and filtered, and precipitates were washed with ethanol and dried in vacuum to obtain solid powder which was identified as a pure phase of PAP-H2 by X-ray powder diffraction and has the yield of 80%.

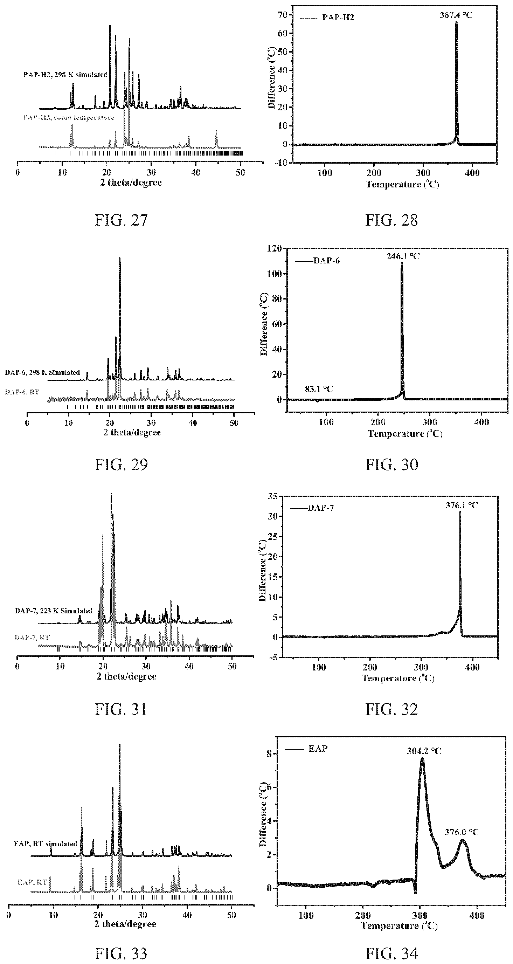

**Powder X-ray diffraction identification pattern:** a powder X-ray diffraction pattern at room temperature is shown in FIG. 27.

**Single crystal structure characterization test:** detailed crystal determination data is shown in Table 11.

**Table 11 Crystal measurement data of PAP-H2**

| Complex | PAP-H2 |
|---|---|
| Formula | C₅H₁₄Cl₃KN₂O₁₂ |
| Formula weight | 439.63 |
| *T*/K | 298(2) |
| *λ*/Å | 1.54184 |
| Crystal system | orthorhombic |
| Space group | *Pbca* |
| *a*/Å | 9.6351(3) |
| *b*/Å | 14.7473(4) |
| *c*/Å | 20.9607(8) |
| *V*/Å³ | 2978.34(17) |
| Z | 8 |
| *D*_{c} /g cm⁻³ | 1.961 |
| reflections collected | 12436 |
| unique reflections | 3028 |
| *R*ᵢₙₜ | 0.0535 |
| *R*₁ [*I* > 2*σ*(*I*)]^{[a]} | 0.0492 |
| *wR*₂ [*I* > 2*σ*(*I*)]^{[b]} | 0.1342 |
| *R*₁ (all data) | 0.0563 |
| *wR*₂ (all data) | 0.1469 |
| GOF on *F*² | 1.073 |

| | |
|---|---|
| ^{[a]} *R*₁ = ∑\|\|*F*_{ο}\| - \|*F*_{c}\|\|/∑\|*F*ₒ\|; ^{[b]} *wR*₂ = {∑w[(*F*ₒ)² - (*F*_{c})²]²/∑w[(*F*ₒ)²]²}^{1/2}; | |

**Differential thermal analysis (DTA) characterization of PAP-H2:** a DTA curve of PAP-H2 is shown in FIG. 28. It can be seen from FIG. 28 that the powdery energetic compound PAP-H2 decomposes at the decomposition peak temperature of 367.4°C, the shape of the decomposition peak is sharp, and the decomposition is rapid.

**Impact sensitivity and friction sensitivity of PAP-H2:** according to the impact and friction test methods developed by the Federal Institute for Materials Research and Testing (BAM), the impact sensitivity of PAP-H2 is 27.5 J, and the friction sensitivity of PAP-H2 is 7 N.

**Detonation heat, detonation pressure and detonation velocity of energetic compound PAP-H2 obtained according to density functional theory (DFT):** according to the DFT (J. Am. Chem. Soc. 2012, 134, 1422), the decomposition heat (decomposition enthalpy Δ*H*_{det}) of PAP-H2 is calculated to be about 1.27 kcal/g, and according to the Kamlet-Jacob formula, the detonation velocity of PAP-5 is calculated to be about 8.17 km/s, and the detonation pressure is calculated to be about 31.1 GPa.

**Volume of** gas **produced per mole of PAP-H2:** regarding the product judgment of complete detonation of energetic materials in an oxygen-free environment, according to the literature (J. Am. Chem. Soc. 2012, 134, 1422; J. Phys. Chem. A. 2014, 118, 4575; Chem. Eur. J. 2016, 22, 1141), final decomposition products are: gaseous substances such as nitrogen, hydrogen halide, water and carbon dioxide, and solid substances such as chloride salts of metals and elemental carbon (if oxygen atoms are not sufficient to completely convert all carbon atoms into carbon dioxide). Therefore, after complete detonation of 1 mole of PAP-H2 in an oxygen-free environment, 12 moles of gaseous substances can be produced, and 2 moles of elemental carbon and 1 mole of potassium chloride solids remain. In the case of mixing sufficient oxidant (such as commonly used NH₄ClO₄), after complete detonation of 1 mole of PAP-H2, 1 mole of potassium chloride solids remain.

### Example 12

Synthesis and test of (C₆H₁₄N₂)(NH₃OH)(ClO₄)₃ (DAP-6) (general formula ABX₃, A is 1,4-diazabicyclo[2.2.2]octane-1,4-diium, B is NH₃OH⁺, and X is ClO₄⁻)

### Synthesis method:

1) 5.74 g of a perchloric acid solution with a mass fraction of 70%-72% was added to 5 mL of water, and then, 1.32 g of a hydroxylamine solution with a mass fraction of 50% was added while stirring and stirred at a room temperature for 5 min;
2) 2.24 g of 1,4-diazabicyclo[2.2.2]octane was dissolved in 5 mL of water; and
3) the solutions in step 1) and step 2) were mixed, stirred for 10 min and filtered, and precipitates were washed with n-butanol and dried in vacuum to obtain solid powder which was identified as a pure phase of DAP-6 by X-ray powder diffraction and has the yield of 80%.

**Powder X-ray diffraction identification pattern:** a powder X-ray diffraction pattern at room temperature is shown in FIG. 29.

**Single crystal structure characterization test:** the schematic diagram of a crystal structure is shown in FIG. 5. As can be seen in FIG. 5, there are six ClO₄⁻ ions at the X site around the NH₃OH⁺ ion at the B site to form a squashed octahedron, adjacent NH₃OH⁺ ions are connected by three *µ₂*-ClO₄⁻ ions to form a one-dimensional chain in a *b*-axis direction, and the organic cation 1,4-diazabicyclo[2.2.2]octane-1,4-diium (H₂dabco²⁺) cation at the A site fills the interchain. Detailed crystal measurement data are shown in Table 12.

**Table 12 Crystal measurement data of DAP-6**

| Complex | DAP-6 |
|---|---|
| Formula | C₆H₁₈Cl₃N₃O₁₃ |
| Formula weight | 446.58 |
| *T*/K | 223(2) |
| *λ*/Å | 1.54184 |
| Crystal system | monoclinic |
| Space group | *P*2₁ |
| *a*/Å | 20.740(1) |
| *b*/Å | 8.2366(2) |
| *c*/Å | 20.790(1) |
| *β*/° | 119.65(1) |
| *V*/Å³ | 3086.4(3) |
| Z | 8 |
| *D*_{c} /g cm⁻³ | 1.922 |
| reflections collected | 21302 |
| unique reflections | 11111 |
| *R*ᵢₙₜ | 0.0490 |
| *R*₁ [*I* > 2σ(*I*)]^{[a]} | 0.0765 |
| *wR*₂ [*I* > 2*σ*(*I*)]^{[b]} | 0.1965 |
| *R*₁ (all data) | 0.0773 |
| *wR*₂ (all data) | 0.1975 |
| GOF on *F*² | 1.043 |
| Completeness | 1.00 |

| | |
|---|---|
| ^{[a]} *R*₁ = ∑\|\|*F*_{ο}\| - \|*F*_{c}\|\|/∑\|*F*ₒ\|; ^{[b]} *wR*₂ = {∑w[(*F*ₒ)² - (*F*_{c})²]²/∑w[(*F*ₒ)²]²}^{1/2}; | |

**Differential thermal analysis (DTA) characterization of DAP-6:** A DTA curve of DAP-6 is shown in FIG. 30. It can be seen from FIG. 30 that the decomposition peak temperature of the powdery energetic compound DAP-6 is 246.1°C, and the shape of the decomposition peak is sharp, indicating that the decomposition process is very rapid.

**Detonation heat, detonation pressure and detonation velocity of energetic compound DAP-6 obtained according to density functional theory (DFT):** according to the DFT (J. Am. Chem. Soc. 2012, 134, 1422), the decomposition heat (decomposition enthalpy Δ*H*_{det}) of DAP-6 is calculated to be about 1.52 kcal/g, and according to the Kamlet-Jacob formula, the detonation velocity of DAP-6 is calculated to be about 9.12 km/s, and the detonation pressure is calculated to be about 38.1 GPa.

**Theoretical specific impulse of energetic compound DAP-6 calculated by DFT and EXPLO5 software:** according to the DFT (J. Am. Chem. Soc. 2012, 134, 1422), the enthalpy of formation of DAP-6 is calculated to be about -373.7 kJ/mol, and the enthalpy of formation is substituted into EXPLO5 v.6.04.02 to calculate the theoretical specific impulse of DAP-6 to be 265.3 s.

**Oxygen balance parameter and volume of gas produced per mole of DAP-6:** for the oxygen balance obtained based on CO₂ product calculation, that is, for the molecular formula CₐH_{b}N_{c}Cl_{d}Oₑ, the oxygen balance parameter is OB[%] = 1600[e - 2a - (b - d)/2]/MW, wherein MW is the relative molecular mass of the molecule; and the oxygen balance parameter of DAP-6 is calculated to be -23.3%. Regarding the product judgment of complete detonation of energetic materials in an oxygen-free environment, according to the literature (J. Am. Chem. Soc. 2012, 134, 1422; J. Phys. Chem. A 2014, 118,4575; Chem. Eur. J. 2016, 22, 1141), final decomposition products are: gaseous substances such as nitrogen, hydrogen halide, water and carbon dioxide, and solid substances such as elemental carbon (if oxygen atoms are not sufficient to completely convert all carbon atoms into carbon dioxide). Therefore, after complete detonation of 1 mole of DAP-6 in an oxygen-free environment, 14.75 moles of gaseous substances can be produced, and 3.25 moles of elemental carbon remains. In the case of mixing sufficient oxidant (such as commonly used NH₄ClO₄), after complete detonation of DAP-6, no solid remains.

### Example 13

Synthesis and test of (C₆H₁₄N₂)(NH₃NH₂)(ClO₄)₃ (DAP-7) (general formula ABX₃, A is a 1,4-diazabicyclo[2.2.2]octane-1,4-diium, B is NH₂NH₃⁺, and X is ClO₄⁻)

### Synthesis method:

1)2.24 g of 1,4-diazabicyclo[2.2.2]octane was dissolved in 5 mL of water, and 5.74 g of a perchloric acid solution with a mass fraction of 70%-72% was added while stirring and stirred at a room temperature for 5 min; and
2)1.02 g of hydrazine hydrate liquid was added while stirring, stirred for 10 min and filtered, and precipitates were washed with ethanol and dried in vacuum to obtain solid powder which was identified as a pure phase of DAP-7 by X-ray powder diffraction and has the yield of 90%.

**Powder X-ray diffraction identification pattern:** a powder X-ray diffraction pattern at room temperature is shown in FIG. 31.

**Single crystal structure characterization test:** the schematic diagram of a crystal structure is shown in FIG. 6. As can be seen in FIG. 6, there are six ClO₄⁻ ions at the X site around the NH₂NH₃⁺ ion at the B site to form a squashed octahedron, adjacent NH₂NH₃⁺ ions are connected by three *µ*₂-ClO₄⁻ ions to form a one-dimensional chain in a *b*-axis direction, and the organic cation 1,4-diazabicyclo[2.2.2]octane-1,4-diium (H₂dabco²⁺) cation at the A site fills the interchain. Detailed crystal measurement data are shown in Table 13.

**Table 13 Crystal measurement data of DAP-7**

| Complex | DAP-7 |
|---|---|
| Formula | C₆H₁₉Cl₃N₄O₁₂ |
| Formula weight | 445.60 |
| *T*/K | 223(2) |
| *λ*/Å | 1.54178 |
| Crystal system | monoclinic |
| Space group | *P*2₁/*m* |
| *a*/Å | 10.378(2) |
| *b*/Å | 8.0505(7) |
| *c*/Å | 10.587(2) |
| *β*/° | 117.99 (2) |
| *V*/Å³ | 781.0(2) |
| Z | 2 |
| *D*_{c} /g cm⁻³ | 1.895 |
| reflections collected | 2680 |
| unique reflections | 1604 |
| *R*ᵢₙₜ | 0.0326 |
| *R*₁ [*I* > 2*σ*(*I*)]^{[a]} | 0.0620 |
| *wR*₂ [*I* > 2*σ*(*I*)]^{[b]} | 0.16.94 |
| *R*₁ (all data) | 0.0638 |
| *wR*₂ (all data) | 0.1704 |
| GOF on *F*² | 1.124 |
| Completeness | 0.99 |

| | |
|---|---|
| ^{[a]} *R*₁ = ∑\|\|*F*_{ο}\| - \|*F*_{c}\|\|/∑\|*F*ₒ\|; ^{[b]} *wR*₂ = {∑w[(*F*ₒ)² - (*F*_{c})²]²/∑w[(*F*ₒ)²]²}^{1/2}; | |

**Differential thermal analysis (DTA) characterization of DAP-7:** A DTA curve of DAP-7 is shown in FIG. 32. It can be seen from FIG. 32 that the decomposition peak temperature ofthe powdery energetic compound DAP-7 is 376.1°C, and the shape ofthe decomposition peak is sharp, indicating that the decomposition process is very rapid.

**Detonation heat, detonation pressure and detonation velocity of energetic compound DAP-7 obtained according to density functional theory (DFT):** according to the DFT (J. Am. Chem. Soc. 2012, 134, 1422*),* the decomposition heat (decomposition enthalpy Δ*H*_{det}) of DAP-7 is calculated to be about 1.43 kcal/g, and according to the Kamlet-Jacob formula, the detonation velocity of DAP-7 is calculated to be about 8.88 km/s, and the detonation pressure is calculated to be about 35.8 GPa.

**Theoretical specific impulse of energetic compound DAP-7 calculated by DFT and EXPLO5 software:** according to the DFT (J. Am. Chem. Soc. 2012, 134, 1422), the enthalpy of formation of DAP-7 is calculated to be about -362.1 kJ/mol, and the enthalpy of formation is substituted into EXPLO5 v.6.04.02 to calculate the theoretical specific impulse of DAP-7 to be 256.9 s.

**Oxygen balance parameter and volume of gas produced per mole of DAP-7:** for the oxygen balance obtained based on CO₂ product calculation, that is, for the molecular formula CₐH_{b}N_{c}Cl_{d}Oₑ, the oxygen balance parameter is OB[%] = 1600[e - 2a - (b - d)/2]/MW, wherein MW is the relative molecular mass ofthe molecule; and the oxygen balance parameter of DAP-7 is calculated to be -28.7%. Regarding the product judgment of complete detonation of energetic materials in an oxygen-free environment, according to the literature (J. Am. Chem. Soc. 2012, 134, 1422; J. Phys. Chem. A 2014, 118,4575; Chem. Eur. J. 2016, 22, 1141), final decomposition products are: gaseous substances such as nitrogen, hydrogen halide, water and carbon dioxide, and solid substances such as elemental carbon (if oxygen atoms are not sufficient to completely convert all carbon atoms into carbon dioxide). Therefore, after complete detonation of 1 mole of DAP-7 in an oxygen-free environment, 15 moles of gaseous substances can be produced, and 4 moles of elemental carbon remains. In the case of mixing sufficient oxidant (such as commonly used NH₄ClO₄), after complete detonation of DAP-7, no solid remains.

### Example 14

Synthesis and testing of (H₂EA)₂(NH₄)(ClO₄)₅(EAP) (general formula B'₂A'X₅, A' is NH₄⁺, B is ethylenediammonium cation H₂EA₂²⁺, and X is ClO₄⁻)

### Synthesis method:

1) 7.14 g of a perchloric acid solution with a mass fraction of 70%-72% was added to 1 mL of water and stirred uniformly;
2) 1.20 g of ethylenediamine was added to 4 mL of water and stirred uniformly; and
3) the solutions in step 1) and step 2) were mixed and stirred for 10 min, then 1.4 g of an aqueous ammonia solution with a mass fraction of 25% was added while stirring, stirred for 30 min and filtered, and precipitates were washed with acetone and dried in vacuum to obtain solid powder which was identified as a pure phase of EAP by X-ray powder diffraction and has the yield of 80%.

**Powder X-ray diffraction identification pattern:** a powder X-ray diffraction pattern at room temperature is shown in FIG. 33.

### Single crystal structure characterization test:

The schematic diagram of a crystal structure is shown in FIG. 8. As can be seen in FIG. 8, adjacent NH₄⁺ ions are connected to form a diamond mesh structure with a side length of 7.205 Å, there are two kinds of perchlorates in the independent unit of the structure, adjacent Cl1 (from ClO₄⁻) ions in one of the perchlorates are also connected to form a diamond mesh structure with a side length of 7.205 Å, two sets of diamond meshes are interspersed with each other, there are 6 ethylenediammonium cations around each NH₄⁺ ion and Cl1 to form an octahedral structure, three octahedrons are connected by a *µ*₃-ethylenediammonium cation, and the other perchlorate (Cl2) is located among the three octahedrons. Detailed crystal measurement data are shown in Table 14.

**Table 14 Crystal measurement data of EAP**

| Complex | EAP |
|---|---|
| Formula | C₄ H₂₄Cl₅N₅O₂₀ |
| Formula weight | 639.53 |
| *T*/*K* | 298(2) |
| λ/Å | 1.54184 |
| Crystal system | tetragonal |
| Space group | I4₁/a |
| *a*/Å | 10.7731(1) |
| *c*/Å | 19.1420(3) |
| *V*/Å³ | 2221.61(5) |
| *Z* | 4 |
| *D_{c}*/g cm⁻³ | 1.912 |
| reflections collected | 6645 |
| unique reflections | 1151 |
| *R*ᵢₙₜ | 0.0461 |
| *R*₁ [*I*> 2σ(*I*)]^{[a]} | 0.0463 |
| *w*R₂[*I*> 2σ(*I*)]^{[b]} | 0.1161 |
| *R*₁ (all data) | 0.0467 |
| *wR*₂ (all data) | 0.1164 |
| GOF on *F*² | 1.191 |
| Completeness | 0.94 |

| | |
|---|---|
| [a] *R*₁ = Σ∥*F*_{ο}\| - \|*F*_{c}\|\|/∑\|*F*ₒ\|; ^{[b]} *wR*₂ = {Σw[(*F*ₒ)² - (*F*_{c})²]²/∑w[(*F*ₒ)²]²}^{1/2}; | |

**Differential thermal analysis (DTA) characterization of EAP:** a DTA curve of EAP is shown in FIG. 34. It can be seen from FIG. 34 that the powdery energetic compound EAP has two decomposition exothermic peaks, and the peak temperatures are 304.2°C and 376.0°C respectively.

**Detonation heat, detonation pressure and detonation velocity of energetic compound EAP obtained according to density functional theory (DFT):** According to the DFT (J. Am. Chem. Soc. 2012, 134, 1422), the decomposition heat (decomposition enthalpy ΔHdet) of EAP is calculated to be about 1.21 kcal/g, and according to the Kamlet-Jacob formula, the detonation velocity of EAP is calculated to be about 8.97 km/s, and the detonation pressure is calculated to be about 37.0 GPa.

**Volume of gas produced per mole of EAP:** regarding the product judgment of complete detonation of energetic materials in an oxygen-free environment, according to the literature *(*J. Am. Chem. Soc. 2012, 134, 1422; J. Phys. Chem. A 2014, 118, 4575; Chem. Eur. J. 2016,22, 1141), final decomposition products are all gaseous products: gaseous substances such as nitrogen, hydrogen halide, water, carbon dioxide and oxygen. Therefore, after complete detonation of 1 mole of EAP in an oxygen-free environment, 22.25 moles of gaseous substances can be produced.

### Example 15

Synthesis and test of (C₄H₁₂N₂)(H₂EA)(ClO₄)₄(PEP) (general formula AB'X₄, A is piperazine-1,4-diium, B is ethylenediammonium cation H₂EA₂²⁺, and X is ClO₄⁻)

### Synthesis method:

1) 11.43 g of a perchloric acid solution with a mass fraction of 70%-72% was added to 15 mL of water, and 1.20 g of ethylenediamine was added while stirring and stirred at a room temperature for 5 min;
2) 1.72 g of anhydrous piperazine was dissolved in 5 mL of water; and
3) the solutions in step 1) and step 2) were mixed, stirred for 10 min and filtered, and precipitates were washed with ethanol and dried in vacuum to obtain solid powder which was identified as a pure phase of PEP by X-ray powder diffraction and has the yield of 85%.

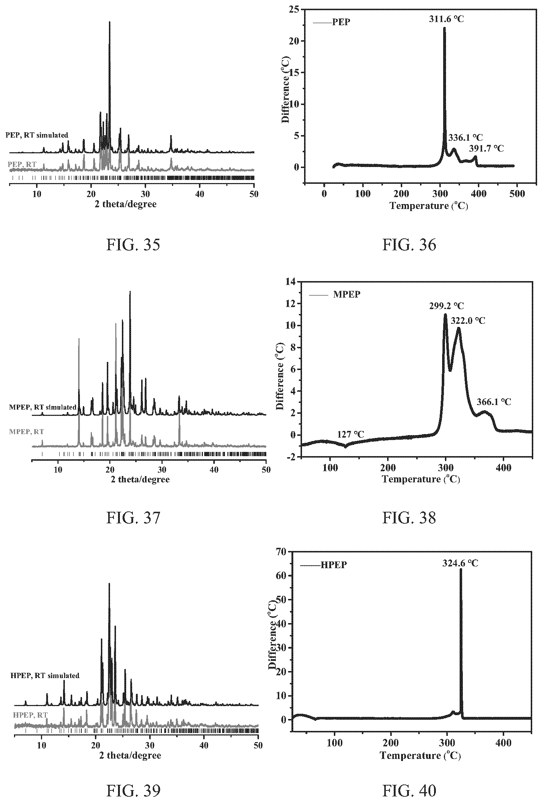

**Powder X-ray diffraction identification pattern:** a powder X-ray diffraction pattern at room temperature is shown in FIG. 35.

**Single crystal structure characterization test:** the schematic diagram of a crystal structure is shown in FIG. 7. There are ten ClO₄⁻ ions around each H₂EA²⁺ ion to form an irregular dodecahedron (as shown in FIG. 7a), adjacent H₂EA²⁺ ions are connected by four *µ*₄-ClO₄⁻ ions and six *µ*₂-ClO₄⁻ ions to form a layered structure in *b*-axis and c-axis directions (as shown in FIG. 7b), and piperazine cations and perchlorate anions are arranged alternately between two adjacent layers (as shown in FIG. 7c). Detailed crystal measurement data are shown in Table 15.

**Table 15 Crystal measurement data of PEP**

| Complex | PEP |
|---|---|
| Formula | C₆H₂₂Cl₄N₄O₁₆ |
| Formula weight | 548.07 |
| *T*/K | 298(2) |
| λ/Å | 1.54184 |
| Crystal system | orthorhombic |
| Space group | *Pbca* |
| *a*/Å | 23.9304(2) |
| *b*/Å | 10.3226(1) |
| *c*/Å | 31.4590(4) |
| *β*/*°* | 90 |
| *V*/Å³ | 7771.12(15) |
| Z | 16 |
| *D_{c}*/g cm⁻³ | 1.874 |
| reflections collected | 48851 |
| unique reflections | 8137 |
| *R*ᵢₙₜ | 0.0545 |
| *R*₁[*I*> 2σ(*I*)]^{[a]} | 0.0479 |
| *wR*₂ [*I*> 2σ(*I*)]^{[b]} | 0.1386 |
| *R*₁ (all data) | 0.0559 |
| *wR*₂ (all data) | 0.1454 |
| GOF on *F*² | 1.046 |
| Completeness | 1.00 |

| | |
|---|---|
| [a] *R*₁ = Σ∥*F*_{ο}\| - \|F_{c}\|\|/∑\|Fₒ\|; [b] *wR*₂ = {Σw[(Fₒ)² - (*F*_{c})²]²/∑w[(*F*ₒ)²]²}^{1/2}; | |

**Differential thermal analysis (DTA) characterization of PEP:** a DTA curve of PEP is shown in FIG. 36. It can be seen from FIG. 36 that the powdery energetic compound PEP decomposes at the decomposition peak temperature of 311.6°C, the shape of the decomposition peak is sharp, and the decomposition is rapid. Subsequently, there are two sustained slow decompositions with the peak temperatures of 336.1°C and 391.7°C respectively.

**Detonation heat, detonation pressure and detonation velocity of energetic compound PEP obtained according to density functional theory (DFT):** according to the DFT *(*J. Am. Chem. Soc. 2012, 134, 1422), the decomposition heat (decomposition enthalpy Δ*H*_{det}) of PEP is calculated to be about 1.46 kcal/g, and according to the Kamlet-Jacob formula, the detonation velocity of PEP is calculated to be about 9.09 km/s, and the detonation pressure is calculated to be about 37.6 GPa.

**Volume of gas produced per mole of PEP:** regarding the product judgment of complete detonation of energetic materials in an oxygen-free environment, according to the literature *(*J. Am. Chem. Soc. 2012, 134, 1422; J. Phys. Chem. A 2014, 118, 4575; Chem. Eur. J. 2016, 22, 1141), final decomposition products are: gaseous substances such as nitrogen, hydrogen halide, water and carbon dioxide, and solid substances such as elemental carbon (if oxygen atoms are not sufficient to completely convert all carbon atoms into carbon dioxide). Therefore, after complete detonation of 1 mole of PEP in an oxygen-free environment, 18.5 moles of gaseous substances can be produced, and 2.5 moles of elemental carbon remains. In the case of mixing sufficient oxidant, after complete detonation of PEP, no solid remains.

### Example 16

Synthesis and test of (C₅H₁₄N₂)(H₂EA)(ClO₄)₄(MPEP) (general formula AB'X₄, A is 1-methylpiperazine-1,4-diium B is ethylenediammonium cation H₂EA₂²⁺, and X is ClO₄⁻)

### Synthesis method:

1) 11.43 g of a perchloric acid solution with a mass fraction of 70%-72% was added to 15 mL of water, and 1.20 g of ethylenediamine was added while stirring and stirred at a room temperature for 5 min;
2) 2.00 g of 1-methylpiperazine was dissolved in 5 mL of water; and
3) the solutions in step 1) and step 2) were mixed, stirred for 10 min and filtered, and precipitates were washed with ethanol and dried in vacuum to obtain solid powder which was identified as a pure phase of MPEP by X-ray powder diffraction and has the yield of 80%.

**Powder X-ray diffraction identification pattern:** a powder X-ray diffraction pattern at room temperature is shown in FIG. 37.

**Single crystal structure characterization test:** detailed crystal measurement data are shown in Table 16.

**Table 16 Crystal measurement data of MPEP**

| Complex | MPEP |
|---|---|
| Formula | C₇H₂₄Cl₄N₄O_{I6} |
| Formula weight | 562.10 |
| *T*/K | 298(2) |
| λ/Å | 1.54184 |
| Crystal system | monoclinic |
| Space group | *P*2₁/*c* |
| *a*/Å | 12.7872(5) |
| *b*/Å | 10.3107(3) |
| *c*/Å | 15.9171(5) |
| *β*/*°* | 99.987(4) |
| *V*/Å³ | 2066.8(1) |
| Z | 4 |
| *D_{c}* /g cm⁻³ | 1.806 |
| reflections collected | 19174 |
| unique reflections | 4203 |
| *R*ᵢₙₜ | 0.0687 |
| *R*₁ [*I*> 2σ(*I*)]^{[a]} | 0.1057 |
| *wR*₂ [*I*> 2σ(*I*)]^{[b]} | 0.3638 |
| *R*₁ (all data) | 0.1089 |
| *wR*₂ (all data) | 0.3671 |
| GOF on *F*² | 1.729 |
| Completeness | 0.99 |

| | |
|---|---|
| [a] *R*₁= Σ\|\|*F*ₒ\| - \|F_{c}\|\|/Σ\|Fₒ\|; [b] *wR*₂ = {Σw[(*F*ₒ)² - (*F*_{c})²]²/∑w[(*F*ₒ)²]²}^{1/2}; | |

**Differential thermal analysis (DTA) characterization of MPEP:** a DTA curve of MPEP is shown in FIG. 38. It can be seen from FIG. 38 that the powdery energetic compound MPEP has multiple steps of decompositions and decomposes at the decomposition peak temperature of 299.2°C, and the decomposition is rapid. Subsequently, there are two sustained slow decompositions with the peak temperatures of 322.0°C and 366.1°C respectively.

**Detonation heat, detonation pressure and detonation velocity of energetic compound MPEP obtained according to density functional theory (DFT):** according to the DFT *(*J. Am. Chem. Soc. 2012, 134, 1422), the decomposition heat (decomposition enthalpy Δ*H*_{det}) of MPEP is calculated to be about 1.40 kcal/g, and according to the Kamlet-Jacob formula, the detonation velocity of MPEP is calculated to be about 8.73 km/s, and the detonation pressure is calculated to be about 34.0 GPa.

**Volume of gas produced per mole of MPEP:** regarding the product judgment of complete detonation of energetic materials in an oxygen-free environment, according to the literature *(*J. Am. Chem. Soc. 2012, 134, 1422; J. Phys. Chem. A 2014, 118, 4575; Chem. Eur. J. 2016, 22, 1141), final decomposition products are: gaseous substances such as nitrogen, hydrogen halide, water and carbon dioxide, and solid substances such as elemental carbon (if oxygen atoms are not sufficient to completely convert all carbon atoms into carbon dioxide). Therefore, after complete detonation of 1 mole of MPEP in an oxygen-free environment, 19 moles of gaseous substances can be produced, and 4 moles of elemental carbon remains. In the case of mixing sufficient oxidant, after complete detonation of MPEP, no solid remains.

### Example 17

Synthesis and test of (C₅H₁₄N₂)(H₂EA)(ClO₄)₄(HPEP) (general formula AB'X₄, A is 1,4-diazepane-1,4-diium, B is ethylenediammonium cation H₂EA₂²⁺, and X is ClO₄⁻)

### Synthesis method:

1) 11.43 g of a perchloric acid solution with a mass fraction of 70%-72% was added to 15 mL of water, and 1.20 g of ethylenediamine was added while stirring and stirred at a room temperature for 5 min;
2) 2.00 g of homopiperazine was dissolved in 5 mL of water; and
3) the solutions in step 1) and step 2) were mixed, stirred for 10 min and filtered, and precipitates were washed with ethanol and dried in vacuum to obtain solid powder which was identified as a pure phase of HPEP by X-ray powder diffraction and has the yield of 75%.

**Powder X-ray diffraction identification pattern:** a powder X-ray diffraction pattern at room temperature is shown in FIG. 39.

**Single crystal structure characterization test:** Detailed crystal measurement data are shown in Table 17.

**Table 17 Crystal measurement data of HPEP**

| Complex | HPEP |
|---|---|
| Formula | C₇H₂₄Cl₄N₄O₁₆ |
| Formula weight | 562.10 |
| *T*/K | 298(2) |
| λ/Å | 1.54184 |
| Crystal system | monoclinic |
| Space group | *P*2₁/*c* |
| *a*/Å | 7.8381(3) |
| *b*/Å | 24.9901(8) |
| *c*/Å | 10.4647(4) |
| *β*/*°* | 93.649(3) |
| *V*/Å³ | 2045.62(13) |
| Z | 4 |
| *D_{c}* /g cm⁻³ | 1.825 |
| reflections collected | 15275 |
| unique reflections | 4158 |
| *R*ᵢₙₜ | 0.0637 |
| *R*₁ [*I*> 2*σ*(*I*)]^{[a]} | 0.0862 |
| *wR*₂ *[I>* 2σ(*I*)]^{[b]} | 0.2664 |
| *R*₁ (all data) | 0.0990 |
| *wR*₂ (all data) | 0.2864 |
| GOF on *F*² | 1.083 |
| Completeness | 0.99 |

| | |
|---|---|
| [a] *R*₁ = Σ∥*F*_{ο}\| - \|*F*_{c}\|\|/Σ\|*F*ₒ\|; [b] *wR*₂ = {Σw[(*F*o)² - (*F*_{c})²]²/∑w[(*F*ₒ)²]²}^{1/2}; | |

**Differential thermal analysis (DTA) characterization of HPEP:** a DTA curve of HPEP is shown in FIG. 40. It can be seen from FIG. 40 that the powdery energetic compound HPEP decomposes at the decomposition peak temperature of 324.6°C, and the decomposition is rapid.

**Detonation heat, detonation pressure and detonation velocity of energetic compound HEAP obtained according to density functional theory (DFT):** according to the DFT *(*J. Am. Chem. Soc. 2012, 134, 1422), the decomposition heat (decomposition enthalpy Δ*H*_{det}) of HPEP is calculated to be about 1.40 kcal/g, and according to the Kamlet-Jacob formula, the detonation velocity of MPEP is calculated to be about 8.76 km/s, and the detonation pressure is calculated to be about 34.4 GPa.

**Volume of gas produced per mole of HPEP:** regarding the product judgment of complete detonation of energetic materials in an oxygen-free environment, according to the literature *(*J. Am. Chem. Soc. 2012, 134, 1422; J. Phys. Chem. A 2014, 118, 4575; Chem. Eur. J. 2016, 22, 1141), final decomposition products are: gaseous substances such as nitrogen, hydrogen halide, water and carbon dioxide, and solid substances such as elemental carbon (if oxygen atoms are not sufficient to completely convert all carbon atoms into carbon dioxide). Therefore, after complete detonation of 1 mole of HPEP in an oxygen-free environment, 19 moles of gaseous substances can be produced, and 4 moles of elemental carbon remains. In the case of mixing sufficient oxidant, after complete detonation of HPEP, no solid remains.

Table 18 shows the performance comparison of the compounds of Examples 14-17 (with general formulas of AB'X₄ and B'₂A'X₅) and the comparative example (with a chemical formula of AB'X₄, A is 1,4-diazabicyclo[2.2.2]octane-1,4-diium, B is ethylenediammonium cation H₂EA₂²⁺, and X is ClO₄⁻).

*ρ* represents specific gravity, *Q* represents detonation heat, *D* represents detonation velocity, P represents detonation pressure,*ΔH_{f}* represents the enthalpy of formation obtained according to the hypothetical detonation reaction by the Hess's law by inversion, *Iₛₚ* represents the specific impulse calculated by EXPLO5 v6.04.02 software according to the enthalpy of formation obtained by inversion, and *OB* represents the oxygen balance obtained based on CO2 calculation. When the molecular formula is CₐH_{b}N_{c}Cl_{d}Oₑ, OB[%] = 1600 [ e - 2a - (b - d) / 2 ] / MW, wherein MW is the relative molecular mass of the molecule.

**Table 18 Performance comparison of compounds in Examples and Comparative Example**

| Compound | *ρ* (g·cm⁻³) | *Q* (kj·g⁻¹) | *D* (km·s⁻¹) | *P* (GPa) | Δ*H*_{f} (kj·mol⁻¹) | *I*ₛₚ (s) | *OB* (%) |
|---|---|---|---|---|---|---|---|
| Example 14 | 1.91 ^{a)} | 5.07 | 9.024 | 37.6 | -1145.67 | 249.1 | 6.25 |
| Example 15 | 1.88 ^{a)} | 6.10 | 9.090 | 37.6 | -625.74 | 264.2 | -14.6 |
| Example 16 | 1.82 ^{b)} | 5.86 | 8.729 | 34.0 | -721.37 | 256.4 | -22.8 |
| Example 17 | 1.83 ^{b)} | 5.87 | 8.764 | 34.4 | -715.75 | 256.8 | -22.8 |
| Comparative Example | 1.87 ^{a)} | 6.03 | 8.867 | 35.7 | -541.05 | 257.6 | -27.8 |

It can be seen from the above examples that:
1) Compared with Comparative Example, the oxygen balance of Examples 14,15, 16 and 17 of this application is closer to zero oxygen balance. In particular, the oxygen balance parameter of Example 14 is positive, meaning that it can be used as an oxidant ingredient in various formulations.
2) Compared with Comparative Example, Example 15 has a similar (slightly higher) density, but has better performances such as higher detonation heat, detonation velocity, detonation pressure and specific impulse.
3) Compared with Comparative Example, the oxygen balance and density of Example 14 are significantly higher, and better technical effects are reflected in performance indexes such as detonation velocity and detonation pressure.

The above implementations are only preferred implementations of this application, and cannot be used to limit the scope of protection of this application. Any insubstantial changes and replacements made by those skilled in the art on the basis of this application fall within the scope of protection claimed in this application.

## Claims

1. A compound ABX₃, AB'X₄, or B'₂A'X₅, consisting of A cation or A' cation, B cation or B' cation, and X anion, wherein
the A cation is a nitrogen-containing organic cation;
the B cation is a cation;
the A' cation is a cation;
the B' cation is a saturated aliphatic diammonium cation; and
the X anion is an anionic energetic ligand.

2. The compound according to claim 1, wherein
the compound is compound ABX₃ consisting of A cation, B cation, and X anion, wherein
the A cation is a nitrogen-containing organic cation;
the B cation is a cation; and
the X anion is an anionic energetic ligand;
or
the compound is compound AB'X₄ consisting of A cation, B' cation, and X anion or is compound B'₂A'X₅ consisting of A' cation, B' cation, and X anion, wherein
the A cation is a divalent nitrogen-containing monocyclic heterocyclic cation;
the A' cation is at least one of an alkali metal ion and a monovalent nitrogen-containing cation;
the B' cation is a saturated aliphatic diammonium cation; and
the X anion is an anionic energetic ligand.

3. The compound according to claim 1 or 2, wherein
the A cation is selected from at least one of ions of Formula (I) and Formula (II), and derivatives thereof, wherein *n*₁, n₂, n₃, n₄, and n₅ each is a positive integer; R₁, R₂, R₃, and R₄ are selected from at least one of hydrogen, methyl, ethyl, propyl, isopropyl, butyl, isobutyl, *sec*-butyl, *tert*-butyl, hydroxyl, carbonyl, carboxyl, amino, halogen, mercapto, peroxy, diazenyl, and nitro groups; the derivatives refer to the organic cationic moiety in which hydrogen atoms are substituted, simultaneously or not, with substituents, and the substituents comprise methyl, ethyl, propyl, isopropyl, butyl, isobutyl, *sec*-butyl, *tert*-butyl, hydroxyl, carbonyl, carboxyl, amino, halogen, mercapto, peroxy, diazenyl, nitro, *etc.,* 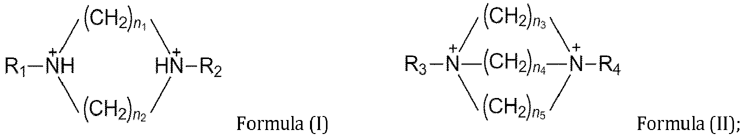 wherein,
the A cation is selected from at least one of organic cations of Formula (I) and derivatives thereof, and
any one of *n*₁ and *n*₂ is greater than 2, or any one of R₁ and R₂ comprises at least one carbon atom;
or
the A cation is selected from at least one of organic cations of Formula (II) and derivatives thereof, and
any one of *n*₃, *n*₄, and *n*₅ is greater than 2, or any one of R₃ and R₄ comprises at least one carbon atom;
preferably, the A cation is selected from at least one of 1-methylpiperazine-1,4-diium, 1-methyl-1,4-diazabicyclo[2.2.2]octane-1,4-diium, and 1,4-diazepane-1,4-diium, and derivatives thereof.

4. The compound according to any one of claims 1 to 3, wherein
the B cation is selected from at least one of potassium ion, ammonium ion, silver ion, hydroxylammonium ion, and hydrazinium ion;
preferably, the B cation is selected from at least one of potassium ion, ammonium ion, and silver ion;
preferably, the B cation is potassium ion;
or preferably, the B cation is ammonium ion;
or preferably, the B cation is silver ion;
or preferably, the B cation is selected from at least one of hydroxylammonium ion and hydrazinium ion;
preferably, the B cation is hydroxylammonium ion;
or preferably, the B cation is hydrazinium ion.

5. The compound according to any one of claims 1 to 4, wherein
the A' cation is a monovalent nitrogen-containing cation,
preferably, the A' cation has a general formula of NR₃R₄R₅R₆⁺, wherein R₃, R₄, R₅, and R₆ are selected from at least one of hydrogen, methyl, ethyl, propyl, isopropyl, butyl, isobutyl, *sec*-butyl, *tert*-butyl, hydroxyl, carbonyl, carboxyl, amino, halogen, mercapto, peroxy, diazenyl, and nitro groups.

6. The compound according to any one of claims 1 to 5, wherein
the B' cation has a general formula of R₇R₈R₉N⁺-(C_{*n*3}H_{2*n*3})-N⁺R₁₀R₁₁R₁₂, wherein *n*₃ is 1, 2, 3, 4, or 5, and R₇, R₈, R₉, R₁₀, R₁₁, and R₁₂ are selected from at least one of hydrogen, methyl, ethyl, propyl, isopropyl, butyl, isobutyl, *sec*-butyl, and *tert-*butyl;
preferably, in the B' cation, *n*₃ is 2 and/or R₇, R₈, R₉, R₁₀, R₁₁, and R₁₂ are hydrogen;
preferably, the B' cation is ethylenediammonium cation.

7. The compound according to any one of claims 1 to 6, wherein
the X anion is selected from at least one of chlorate ion, perchlorate ion, bromate ion, perbromate ion, iodate ion, periodate ion, nitrate ion, fulminate ion, diazenyl group, and azide ion;
preferably, the X anion is selected from at least one of perchlorate ion, perbromate ion, and periodate ion;
preferably, the X anion is perchlorate ion.

8. A method of preparing a compound, comprising:
mixing A component or A' component, B component or B' component, and X component, in any order, in a liquid reaction system; and
obtaining a solid product produced in the liquid reaction system;
wherein,
the A component is selected from at least one of nitrogen-containing organic compounds and salts thereof;
the B component and the A' component are selected from at least one of alkali metal salts and hydroxides, ammonium salts, ammonia, silver salts, monovalent nitrogen-containing cation salts, and monovalent nitrogen-containing bases;
the B' component is selected from at least one of saturated aliphatic diammonium salts and saturated aliphatic diamines;
the X component is selected from at least one of acids and salts comprising anionic energetic ligands; and
the liquid reaction system is a polar solvent dissolving the A component or A' component, the B component or B' component, and the X component;
preferably, the steps also comprise further purification;
preferably, the compound is the compound of any one of claims 1 to 7.

9. The method according to claim 8, wherein
the compound is compound ABX₃ consisting of A cation, B cation, and X anion, wherein
the A cation is a nitrogen-containing organic cation;
the B cation is a cation;
the X anion is an anionic energetic ligand;
the A component is at least one of nitrogen-containing organic compounds and salts thereof;
the B component is selected from at least one of salts and hydroxides comprising cations, and ammonia; and
the X component comprises at least one of anionic acids and salts;
or
the compound is compound AB'X₄ consisting of A cation, B' cation, and X anion, or is compound B'₂A'X₅ consisting of A' cation, B' cation, and X anion, wherein
the A cation is a divalent nitrogen-containing monocyclic heterocyclic cation;
the A' cation is selected from at least one of nitrogen-containing organic compounds and salts thereof;
the B' cation is a saturated aliphatic diammonium cation;
the X anion is an anionic energetic ligand;
the A component is selected from at least one of divalent nitrogen-containing monocyclic heterocycles and salts thereof, comprising onium salts;
the A' component is selected from at least one of salts and hydroxides of alkali metal, monovalent nitrogen-containing cation salts, and monovalent nitrogen-containing bases;
the B component is selected from at least one of saturated aliphatic diammonium salts and saturated aliphatic diamines; and
the X component is selected from at least one of acids and salts comprising anions.

10. The method according to claim 8 or 9, wherein
the A component is selected from at least one of compounds of Formula (XI), compounds of Formula (XII), salts of ions of Formula (I) comprising onium salts thereof, and salts of ions of Formula (II) comprising onium salts thereof, and derivatives thereof, wherein n_{1,} n₂, n₃, n₄, and n₅ each is a positive integer; R₁, R₂, R₃, and R₄ are selected from at least one of hydrogen, methyl, ethyl, propyl, isopropyl, butyl, isobutyl, *sec*-butyl, *tert*-butyl, hydroxyl, carbonyl, carboxyl, amino, halogen, mercapto, peroxy, diazenyl, and nitro groups; the derivatives refer to the organic cationic moiety in which hydrogen atoms are substituted, simultaneously or not, with substituents, and the substituents comprise methyl, ethyl, propyl, isopropyl, butyl, isobutyl, *sec-*butyl, *tert*-butyl, hydroxyl, carbonyl, carboxyl, amino, halogen, mercapto, peroxy, diazenyl, nitro, *etc.* 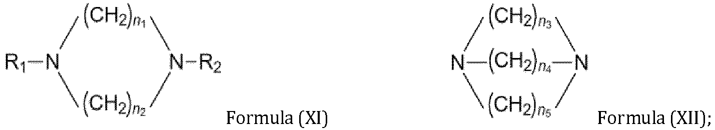 wherein
the A component is selected from at least one of compounds of Formula (XI), and salts of Formula (I), comprising onium salts thereof, and derivatives thereof, and
any one of n₁ and n₂ is greater than 2, or any one of R₁ and R₂ comprises at least one carbon atom;
or
the A component is selected from at least one of compounds of Formula (XII), and salts of ions of Formula (II), comprising onium salts thereof, and derivatives thereof, and
any one of n₃, n₄, and n₅ is greater than 2, or any one of R₃ and R₄ comprises at least one carbon atom;
preferably, the A component is selected from at least one of 1-methylpiperazine, 1-methylpiperazine-1,4-diium, 1-methyl-1,4-diazabicyclo[2.2.2]octane-1,4-diium, 1,4-diazepane, and 1,4-diazepane-1,4-diium, and derivatives thereof.

11. The method according to any one of claims 8 to 10, wherein
the B component is selected from at least one of potassium salts, potassium hydroxide, ammonium salts, ammonia, silver salts, hydroxylammonium ion-containing salts, hydroxylammonium ion-containing bases, hydrazinium ion-containing salts, and hydrazinium ion-containing bases;
preferably,
the B component is selected from at least one of potassium salts and potassium hydroxide, and
the A component is selected from at least one of piperazine, piperazine-1,4-diium, 1,4-diazepane, and 1,4-diazepane-1,4-diium, and derivatives thereof;
or
the B component is selected from at least one of ammonium salts and ammonia, and
the A component is selected from at least one of 1,4-diazabicyclo[2.2.2]octane and hydrogen peroxide reaction products, 1-hydroxy-1,4-diazabicyclo[2.2.2]octane -1,4-diium, 1-methylpiperazine, 1-methylpiperazine-1,4-diium, 1-methyl-1,4-diazabicyclo[2.2.2]octane -1,4-diium, 1,4-diazepane, and 1,4-diazepane-1,4-diium, and derivatives thereof;
or
the B component is selected from silver salts;
preferably,
the B component is selected from at least one of potassium salts and potassium hydroxide, and
the A component is selected from at least one of piperazine and piperazine-1,4-diium, and derivatives thereof;
or
the B component is selected from at least one of ammonium salts and ammonia, and
the A component is selected from at least one of 1,4-diazabicyclo[2.2.2]octane and hydrogen peroxide reaction products, 1-hydroxy-1,4-diazabicyclo[2.2.2]octane -1,4-diium, 1-methylpiperazine, 1-methylpiperazine-1,4-diium, 1,4-diazepane, and 1,4-diazepane-1,4-diium, and derivatives thereof;
or
the B component is selected from silver salts, and
the A cation is selected from at least one of 1,4-diazabicyclo[2.2.2]octane, 1,4-diazabicyclo[2.2.2]octane-1,4-diium, piperazine, piperazine-1,4-diium, 1-methylpiperazine, 1-methylpiperazine-1,4-diium, 1,4-diazepane, and 1,4-diazepane-1,4-diium salt, and derivatives thereof;
or preferably, the B component is selected from at least one of NH₃OH⁺ ion-containing salts and NH₃OH⁺ ion-containing bases;
or preferably, the B component is selected from at least one of NH₃NH₂⁺ ion-containing salts and NH₃NH₂⁺ ion-containing bases.

12. The method according to any one of claims 8 to 11, wherein
the A' component is at least one of salts and hydroxides of monovalent cations;
preferably, the A' component is at least one of salts, bases, and hydroxides of cations of a general formula NR₃R₄R₅R₆⁺, wherein R₃, R₄, R₅, and R₆ are selected from at least one of hydrogen, methyl, ethyl, propyl, isopropyl, butyl, isobutyl, sec-butyl, tert-butyl, hydroxyl, carbonyl, carboxyl, amino, halogen, mercapto, peroxy, diazenyl, and nitro groups; or is compounds of a general formula NR₃R₄R₅;
preferably, the A' component is ammonia or ammonium salts.

13. The method according to any one of claims 8 to 12, wherein
the B component is at least one of salts and hydroxides of ions of a general formula R₇R₈R₉N⁺-(C_{*n*3}H_{2*n*3)}-N⁺R₁₀R₁₁R₁₂, and amines of a general formula R₇R₈N-(C_{*n*3},H_{2*n*3})-NR₁₀R₁₁, wherein *n*₃ is 1,2,3,4, or 5, and R₇, R₈, R₉, R₁₀, R₁₁, and R₁₂ are selected from at least one of hydrogen, methyl, ethyl, propyl, isopropyl, butyl, isobutyl, *sec*-butyl, and *tert*-butyl;
preferably, in the B component, *n*₃ is 2;
preferably, the B component is at least one of ethylene diamine and ethylenediammonium salts.

14. The preparation method according to any one of claims 8 to 12, wherein
the X component is selected from at least one of chloric acid, chlorate, perchloric acid, perchlorate, bromic acid, bromate, perbromic acid, perbromate, iodic acid, iodate, periodic acid, periodate, nitric acid, nitrate, fulminic acid, fulminate, azo salts, and azide salts;
preferably, the X component is selected from at least one of perchloric acid, perchlorate, perbromic acid, perbromate, periodic acid, and periodate;
preferably, the X component is selected from at least one of perchloric acid and perchlorate.

15. An energetic material, comprising
the compound of any one of claims 1 to 7, and/or
the compound prepared by the preparation method of any one of claims 8 to 14;
preferably, the energetic material comprises a primer, explosive, gunpowder propellant, or pyrotechnic.
